(19) Europäisches Patentamt / European Patent Office / Office européen des brevets

(11) **EP 4 005 469 B1**

(12) **EUROPÄISCHE PATENTSCHRIFT**

(45) Veröffentlichungstag und Bekanntmachung des Hinweises auf die Patenterteilung:
**10.07.2024   Patentblatt 2024/28**

(21) Anmeldenummer: **21216003.0**

(22) Anmeldetag: **18.12.2014**

(51) Internationale Patentklassifikation (IPC):
*A61B 5/00* (2006.01)   *G01N 21/47* (2006.01)
*G01N 21/49* (2006.01)   *A61B 5/145* (2006.01)
*G01N 21/17* (2006.01)

(52) Gemeinsame Patentklassifikation (CPC):
**G01N 21/49; A61B 5/14546; A61B 5/1455;**
**G01N 21/474; G01N 21/4795;** A61B 5/14532;
A61B 2562/046; G01N 2021/1782;
G01N 2021/4752; G01N 2201/06153;
G01N 2201/0621; G01N 2201/0626;
G01N 2201/0633; G01N 2201/066;
G01N 2201/1211;                    (Forts.)

(54) **VORRICHTUNG UND VERFAHREN ZUR BESTIMMUNG EINER KONZENTRATION IN EINER PROBE**

DEVICE AND METHOD FOR DETERMINING A CONCENTRATION IN A SAMPLE

DISPOSITIF ET PROCÉDÉ DE DÉTERMINATION D'UNE CONCENTRATION DANS UN ÉCHANTILLON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.01.2014   DE 102014100112**
**14.02.2014   DE 102014101918**

(43) Veröffentlichungstag der Anmeldung:
**01.06.2022   Patentblatt 2022/22**

(62) Dokumentnummer(n) der früheren Anmeldung(en) nach Art. 76 EPÜ:
**16201139.9 / 3 175 783**
**14815357.0 / 3 013 217**

(73) Patentinhaber: **Opsolution GmbH**
**34131 Kassel (DE)**

(72) Erfinder:
• **MAGNUSSEN, Björn**
**34131 Kassel (DE)**
• **STERN, Claudius**
**34929 Ahnatal (DE)**
• **KÖCHER, Wolfgang**
**34121 Kassel (DE)**

(74) Vertreter: **Kluin Patent**
**Patentanwälte Kluin Debelius Weber PartG mbB**
**Benrather Schlossallee 111**
**40597 Düsseldorf (DE)**

(56) Entgegenhaltungen:
WO-A1-2010/056973     WO-A1-94/10901
US-A1- 2002 058 865     US-A1- 2005 020 892
US-A1- 2008 232 653

EP 4 005 469 B1

(52) Gemeinsame Patentklassifikation (CPC): (Forts.)
G01N 2201/1214

**Beschreibung**

**Stand der Technik**

Es sind Anordnungen und Apparate zur optischen in vivo Messung von Analyten in Proben, beispielsweise biologischem Gewebe bekannt. Grundlage sind spektroskopische Verfahren, bei denen Licht in die Probe eingestrahlt wird, es durchläuft und an einem anderen Ort aus der Probe austritt. Die infolge Absorption und Streuung erfolgte Abschwächung des Lichts wird am Austrittort über einen Detektor gemessen. Mit Hilfe entsprechender Referenzmessungen kann aus dieser Messgröße die Konzentration von Analyten bestimmt werden.

Da insbesondere im biologischen Gewebe das detektierte Signal von der Streuung dominiert wird, kommen Verfahren zur Trennung von Absorption und Streuung zu Anwendung. Sie verfügen über räumlich getrennte Detektoren, denen jeweils mindestens zwei Emitter unterschiedlichen Abstands zugeordnet sind, so dass sich mehrere Emitter-Detektoren Paare ergeben, die hinsichtlich ihres Abstandes miteinander verglichen werden können. Wenn dies für eine oder mehrere Wellenlängen erfolgt, kann über eine spezielle Verrechnung der Messwerte die Konzentration des Analysten bestimmt werden. Ein Beispiel für solch eine Anordnung ist in DE 4427101 (Hein) gezeigt.

Es werden oft unterschiedlich lange Lichtwege gemessen. Aus dem Vergleich der Messergebnisse kürzerer und längerer Lichtwege kann auf die Eigenschaften an einem Ort in der zu untersuchenden Probe geschlossen werden.

Diese Vorgehensweise ist bekannt und unter SRR-Methode veröffentlicht.

Gängig ist die Vorverarbeitung von Messwerten wie z.B. durch Differenzmessungen zwischen Hell- und Dunkelmesswert bei getakteter Beleuchtung oder durch Mittelung mehrerer Messwerte.

Gängig sind dabei auch Verfahren die die gemessenen Lichtintensitäten mit $1/r^2$ gewichten (r=Abstand zwischen LED und Lichtempfänger) oder die Lichtintensität logarithmisch gewichten.

Gängig ist auch der Vergleich verschiedener Wellenlängen durch Subtraktion oder Quotientenbildung.

Gängig ist auch die gewichtete Summation so erhaltener aufbereiteter Messwerte um daraus auf Konzentrationen bestimmter Stoffe in der zu untersuchenden Probe rückschließen zu können.

Gängig ist auch die Bestimmung von Gewichtungsfaktoren des Auswertealgorithmus, um die Ausgabewerte eines Auswertungsalgorithmus bei gemessenen Rohdaten an gemessene Referenzdaten anzupassen.

Gängig ist auch, dass solche Referenzdaten sowohl von Probanden, als auch von künstlichen Phantomen stammen können.

Gängig ist auch die Übertragung von Kalibrierdaten eines Messsystems mit Hilfe von Hautphantomen auf ein zweites Messsystem.

Die genannten Verfahren konnten bisher zur Messung an z.B. Gewebe menschlicher Haut noch keine weitreichende kommerzielle Anwendung finden. Dies liegt darin begründet, dass die Qualität der Messmethode noch nicht ausreicht. Zur Lösung sind bereits einige Ansätze vorgeschlagen. Durch eine spezifische Verrechnung versucht z.B. US 7,139,076 (Marbach) die Oberflächeninhomogenität des Gewebes zu eliminieren. Dabei wird davon ausgegangen, dass am Einstrahlungspunkt das Licht rotationssymmetrisch für alle Raumwinkel ins Gewebe eingestrahlt wird und vom Detektor in gleicher Weise empfangen werden kann.

Durch gezielte Einstrahlung des Lichts in das Gewebe, mit einem Einfallswinkels von 5° bis 85° zu einem Lot auf die Oberfläche des Gewebes, erreicht DE 10163972 verbesserte Homogenitätsbedingungen für die Messung und damit ein genaueres Messresultat. Die WO 94/10901 A1 offenbart ein Verfahren und eine Vorrichtung zur Analyse von Glucose in einer biologischen Matrix. Es wird davon ausgegangen, dass die Änderung der Glucosekonzentration zu einer Änderung des Brechungsindex der in der biologischen Matrix enthaltenen Flüssigkeit führt. Diese Änderung des Brechungsindex führe zu einer Änderung der Lichtstreuung. Diese Veränderung wird gemessen, indem eine Vielzahl von Streuprozessen vieler Photonen, die einen untereinander ähnlichen Lichtweg in der biologischen Matrix durchlaufen haben, erfasst (Seite 12f).

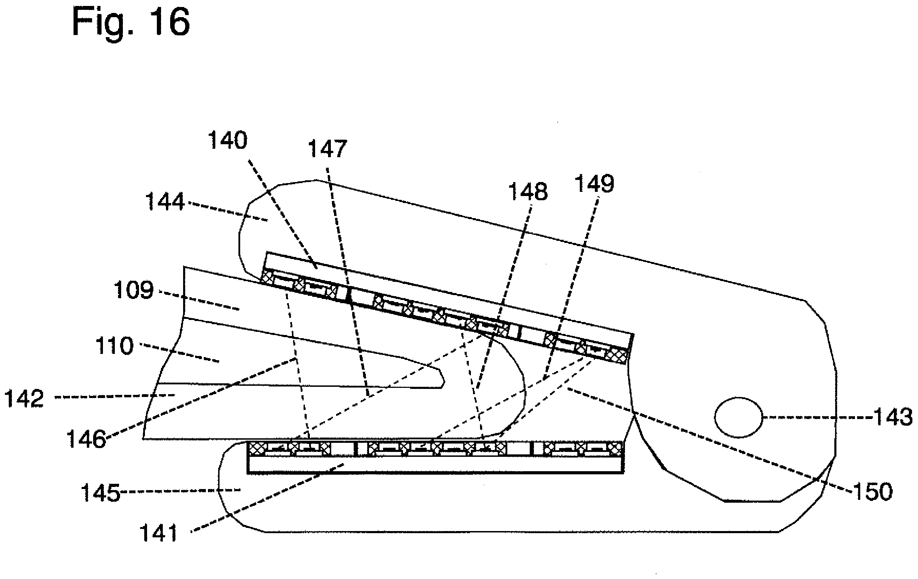

Auf diese Weise kann eine Konzentrationsänderung über einen längeren Zeitraum durch kontinuierliche Messung (etwa Figur 16) erfolgen. Es ist ausgeführt, dass, obwohl Messungen bei einer Wellenlänge ausreichten, es sinnvoll sein könne, bei weiteren zusätzlichen Wellenlängen zu messen (Seite 22). In Figur 21 ist die abwechselnde Anordnung von Lichtsendern und Lichtempfängern auf einer quadratischen Grundfläche gezeigt. Hierbei sind Halbleiterlichtsender und Halbleiterlichtempfänger für eine erste Wellenlänge gezeigt und Halbleiter-Lichtsender und Halbleiter-Lichtempfänger für eine zweite Wellenlänge. Während WO 94/10901 (Simonson S.19) davon ausgeht, dass der Detektionswinkel für die Messung keine Bedeutung hat, spricht DE 10163972 davon, dass sich das Ergebnis verbessert, wenn auch die Detektion unter einem entsprechenden Austrittswinkel aus dem Gewebe erfolgt.

Die WO 94/10901 A1 beschreibt ein Verfahren zur Bestimmung der Konzentration von Glucose in einer biologischen Matrix. Das Verfahren umfasst mindestens zwei Detektionsmessungen, bei denen jeweils Licht als Primärlicht in die biologische Matrix eingestrahlt wird, das Licht in der biologischen Matrix entlang einem Lichtweg propagiert und eine als Sekundärlicht austretende Lichtintensität gemessen wird. Das Verfahren umfasst zudem einen Auswerteschritt, bei dem aus den Intensitätsmeßwerten der Detektionsmessungen mittels eines Auswertealgorithmus und einer Kalibration die Glucosekonzentration abgeleitet wird. Eine gute Analysegenauigkeit soll mit einfachen Mitteln dadurch erreicht werden, dass mindestens eine Detektionsmessung eine ortsaufgelöste Messung von vielfach gestreutem Licht ist, bei der das Primärlicht an einem definierten Einstrahlungsort in die biologische Matrix eingestrahlt wird, die Intensität des an einem definierten Detektionsort aus der biologischen Matrix austretenden Sekundärlichts gemessen wird und der Detektionsort relativ zu dem Einstrahlungsort so angeordnet ist, dass an Streuzentren der biologischen Matrix vielfach gestreutes Licht detektiert wird, dessen Intensität für die Konzentration der Glucose charakteristisch ist.

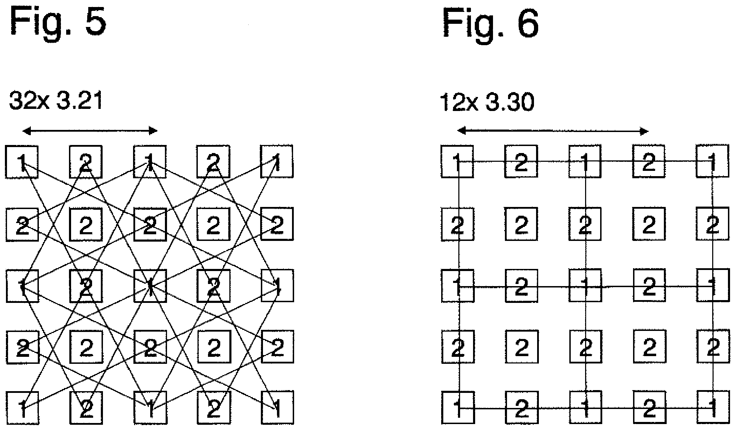

Die US 2008/232653 A1 beschreibt eine Methode, eine biometrische Funktion bereitzustellen. Spektrale Information kann detektiert werden. Beispielsweise können bei einem festen Abstand zwischen einer Lichtquelle und einem Bildgeber verschiedene Frequenzenkompositionen erhalten werden, durch den Gebrauch verschiedener Beleuchtungswellenlängen, Polarisationen, Filter und andere Beleuchtungseigenschaften. Auch textorale Information kann detektiert werden. Es ist auch ein Fingerabdrucksensor offenbart. Figur 5 zeigt drei verschiedene, nicht auf einer Linie angeordnete Lichtquellen und drei verschiedene, nicht auf einer Linie angeordnete Detektoren. Es ist erwähnt, dass reine spektrale Informationen zur Beschaffung der biometrischen Funktion verwendet werden kann, um ein Individuum zu identifizieren.

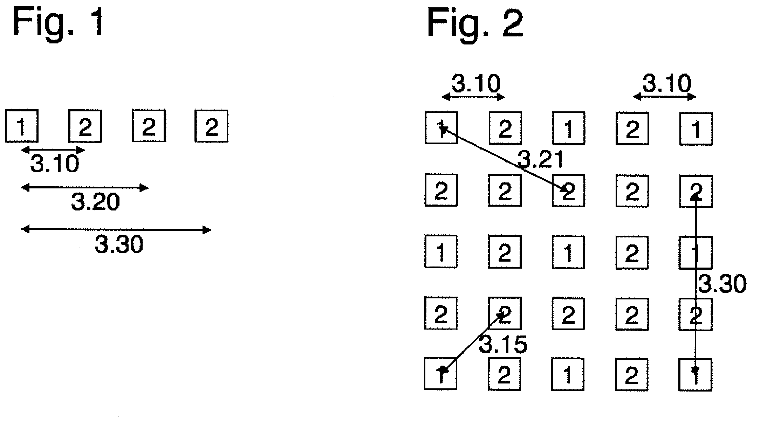

Die US 2002/058865 A1 offenbart ein System und eine Methode zur Messung des Sauerstoffgehalts des Bluts. Als Stand der Technik ist zunächst eine Methode offenbart, bei der eine Verzögerung zwischen dem Eintritt und dem Austritt von elektromagnetischen Wellen berücksichtigt wird, sowie eine weitere Vorgehensweise, bei der die Beeinflussung der Frequenz der elektromagnetischen Wellen durch die Probe, etwa eine Phasenverschiebung, gemessen wird. Als nachteilig bei diesen Methoden wird genannt, dass diese zu aufwändig seien und zu sperrige Vorrichtungen benötigen würden. Daraufhin wird eine weitere Methode dargestellt, bei der weder eine zeitliche Verzögerung noch eine Auswirkung auf die Frequenz berücksichtigt werde, sondern "lediglich" die Intensität der eingestrahlten und empfangenen elektromagnetischen Wellen gemessen werden. Als nachteilig hierbei wird dargestellt, dass bisher lediglich relative Werte oder Veränderungen und nicht absolute Werte ermittelt werden könnten. Dieses System solle erfindungsgemäß dadurch verbessert werden, dass eine Strahlungsquelle mit mindestens zwei Sets von Strahlung mit verschiedenen Wellenlängencharakteristiken verwendet werde. Die verschiedenen Sets der elektromagnetischen Wellen könnten sich durch verschiedene Wellenlängen unterscheiden. Es ist (etwa in Figur 1) offenbart, Lichtquellen und Lichtdetektoren auf einer Linie anzuordnen. Es ist auch erwähnt, dass die in Figur 1 gezeigte Anordnung mehrfach untereinander angeordnet sein könne.

Die WO 2010/056973 A1 offenbart eine Vorrichtung zur "invivo" optischen Prüfung und Überwachung von ausgewählten Stoffen im Blut und zu diesem Zwecke das Durchführen von ausgewählten Wellenlängen des Lichts in einem bestimmten Bereich des biologischen Gewebes und Empfangen des sich ergebenen Lichts, das aus einer Ebene austritt, sowie das Analysieren des empfangenen Lichts zur Bestimmung der gewünschten Daten basierend auf Lichtabsorption. Es ist erwähnt, dass mehr als zwei Emitter und mehr als zwei Detektoren vorgesehen sein können.

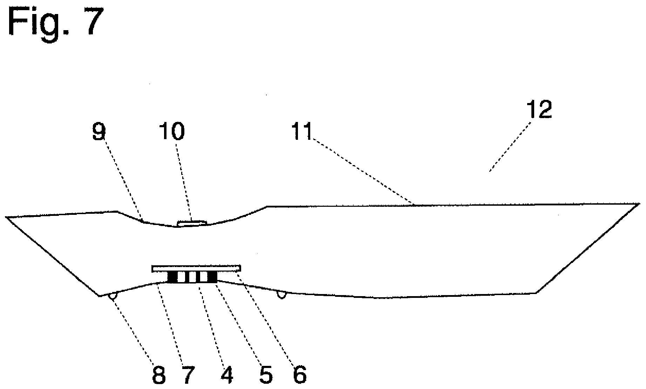

Die US 2005/020892 A1 betrifft eine Messung von biologischen Parametern durch Nahe-Infrarotspektroskopie. Als bevorzugter Analyt ist Glucose genannt. Interferenzen der Fettschicht sollen vermieden werden. Durch Limitierung des Abstandes von Beleuchtungs- und Detektionsfasern soll die Eindringtiefe des Lichts optimiert werden. Es ist ein optisches System offenbart, das aus mehreren Sonden zusammengesetzt ist. Figur 7 zeigt, dass jede dieser Sonden beispielsweise 22 optische Wege als Beleuchtungsfasern und 10 Detektionsfasern aufweisen kann.

Insbesondere hinsichtlich der Wiederholgenauigkeit bei aufeinander folgenden Messungen stellen die Inhomogenität von Gewebe und Oberflächenstruktur, sowie die nicht homogene Verteilung der Analyten in der Probe ein Problem dar, dass mit den bekannten Verfahren und Apparaten nicht gelöst ist. Beim erneuten Auflegen der Vorrichtung auf das zu messende Gewebeareal findet automatisch eine Variation des Messortes statt, die zu Messabweichungen führt. Aufwendige Messortbestimmungs- und wieder Auffindungstechnik, die ein Platzieren der Vorrichtung auf einer früheren Messposition ermöglicht, sind technisch kompliziert und teuer. Ihr Einsatz ist deshalb ausschließlich unter Laborbedingungen sinnvoll und möglich. Für die Alltagsnutzung stellen sie keine Alternative dar. Bei Wiederholungsmessung werden in der Alltagsnutzung auch Gewebestellen mit anderer Konzentration des Analyten und veränderten Streuzentren erfasst. Diese Variation führt auch bei exakter Konzentrationsbestimmung zwangsläufig zu einem veränderten Messergebnis. Ein der Inhomogenität der Verteilung des Analyten geschuldetes Messergebnis kann nicht mit früheren Messergebnisse verglichen werden. Die Frage, ob die Konzentration des Analyten zu oder abgenommen hat oder konstant geblieben ist, ist deshalb nicht eindeutig beantwortbar. Bei medizinischen Problemstellungen ist aus dem Ergebnis solcher Messreihen deshalb keine Diagnose ableitbar. Eine Überwachung von Konzentrationswerten ist mit einem solchen Gerät daher nicht sinnvoll.

Weitere Probleme liegen in der Anisotropie der untersuchten Probe. Sowohl Oberflächenstruktur, Zellstruktur, als auch z.B. Blutgefäße stellen Ursachen für die Anisotropie der Probe dar. Bei einem Großteil der bekannten Vorrichtungen erhält man Messwerte, die vom Winkel der Auflage der Vorrichtung auf die Probe abhängen. Auch dies führt unter Alltagsbedingungen zu Messabweichungen.

Weitere Probleme liegen darin, dass zur Detektion eines bestimmten Stoffes in z.B. menschlichem Gewebe mit einer erheblichen Anzahl von Stoffen gerechnet werden muss, die ebenfalls eine Auswirkung auf die optischen Signale, die zur Detektion genutzt werden aufweisen. Diese Substanzen tragen ebenfalls zum Verfälschen des Messergebnisses bei. Die Menge dieser Substanzen in der Probe kann stark und auch sehr schnell variieren. So ist z.B. bei stärkerem oder schwächerem Andruck der Probe an die Vorrichtung mehr oder weniger Blut im Gewebe, was bei bekannten Analyseverfahren zu einer deutlichen Druckabhängigkeit der Messung führt.

Benötigt wird deshalb eine Vorrichtung und ein Verfahren, welches ein weitestgehend korrektes und stabiles Messergebnis für die Konzentration des oder der relevanten Analyten bestimmt. Dabei müssen Randbedingungen wie die Inhomogenität der Probe, die Anisotropie der Probe, das Vorhandensein einer Vielzahl von Substanzen in der Probe und variierende Messumgebungsbedingungen toleriert werden. Die Unterschiede zwischen wiederholten Messungen müssen so gering sein, dass sie sich nicht signifikant auf das Messergebnis auswirken.

## Aufgabe der Erfindung

Aufgabe der vorliegenden Erfindung ist es, durch das erfindungsgemäße Verfahren und die erfindungsgemäße Vorrichtung die Konzentrationsbestimmung eines Analyten unter im Alltag erreichbaren Bedingungen durchzuführen, die ihre Repräsentativität für die Probe auch bei erneuten Auflegen der Vorrichtung auf die Probe nicht verlieren und bei der die Bestimmung der Konzentration mit guter Reproduzierbarkeit erfolgen kann. Weiterhin soll das System technisch unaufwändig, tragbar und kostengünstig produzierbar sein.

## Lösungsansätze

Stellt die Inhomogenität einer Probe ein Problem dar, so wird dies vom Fachmann oft gelöst, indem mehrfache Messungen an unterschiedlichen Orten der Probe durchgeführt werden. Dies kann durch sukzessive Messungen erfolgen, was oft zu hohem zeitlichen Aufwand für die Messung führt, der wirtschaftlich oder aus Komfortgründen nicht tragbar ist. Eine Lösung ist es mehrere Emitter und Detektoren in ein Messgerät zu integrieren, die einfach nebeneinander gesetzt werden. Auch für den hier genannten Anwendungsfall kann man entsprechende Veröffentlichungen finden.

Zur Berücksichtigung der Inhomogenität in der Tiefe der Probe gibt es Ansätze, die über unterschiedliche Abstände zwischen Lichtemittern und Lichtdetektoren arbeiten, die aber weiter verbessert werden müssen, um einsetzbar zu sein.

Zur Lösung des Problems der Anisotropie wären mehrere Messungen unter unterschiedlichen Winkeln eine mögliche Lösung.

Zur Detektion von Störsubstanzen ist eine Beleuchtung mit einer größeren Anzahl monochromatischer Lichtwellenlängen bekannt. Auch eine spektroskopische Untersuchung des aus der Probe austretenden Lichts ist eine bekannte Lösung, um den Einfluss von Störsubstanzen zu ermitteln.

Die Kombination dieser Techniken ist jedoch nicht möglich, da dazu eine Vielzahl von Messstrecken benötigt werden würde. Da die Vorrichtung zur Analyse der Probe jedoch nicht größer sein kann, als die Probe selbst, und da Detektoren und Emitter zur Funktion gewisse Flächen aufweisen müssen, gibt es bisher trotz der Miniaturisierungsfortschritte der Mikroelektronik keine bekannte Lösung eine ausreichende Anzahl von Messweglängen, Lichtemittern unterschiedlicher Wellenlängen und unterschiedliche Orientierungen der Messwege in die zur Messung benötigte Vorrichtung zu integrieren.

Die hier erläuterte Erfindung beschreibt, wie das Problem gelöst werden kann und welche Verfahren zum Betrieb der Vorrichtung angewendet werden können.

## Übersicht über die erfindungsgemäße Lösung

Die erfindungsgemäße Lösung betrifft eine Vorrichtung zur Bestimmung einer Konzentration eines Analyten in einer Probe sowie ein Verfahren zur Bestimmung einer Sauerstoffsättigung in der Probe unter Verwendung dieser Vorrichtung. Die Vorrichtung weist die Merkmale des Anspruchs 1 auf und das Verfahren die Merkmale des Anspruchs 33.

Im Rahmen dieses Dokuments werden die Begriffe "Photonen" und "Licht" synonym verwendet.

Durch eine geeignete flächige Anordnung von Lichtemittern und Detektoren gelingt eine mehrfache Nutzung von Komponenten. Diese Mehrfachnutzung ermöglicht mit einer geringeren Gesamtzahl von Emittern und Detektoren auszukommen.

Erfindungsgemäß wird durch die Anordnung der Komponenten in der Fläche erreicht, dass eine große Zahl verschiedener Abstände zwischen Emitter und Detektor mit einer relativ geringen Anzahl Komponenten erreicht werden kann. Es wird erreicht, dass die optischen Eigenschaften an mehreren, signifikant unterschiedlichen Stellen ermittelt werden können. Und es wird erreicht, dass die Messung in verschiedenen Orientierungen durchgeführt werden kann. Weiterhin wird eine hohe spektrale Auflösung bei reduzierter Anzahl von Komponenten erreicht.

Durch Verwendung einer erfindungsgemäßen Messschaltung wird eine weitere Erhöhung der Informationsmenge erreicht, ohne, dass die Zahl optischer Komponenten erhöht werden muss. Die Schaltung erlaubt durch ihren sehr hohen Dynamikbereich die Nutzung längerer und kürzerer Lichtwege, als mit bekannten Schaltungen von vergleichbarem technischem Aufwand möglich ist, wodurch bei gleicher Zahl an Lichtemittern und Lichtdetektoren die Zahl benutzbarer Lichtemitter und Lichtdetektorpaare steigt.

Durch vorteilhafte optische Geometrie wird die Informationsmenge weiter gesteigert und somit das Messergebnis weiter präzisiert. Durch richtungsabhängig variable Abstrahlcharakteristik lässt sich auch bei in der Tiefe der Probe variierender Konzentration eine Bestimmung des Analyten vornehmen. Dadurch lässt sich das Messergebnis weiter verbessern.

Mit einem Verfahren zur Kombination von Emittern unterschiedlicher Wellenlängencharakteristik mit Detektoren unterschiedlicher Wellenlängencharakteristik wird bei geringer Bauteilzahl eine hohe spektrale Auflösung der Messung erreicht. Damit ist es möglich den Einfluss von Substanzen in der Probe, die den Messwert beeinflussen, zu reduzieren.

Weiterhin wird ein Verfahren erläutert, welches die Auswertung der genannten Vielzahl an Information in vorteilhafter Weise implementiert.

Durch Kombination des Auswerteverfahrens mit der erfindungsgemäßen Ausgestaltung der Messvorrichtung lässt sich ein auch unter Alltagsumgebungen praktikabel anwendbares Messsystem realisieren. Durch Kombination der genannten Verfahren ist es überraschender Weise möglich sämtliche für eine wiederholbare Genauigkeit der Messung nötigen Komponenten auf der zur Verfügung stehenden durch die Probe begrenzten Fläche unterzubringen. Das erfindungsgemäße Messsystem ist dabei sogar so klein und kompakt, dass es in Alltagsgegenstände integriert oder auch als tragbares Taschengerät eingesetzt werden kann.

In vorteilhafter Variante lässt sich das System als elektronische Leiterplatte aufbauen und benötigt keine Lichtleitfasern. Es ist somit klein und kostengünstig realisierbar.

Für den Fachmann überraschend ist, dass zwischen den genannten Ansätzen eine Symbiose entsteht, die bei einer geringfügigen Erhöhung der Komponentenzahl noch keine signifikante Auswirkung auf das Gesamtsystem hat. Wird ein System gemäß der nachstehend erläuterten Regeln entworfen, so zeigt sich jedoch, dass die Symbiose für größere Komponentenzahlen überproportional zunimmt und die eingangs erläuterten Forderungen dadurch auf der für die Optoelektronik verfügbaren Fläche gelöst werden können.

Die gezielte Erzeugung dieser Symbiose und die dadurch entstehende Möglichkeit eine in Bezug auf die Zahl der verwendeten Emitter und Detektoren eine in Quantität und Qualität überproportional ansteigende Informationsbasis zu erzeugen sind zusammen mit der Methode diese Informationsbasis auszuwerten der wichtigste Grundgedanke der vorliegenden Erfindung. Um die angestrebte Symbiose zu erreichen ist es vorteilhaft eine weiter unten im Text angegebene Komponentenzahl zu überschreiten und die angegebenen geometrischen Anordnungsregeln einzuhalten.

Im Folgenden werden die im voranstehenden Text angesprochenen Lösungen ausführlich erläutert.

Erfindungsgemäß umfasst die Vorrichtung mindestens drei Emitter in einer flächigen Anordnung, die sich nicht auf einer Linie befinden und mindestens drei Detektoren in einer flächigen Anordnung, die sich nicht auf einer Linie befinden und die Emitter und/oder die Detektoren weisen mindestens drei unterschiedliche Wellenlängencharakteristika auf.

In bevorzugter Variante sind jeweils mindestens sechs Emitter in einer flächigen Anordnung vorgesehen, die sich nicht auf einer Linie befinden und weiter bevorzugt mindestens sechs Detektoren in einer flächigen Anordnung, die sich nicht auf einer Linie befinden und die Emitter und/oder die Detektoren weisen vorzugsweise mindestens drei unterschiedliche Wellenlängencharakteristika auf.

In alternativer bevorzugter Variante sind jeweils mindestens 50 oder 100 Emitter in einer flächigen Anordnung vorgesehen, die sich nicht auf einer Linie befinden und bevorzugt mindestens vier oder acht Detektoren in einer flächigen Anordnung, die sich nicht auf einer Linie befinden und die Emitter und/oder die Detektoren weisen bevorzugt mindestens drei oder fünf unterschiedliche Wellenlängencharakteristika auf.

Bevorzugt umfasst die Vorrichtung mindestens zwei oder drei Detektoren unterschiedlicher Wellenlängencharakteristik und mindestens zwei oder drei Emitter unterschiedlicher Wellenlängencharakteristik. Die Wellenlängencharakteristika sind bevorzugt derart breitbandig, dass durch Kombination von verschiedenen Emittern mit verschiedenen Detektoren mehr unterschiedliche Wellenlängencharakteristika erzielt werden, als die größere Anzahl von Detektoren oder Emitter unterschiedlicher Wellenlängencharakteristika.

Bevorzugt umfasst die Vorrichtung Paare, die jeweils einen Emitter und einen Detektor haben, die etwa einen gleichen Abstand zwischen Emitter und Detektor und etwa die gleiche Wellenlängencharakteristik aufweisen. Bevorzugt werden mit dem Begriff "gleiche Emitter-Detektorpaare" oder "gleiche Paare" oder "Gruppe gleicher Paare" im Rahmen dieser Druckschrift mehrere Emitter-Detektorpaare bezeichnet, die jeweils genau einen Emitter und genau einen Detektor aufweisen und deren Emitter-Detektor-Abstand etwa übereinstimmt und die etwa die gleiche Wellenlängencharakteristik aufweisen.

Die Begriffe "gleiche Paare" und "vergleichbare Paare" werden im Rahmen dieser Druckschrift synonym verwendet.

Bevorzugt sind die Emitter und/oder Detektoren in mindestens zwei bevorzugt mindestens sechs solcher Paare Mitglied.

Bevorzugt ist oder sind mindestens ein Emitter und/oder Detektor oder mehrere Emitter und/oder Detektoren oder alle Emitter und/oder Detektoren in mindestens zwei oder mindestens sechs verschiedenen Gruppen gleicher Paare Mitglied.

Bevorzugt umfasst die Vorrichtung gleiche Emitter-Detektorpaare deren Verbindungslinien nicht ausschließlich parallel verlaufen.

Bevorzugt umfasst die Vorrichtung Emitter-Detektorpaare - bevorzugt zumindest auch gleiche Emitter-Detektorpaare - bei denen die Orientierung der Verbindungslinien bei virtueller Kreuzung auf einer zweidimensionalen Fläche zu einem spitzen Winkel von 30° bis 45° oder vorzugsweise zu einem Winkel von >45° und besonders bevorzugt zu einem Winkel von 90° führt.

Bevorzugt werden die Emitter und Detektoren so angeordnet, dass für die relevanten Werte aus Abstand und Wellenlängencharakteristik eine ausreichende Abdeckung aller erforderlichen Orientierungen der Verbindungslinie zwischen Emitter und Detektor und/oder zwischen Detektor und Emitter gegeben ist. Besonders bevorzugt ist, wenn die Orientierungen sich an keinem Winkel um mehr als 180 Grad, besser 120 Grad noch besser 70 Grad unterscheiden. Besonders bevorzugt ist, wenn sich zwischen 3 und 5 besser zwischen 4 und 8 Orientierungen ergeben, die auf der Winkelskala etwa gleichmäßig verteilt sind. Da dies aufgrund der weiteren erfindungsgemäßen Randbedingungen nicht immer erreicht werden kann wird in einer bevorzugten Anordnung zumindest für einen Teil der Gruppen vergleichbarer Lichtwege eine Anordnung gewählt, bei der in jedem Winkelbereich einer festgelegten Winkelsektorbreite mindestens eine Verbindungslinie existiert, deren Orientierung in den Winkelbereich fällt. Die Breite eines solchen Winkelsektors beträgt höchstens 180 Grad, besser jedoch 120, 70, 50, 20 oder sogar 10 Grad.

Bevorzugt umfasst die Vorrichtung Emitter-Detektorpaare - bevorzugt zumindest auch gleiche Emitter-Detektorpaare - die einem flächenbasierten Abstandskriterium und/oder einem volumenbasierten Abstandskriterium und/oder einem orientierungsbasierten Abstandskriterium genügen. Bevorzugt genügen mehrere oder alle gleichen Emitter-Detektorpaare einem oder mehreren dieser Abstandskriterien.

Bevorzugt umfasst die Vorrichtung eine Einrichtung zur Beeinflussung der Abstrahlrichtung der von den Emittern emittierten Photonen.

Bevorzugt umfasst die Vorrichtung eine Einrichtung, mit der die Beeinflussung der Empfangsrichtcharakteristik der Detektoren erfolgt.

Die Vorrichtung umfasst Mittel, die bewirken, dass die von den Emittern emittierten Photonen und/oder die von den Detektoren erfassten Photonen nicht rotationssymmetrisch emittiert und/oder detektiert werden.

Bevorzugt ist die Vorrichtung derart ausgebildet, dass die von den Emittern emittierten Photonen im Wesentlichen in einem Winkel zwischen 5° und 90° zu der Ebene, in der sich die Emitter und die Detektoren befinden, emittiert werden und im Wesentlichen unter einem Winkel zwischen 5° und 90° zu der vorgenannten Ebene von den Detektoren empfangen werden.

Bevorzugt umfasst die Vorrichtung Emissions- und Detektionswinkel, die durch eine rechteckige Geometrie von Emittern, Detektoren und/oder Photonenleitelementen hervorgerufen sind.

Bevorzugt ist die Vorrichtung derart ausgebildet, dass die unterschiedliche Richtcharakteristik durch unterschiedliche Orientierungen der Bauelemente und/oder Chips der Emitter und/oder Detektoren zur durch die Emitter und Detektoren aufgespannten Ebene erzielt ist.

Bevorzugt ist die Vorrichtung derart ausgebildet, dass die unterschiedliche Richtcharakteristik durch Brechung und/oder Beugung und/oder Spiegelung und/oder Abschattung der emittierten und/oder detektierten Photonen erzielt ist.

Bevorzugt ist die Vorrichtung derart ausgebildet, dass für mindestens eine Wellenlänge die Abstände zwischen den für diese Wellenlänge vorgesehenen Detektoren und Emittern im Bereich der zur Nutzung ausreichend kurzen Abstände zwischen Detektor und Emitter in einer feineren Abstufung angeordnet sind, als für eine andere Wellenlänge, wobei die Abstände um den Faktor 0,9 oder 0,8 oder 0,6 oder 0,3 kleiner sind als bei der anderen Wellenlänge.

Bevorzugt umfasst die Vorrichtung optoelektronische Komponenten, die derart angeordnet sind, dass sich Lichtwellenlängenunterschiede für eine Wellenlänge ergeben, die kleiner sind als die Detektorgröße, die Emittergröße oder der Abstand von Detektor zu Emitter, insbesondere auch so, dass sich Lichtweglängenunterschiede ergeben, die nur 50 % oder 20 % oder 10 % oder sogar 5 % dieser Maße ergeben.

Bevorzugt umfasst die Vorrichtung optoelektronische Komponenten, die derart angeordnet sind, dass sich Abstände zwischen Detektoren und Emittern ergeben, deren Längenunterschiede kleiner sind als der doppelte Detektorabstand, als der einfache Detektorabstand oder als 55 % des Detektorabstandes.

Bevorzugt umfasst die Vorrichtung optoelektronische Komponenten, die derart angeordnet sind, dass die Anzahl signifikant unterschiedlicher Abstände zwischen Detektoren und Emittern größer ist, als die Anzahl der Detektoren oder die Anzahl der Emitter oder der Summe aus beiden.

Unter signifikant unterschiedlich wird verstanden, dass die Lichtwege sich um mindestens einen Betrag unterscheiden, der größer ist als der dichteste Abstand zwischen Detektoren untereinander oder der dichteste Abstand zwischen Emittern untereinander oder dem jeweils größeren dieser Werte oder der Summe dieser beiden Abstände. In vorteilhafter Variante ist als Abstand zwischen zwei Bauteilen der Abstand zwischen der Lichtein/-Austrittsöffnung in die Probe eines Bauteils und der Lichtein-/-Austrittsöffnung eines benachbarten Bauteils gemeint. In vorteilhafter Variante befindet sich in Bereich dieses Abstands intransparentes Material.

Bevorzugt ist die Vorrichtung derart ausgebildet, dass die Oberfläche der Anordnung nicht eben ist oder aus mehreren unterschiedlich orientierten Detektorelementen besteht.

Bevorzugt umfasst die Vorrichtung einige optische Komponenten, die beweglich gegenüber anderen optischen Komponenten angeordnet sind.

Bevorzugt ist die Vorrichtung derart ausgebildet, dass die Vorrichtung eine Kontaktfläche aufweist, die zur Messung direkt in Kontakt zu der zu messenden Probe gebracht werden kann.

Bevorzugt ist die Vorrichtung derart ausgebildet ist, dass sie zum Inkontaktbringen insbesondere mit biologischem Material, insbesondere mit menschlichem oder tierischem Gewebe geeignet ist. In vorteilhafter Variante besteht sie aus Material, welches leicht zu reinigen ist. In vorteilhafter Variante besteht sie aus Material, welches eine hohe chemische Beständigkeit aufweist.

Bevorzugt ist die Vorrichtung derart ausgebildet, dass zumindest ein Teil der Emitter mittels einer Ansteuerelektronik matrixförmig angesteuert ist.

Bevorzugt ist die Vorrichtung derart ausgebildet, dass zumindest ein Teil der Detektoren von einer Messelektronik matrixförmig angesteuert ist.

Bevorzugt ist die Vorrichtung derart ausgebildet, dass die Detektoren einen Dynamikbereich von zumindest 1:100, besonders bevorzugt von mindestens 1:50.000, besonders bevorzugt von mindestens 1:1.000.000 und weiterhin bevorzugt von mindestens 1:10.000.000 aufweisen.

Bevorzugt umfasst die Vorrichtung eine Auswerteelektronik, die die vorgenannte Dynamik ermöglicht.

Bevorzugt ist die Vorrichtung derart ausgebildet, dass die Emitter und/oder Detektoren Halbleiterbauelemente umfassen.

Bevorzugt ist die Vorrichtung derart ausgebildet, dass die Halbleiterbauelemente nicht weiter als 50 mm, vorzugsweise nicht weiter als 6 mm, und besonders bevorzugt weniger als 1,5 mm von der Probe entfernt sind, wenn sich die Vorrichtung in ihrer Messposition befindet.

Bevorzugt umfasst die Vorrichtung Sensoren und/oder Datenschnittstellen weiterer Umgebungsbedingungen und/oder physikalischer oder chemischer Parameter und/oder von Lebensumständen des zur Probe gehörigen Organismus.

Bevorzugt umfasst die Vorrichtung Mittel zur Beeinflussung der Mess- und/oder Umgebungsbedingungen, insbesondere Heiz-, Trocknungs- und/oder Anpresseinrichtungen.

Bevorzugt ist die Vorrichtung derart ausgebildet, dass sie in Einrichtungen integrierbar ist, die andere Funktionalitäten aufweisen und die zu der Gruppe der folgender Einrichtungen gehören: Smartphone, Haushaltsgerät, Körperwaagen, Fahrzeuglenkrad, Kleidungsstück, Schmuckstück, Werkzeug, Griff, Möbel, WC-Sitz, Eingabegerät.

In bevorzugter Variante wird die Vorrichtung in einen Gegenstand integriert, der vom Benutzer regelmäßig berührt wird.

Bevorzugt umfasst die Vorrichtung Mittel zur Positionierung relativ zu einer Probe.

Bevorzugt ist die Vorrichtung derart ausgebildet, dass sie die Detektion von Karotinoiden und/oder anderen Antioxidantien und/oder Blutinhaltsstoffen und/oder im menschlichen oder tierischen Gewebe vorkommende Substanzen und/oder physiologische Zusammenhänge und/oder flüssige und/oder feste und/oder gasförmige Substanzen ermöglicht.

Bei dem erfindungsgemäßen Verfahren zur optischen Detektion von Analyten in einer Probe, insbesondere bestehend aus tierischem oder menschlichem Gewebe, werden von zumindest drei flächig nicht auf einer Linie angeordneten Emittern Photonen in die Probe eingestrahlt. Ein aus der Probe austretender Teil dieser Photonen wird von mindestens drei flächig nicht auf einer Linie angeordneten Detektoren erfasst, wobei die genutzten Paarungen von Emittern und Detektoren mindestens drei unterschiedliche Wellenlängencharakteristika aufweisen und aus der Analyse der Messwerte der Detektoren mithilfe mathematischer Verfahren die Konzentration des Analyten bestimmt wird.

Bei dem Verfahren zur optischen Detektion von Analyten in einer Probe strahlen bevorzugt mindestens drei vorzugsweise flächig angeordnete Emitter Photonen in die Probe ein. Die Emitter haben bevorzugt unterschiedliche Wellenlängencharakteristika und befinden sich vorzugsweise nicht auf einer Linie. Die aus der Probe austretenden Photonen werden bevorzugt von mindestens drei Detektoren, vorzugsweise unterschiedlicher Wellenlängencharakteristika erfasst. Bevorzugt wird aus der Analyse der aus der Probe austretenden Photonen vorzugsweise mithilfe mathematischer Verfahren dann die Konzentration des Analyten bestimmt.

Bevorzugt ist weiterhin ein Verfahren bei dem Photonen, die von einem Emitter - bevorzugt genau einem Emitter - emittiert werden von mindestens zwei Detektoren unterschiedlicher Wellenlängencharakteristik erfasst werden und/oder die von zumindest zwei Emittern unterschiedlicher Wellenlängencharakteristika emittierten Photonen von genau einem Detektor empfangen werden.

Bevorzugt ist weiterhin ein Verfahren, das zur Konzentrationsbestimmung des Analyten die von den Detektoren erfassten Photonen unterschiedlicher Emitter-Detektorpaare, die etwa gleichen Abstand und etwa gleiche Wellenlängencharakteristik haben, miteinander verrechnet.

Bevorzugt ist weiterhin ein Verfahren, das zur Konzentrationsbestimmung des Analyten die von den Detektoren erfassten Photonen unterschiedlicher Emitter-Detektorpaare, die etwa gleichen Abstand und etwa gleiche Wellenlängencharakteristik und eine Richtcharakteristik mit vergleichbarer Wirkung haben, miteinander verrechnet.

Bevorzugt ist weiterhin ein Verfahren, das repräsentative Werte für Emitter-Detektorpaare etwa gleichen Abstands und etwa gleicher Wellenlängencharakteristik und in vorteilhafter Variante zusätzlich mit einer Richtcharakteristik mit vergleichbarer Wirkung, ermittelt werden - bevorzugt für zumindest nahezu alle Emitter-Detektorpaare etwa gleichen Abstandes und etwa gleicher Wellenlängencharakteristik und/oder vergleichbarer Richtcharakteristik - und aus diesen Werten die Konzentration eines Analyten in der Probe bestimmt wird.

Bevorzugt ist weiterhin ein Verfahren, das die Konzentration des Analyten in der Probe an verschiedenen Orten unter Verwendung der von den Detektoren erfassten Photonen unterschiedlicher Wellenlängencharakteristika und/oder unterschiedlicher Lichtweglängen bestimmt. Die Gesamtkonzentration des Analyten wird dann unter Berücksichtigung der Messwerte für die verschiedenen Orte bestimmt.

Bevorzugt ist weiterhin ein Verfahren, bei dem die von zumindest zwei Emittern unterschiedlicher Wellenlängencharakteristika emittierten Photonen von mindestens zwei Detektoren unterschiedlicher Wellencharakteristika erfasst werden, so dass Messwerte entstehen, die eine Anzahl von Wellenlängencharakteristika aufweisen, die größer ist als die größere Anzahl oder die Summe der unterschiedlichen Wellenlängencharakteristika von den Emittern und den Detektoren.

Bevorzugt ist weiterhin ein Verfahren, bei dem Emitter-Detektorpaare etwa gleichen Abstands und etwa gleicher Wellenlängencharakteristik gemittelt oder miteinander verrechnet werden, die einem flächigen und/oder räumlichen und/oder orientierungsmäßigen Abstandskriterium genügen.

Bevorzugt ist weiterhin ein Verfahren, bei dem Messwerte mehrfach hintereinander aufgenommen werden, um Veränderungen und/oder Schwankungen in der Probe zu erfassen und/oder auszumitteln.

Bevorzugt ist weiterhin ein Verfahren, bei dem Lichtwege unterschiedlicher Länge zwischen den Emittern und den Detektoren und/oder unterschiedlichen Ortes und/oder unterschiedlicher Abstrahlcharakteristik verwendet und für die jeweiligen Lichtwege Messwerte aufgenommen werden. Bevorzugt wird durch Verrechnung der Messwerte dieser Lichtwege miteinander, auf die Eigenschaften der Probe in Abhängigkeit der Tiefe unter einem Punkt der Oberfläche geschlossen.

Bevorzugt ist weiterhin ein Verfahren, das die Einzelmesswerte einer Gruppe gleicher Paare miteinander vergleicht oder verrechnet und ein Maß für deren Schwankungsbreite ermittelt.

Bevorzugt ist weiterhin ein Verfahren, bei dem zumindest einige der Emitter und/oder Detektoren zur Ermittlung von Messwerten an mehr als einem Messort verwendet werden.

Bevorzugt ist weiterhin ein Verfahren, bei dem Gruppen von Paaren etwa gleichen Abstands und etwa gleicher Wellenlängencharakteristik gebildet werden, bei denen auch die Emissions- und Detektionswinkel der Emitter-Detektorpaare gleiche Charakteristika aufweisen.

Bevorzugt ist weiterhin ein Verfahren, bei dem die Belichtungszeit der Detektoren einem Vielfachen der halben Periode der Netzfrequenz entspricht.

Bevorzugt ist weiterhin ein Verfahren, bei dem die von einem Detektor erfasste Photonenmenge gemessen wird, indem der Strom durch den optischen Detektor gemessen wird, und/oder in dem eine Kapazität entladen wird und bei der die Höhe der Entladung und/oder die Anzahl der Entladungen in kurzer Zeit gemessen werden und/oder diese Messung mit der direkten Messung des Stroms kombiniert wird.

Bevorzugt ist weiterhin ein Verfahren, bei dem zumindest zwei der zuvor genannten Verfahren zur Messung des Stroms verwendet werden.

Bevorzugt ist weiterhin ein Verfahren, bei dem die Auswahl der Messverfahren, mit denen der Strom durch den Detektor gemessen wird, während der Messung selbsttätig erfolgt. Die Auswahl der Messverfahren erfolgt, bevorzugt ohne dass vor Beginn der Messung die Höhe des zu erwartenden Messwerts bekannt ist.

Bevorzugt ist weiterhin ein Verfahren, bei dem die Messung in jedem Messmodus über einen vom Messwert unabhängigen, vorab festgelegten Zeitraum erfolgt.

Bevorzugt ist weiterhin ein Verfahren, bei dem durch eine Abfolge von Messungen die Schwankungsbreite der Messwerte ermittelt wird, um die für die jeweilige Probe minimal nötige Anzahl von Messungen zu bestimmen, welche bei sukzessive ausgeführten Messzyklen die Abweichungen der Messergebnisse minimiert.

Bevorzugt ist weiterhin ein Verfahren, bei dem bei der Auswertung weitere Informationen wie beispielsweise Temperatur und Luftfeuchte der Umgebung, Temperatur des Sensors und/oder der Probe berücksichtigt werden.

Bevorzugt ist weiterhin ein Verfahren, bei dem bei der Weiterverarbeitung von mit dem Messverfahren gewonnener Werte Informationen mitverarbeitet werden, die die Probe und den zur Probe gehörenden Organismus betreffen. Insbesondere werden Verläufe der Futter- bzw. Ernährungszusammensetzungen, der Menge sowie Verläufe der Belastungssituationen, Krankheiten oder Umgebungsbedingungen berücksichtigt.

Bevorzugt ist weiterhin ein Verfahren, bei dem das zu einer Probe gehörende Individuum durch Vergleich der an der Probe eines anderen Individuums gewonnen Messergebnisses mit einer gewissen Wahrscheinlichkeit von dem anderen Individuum unterschieden werden kann.

Bevorzugt ist weiterhin ein Verfahren, bei dem die Messergebnisse durch einen Algorithmus ermittelt werden und algorithmische Elemente und/oder Konstanten des Algorithmus durch iterative Berechnungen, vorzugsweise mit Hilfe einer Datenverarbeitungsanlage, ermittelt werden.

**Anordnung und Verteilung** von **Emittern und Detektoren**

Die Anordnung kann vorteilhafter Weise verschiedene Lichtemitter z.B. mit verschiedener spektraler Charakteristik aufweisen.

Die Anordnung kann vorteilhafter Weise verschiedene Detektoren z.B. mit verschiedener spektraler Charakteristik aufweisen.

Im Folgenden wird eine bevorzugte Regel zur Auswahl der Positionen von Emittern und Detektoren angegeben, nach der sich bestimmen lässt, wie dicht von unterschiedlichen Paaren von Emittern und Detektoren durchleuchtete Bereiche beieinander liegen dürfen, um den Informationsgewinn durch eine größere Anzahl solcher Paare hoch zu halten. Zweck der Regel ist es unter anderem, die Paarungen, die nach der Regel nicht erforderlich sind einzusparen, um den Bauraum für z.B. eine verbesserte spektrale Auflösung zu nutzen. Durch die gemeinsame geometrische Anordnung stehen die hier erläuterten Verfahren in sehr enger gegenseitiger Wechselwirkung und können nicht immer einzeln angewandt und optimiert werden. Um die Beschreibung übersichtlicher zu machen, müssen die Aspekte jedoch zunächst einzeln erläutert werden.

In einer vorteilhaften Lösung zur repräsentativen Erfassung der Inhomogenität einer Probe wird die Anordnung und Verteilung von Emitter-Detektoren Paaren etwa gleicher emittierter Wellenlängencharakteristik auf einer zweidimensionalen Fläche so gestaltet, dass es Emitter-Detektor Paare gibt, bei denen auch die Strecke, die der direkten Verbindungslinien zwischen Emitter und Detektor entspricht, etwa gleich ist. Wie bereits erwähnt werden derartige Emitter - Detektorpaare bevorzugt als gleiche Emitter- Detektorpaare bezeichnet. Eine etwa gleiche Wellenlängencharakteristik liegt beispielsweise vor, wenn die Unterschiede zwischen zwei Emittern nicht größer sind, als die produktionsbedingte Varianz für jeden Emitter. Als etwa gleiche Entfernung zwischen Emitter und Empfänger gelten im Rahmen dieser Druckschrift insbesondere Abstände von zwei Paaren, bei denen die Differenz zur mittleren Strecke beider Entfernungen max. 30%, besser max. 20%, noch besser max. 10% und idealer Weise 5% oder weniger beträgt. Einer Gruppe von Paaren etwa gleicher Wellenlängencharakteristik und etwa gleicher Entfernung können zwei oder mehr Emitter-Detektor Paare angehören. Je mehr Gruppen es gibt und je mehr Emitter-Detektor Paare einer Gruppe angehören, je besser kann die Inhomogenität der Probe erfasst werden.

In bevorzugter Variante wird die Definition, welche Emitter Detektor Paare als für die Zugehörigkeit zu einer Gruppe gleicher Paare als hinreichend gleich anzusehen ist erweitert, um Aspekte der Orientierung der Emitter/Detektor Verbindung und/oder der vorzugsweisen Orientierung der Bewegungsbahnen der Photonen und/oder um Aspekte der Richtcharakteristik der Emitter und Detektoren. Der sprachlichen Einfachheit halber wird in der Erläuterung im Folgenden meist nur auf etwa gleichen Abstand und Wellenlängencharakteristik eingegangen, da dem Fachmann nach der Erwähnung der oben genannten anderen Kriterien klar ist, wie diese analog anzuwenden sind.

Photonen, die vom Emitter in Richtung des Detektors wandern durchlaufen in der Probe einen dreidimensionalen Raum. Die Aufenthaltswahrscheinlichkeit an einem Ort in diesem Raum steht im Zusammenhang mit dem Abstand dieses Ortes von der direkten Verbindungslinie zwischen beiden. In einem großen Abstand zu dieser Linie sind weniger Photonen anzutreffen als in einem kleinen Abstand.

Bei den Untersuchungen zur repräsentativen Berücksichtigung der Inhomogenität einer Probe konnte überraschender Weise festgestellt werden, dass bei der Verteilung von Emitter und Detektoren etwa gleicher emittierter Wellenlängencharakteristik auf einer zweidimensionalen Fläche neben der Entfernung von Emitter und Detektor auch der von Photonen durchwanderten Raum berücksichtigt werden kann, um das Messergebnis zu optimieren.

Bei einer bevorzugten Lösung sollten sich die von Photonen durchwanderten Räume zu mind. 5%, besser zu mind. 20%, noch besser zu mind. 50%, noch etwas besser zu mind. 80% und in idealer Weise zu 95% **nicht** überlappen.

In einer bevorzugten Ausgestaltung der Erfindung werden die Bauräume, die sich durch die Anwendung des Abstandskriteriums für Bauteile ergeben genutzt, um Bauteile einer anderen Wellenlängencharakteristik zu positionieren.

Der Überlappungsgrad der Räume kann dabei wie folgt bestimmt werden:
Zunächst wird für jedes Raumelement der Probe die Aufenthaltswahrscheinlichkeit eines Photons für jeden zu betrachtenden Lichtweg zwischen Emitter und Detektor ermittelt. In vorteilhafter Variante wird dieser Wert anschließend auf eine Bezugsgröße bezogen. Dies kann z.B. die durchschnittliche Aufenthaltswahrscheinlichkeit eines zu dem gleichen Lichtweg gehörigen gleich großen Volumenelements der gesamten Probe, oder auch die maximale Aufenthaltswahrscheinlichkeit eines Photons auf einem Volumenelement der Mittelebene zwischen Emitter und Detektor oder auch eine Konstante sein. Aus den für die aus der Aufenthaltswahrscheinlichkeit gewonnenen Kennzahlen für jedes Volumenelement wird eine Kennzahl ermittelt, aus der entschieden wird, ob das Volumenelement zum Lichtweg gehörig ist, oder nicht. Diese Entscheidung kann binär sein, aber auch analog. Durch Volumenintegration über das Probenvolumen kann anschließend das Volumen des Lichtweges berechnet werden. Zum Vergleich zweier Emitter Detektor Paare wird das Volumen der Lichtwege für jedes Paar getrennt berechnet. Anschließend wird das Volumen des Überlappungsbereiches ermittelt. Dies kann beispielsweise durch Produktbildung der Zugehörigkeitskennzahlen der Volumenelemente zu den einzelnen Paaren erfolgen. Alternativ kann eine Tabelle genutzt werden, die die Zugehörigkeit zum Überlappungsbereich in Abhängigkeit von den beiden Zugehörigkeitskennzahlen in Bezug auf die beiden betrachteten Emitter Detektor Paare festlegt. Durch Volumenintegration erhält man die Größe des Überlappungsbereiches zwischen zwei Paaren.

Setzt man das Volumen des Überlappungsbereichs ins Verhältnis zum Volumen des Lichtweges eines der beiden Paare, so erhält man die Überlappung als prozentualen Wert. Auf diesen lassen sich die oben angegebenen Überlappungsgrenzwerte anwenden.

Wesentlich ist noch, dass die angegebenen Überlappungsgrenzwerte nicht nur für jedes einzelnen Lichtwegpaar angewendet werden können, sondern stattdessen in einer gemittelten Betrachtung über alle Lichtwege einer vergleichbaren Gruppe.

Die Überlappung eines Volumenelementes kann in vorteilhafter Variante dazu für die Paarungen von jedem Paar mit jedem anderen Paar festgestellt werden und anschließend zu einer Überlappungsgesamtkennzahl des Volumenelementes verrechnet werden. Dies kann z.B. durch Maximumbildung oder Summenbildung der paarweisen Analyse erfolgen. Durch Volumenintegration der Gesamtkennzahl ist die Größe des gesamten Überlappungsbereiches bestimmbar. Durch Volumenintegration der Volumina, die zu einem Lichtweg gehören erhält man eine Gesamtkennzahl für das Volumen der Lichtwege. Diese können zueinander ins Verhältnis gesetzt werden um einen Überlappungsgrenzwert zu erhalten, auf den das oben genannte Kriterium angewendet werden kann.

In Vorteilhafter Variante wird das Kriterium für ausgewählte Gruppen mit vergleichbaren Emitter Detektor Paaren einzeln durchgeführt.

In alternativer Vorteilhafter Variante wird das Kriterium für ausgewählte Gruppen mit vergleichbaren Emitter Detektor Paaren gruppenübergreifend durchgeführt.

Die oben beschriebene Methode zur Ermittlung der Überlappungskennzahl wird als volumenorientiertes Abstandskriterium bezeichnet.

In einer vorteilhaften Variante werden weiterhin die Bewegungsrichtungen der Photonen berücksichtigt. Sind diese deutlich unterschiedlich, so wird trotz hoher Aufenthaltswahrscheinlichkeit von Photonen verschiedener Lichtwege eine verringerte Überlappungskennzahl angesetzt. Dadurch lässt sich Anisotropie der Probe besser berücksichtigen, wie an anderer Stelle dieses Dokumentes erläutert wird. Diese Methode wird als orientierungsbasiertes Abstandskriterium bezeichnet.

In einem vereinfachten Verfahren zur Bestimmung und Überprüfung der Überlappung wird der dreidimensionale Raum, den die Photonen vom Ort der Einstrahlung zum Ort ihrer Detektion durchwandern, auf die Ebene projiziert, auf der sich die Emitter und Detektoren befinden. Für jedes Emitter-Detektor Paar wird auf dieser Ebene eine Fläche bestimmt, bei der alle Punkte der Fläche zu jedem Punkt auf der direkten Verbindungslinie einen Abstand von mindestens 0,2mm, besser von mindestens 1mm, noch besser von mindestens 2mm, noch etwas besser von mindestens 3 mm und idealer Weise von 5mm haben. Hinsichtlich der Überlappung werden nacheinander jeweils zwei Paare miteinander verglichen. Auf die Überlappungsflächen werden die oben genannten Grenzwerte angewendet.

In alternativer Variante werden die durchlaufenen Räume in der Fläche der Emitter und Detektoren durch geeignete Ellipsen repräsentiert.

Auch in diesem flächenbasierten Abstandskriterium kann die Überlappungskennzahl für jeweils zwei Lichtwege paarweise angewendet werden. Vorteilhafter ist jedoch den über die ganze Gruppe gleicher Paare gemittelten Wert zu betrachten.

Bei einer weiteren Untersuchung konnte überraschender Weise festgestellt werden, dass bei der Verteilung und Zuordnung der Emitter und Detektoren auf einer zweidimensionalen Fläche auch die Orientierung der direkten Verbindungslinie zwischen Emitter und Detektor für die Erfassung der Inhomogenität einer Probe von Bedeutung ist.

Bei einer vorteilhaften Ausführung erfolgt die Verteilung der Emitter und Detektoren auf dieser Fläche derart, dass Emitter-Detektor Paare entstehen, bei denen die Wellenlängencharakteristik etwa die gleiche ist, eine etwa gleiche Entfernung vorliegt, die Überlappung ihres von Photonen durchwanderten Raums in erfindungsgemäßer Art und Weise erfolgt und bei denen auch die Orientierung ihrer direkten Verbindungslinie etwa gleich ist. Eine gleiche geometrische Orientierung von Emitter-Detektor Paaren liegt vor, wenn die Strecken der direkten Verbindungslinien unter Beibehaltung ihrer Orientierung auf einer zweidimensionalen Fläche virtuell zur Kreuzung gebracht werden und der sich ergebende spitze Winkel maximal 30° beträgt. In einer besonders bevorzugten Lösung darf er maximal 10° betragen.

Bei einer weiteren vorteilhaften Ausführung werden Emitter-Detektoren Paare gleicher Orientierung mit Emitter-Detektoren Paaren einer anderen Orientierung verrechnet. Zu der Gruppe der anderen Orientierung gehören Emitter-Detektor Paare, die sich zwar hinsichtlich ihrer Entfernung und der emittierten Wellenlängencharakteristik nicht von den Paaren gleicher Orientierung unterscheiden, bei denen aber die virtuelle Kreuzung der Verbindungslinien zu spitzen Winkeln führt, die zwischen 30°-45° liegen, noch vorteilhafter über 45° betragen und idealer Weise nahezu rechtwinklig zueinander verlaufen. Der Vergleich ergibt ein repräsentatives Abbild der Anisotropie der Probe. Wie sich bei Versuchen überraschend herausstellte, bewirkt der Vergleich, dass eine hohe Wiederholgenauigkeit der Messungen erreicht wird, ohne dass die Vorrichtung zur Wiederholungsmessung am gleichen Ort des Messareals platziert werden muss.

Dieser Umstand ergibt sich aus folgendem Zusammenhang: Beim Auflegen des Messgeräts mit erfindungsgemäßer Verteilung und Orientierung von Emittern und Detektoren, propagiert das Licht entlang von Lichtwegen, die entsprechend der Emitter-Detektor Orientierung die Inhomogenität der Probe in unterschiedlichen Vorzugsrichtungen durchlaufen. Im biologischen Gewebe trifft das Licht infolge dieser Orientierungen mit unterschiedlichen Vorzugswinkeln auf Blutgefäße und Hautrillen. Dadurch ändert sich auch bei erneuten Auflegen der Vorrichtung mit einem möglicherweise anderen Winkel die Orientierung, in der das Licht das Gewebe durchläuft, nicht signifikant. Es ergibt sich daher bei geeigneter Auswertung auch bei Wiederholungsmessung ein vergleichbares Messergebnis.

Mit der Anzahl der Emitter und Detektoren, ihrer Packungsdichte auf zweidimensionaler Ebene, ihrer Orientierung sowie der Abstrahl- und Detektionscharakteristik der Emitter und Detektoren, steigt nach der vorliegenden Erfindung daher sowohl die Messgenauigkeit wie die Wiederholgenauigkeit der Konzentrationsbestimmung.

In vorteilhafter Ausgestaltung der Erfindung wird die Messung mit der beschriebenen Anordnung mindestens einmal vorzugsweise jedoch mehrfach wiederholt. Dies erfolgt in einer vorzugsweisen Variante in direktem Anschluss an die erste Messung, ohne dass die Probe entfernt wird. In einer anderen vorteilhaften Variante wird die Probe entfernt und neu aufgelegt, oder es wird eine weitere Probe aufgelegt. In einer dritten vorteilhaften Variante werden beide Methoden kombiniert.

Durch Vergleich oder Verrechnung der mehrfach erfolgten Messungen können veränderliche Bestandteile erkannt und gezielt ausgewertet werden. Ebenso ist die Unterdrückung veränderlicher Signalanteile durch Mittelung oder Verrechnung möglich. Mit diesem Verfahren ist beispielsweise die Erkennung der Pulsfrequenz in einer lebendigen Probe möglich, aber auch die Unterdrückung des durch den Puls verursachten Messfehlers. Durch erneutes Auflegen der Probe können Bedienungsfehler oder Ortsabhängigkeiten erkannt und kompensiert werden. Durch Messung unterschiedlicher Proben können Fehler der Proben erkannt und ausgeglichen werden.

Unter **Wellenlängencharakteristik** wird im Zusammenhang dieses Dokumentes insbesondere verstanden, dass ein optischer Emitter oder Detektor oder ein Stoff der von Licht durchlaufen wird Licht unterschiedlicher Wellenlänge unterschiedlich beeinflusst oder verarbeitet. Die Wellenlängencharakteristik beschreibt bevorzugt, welche Wellenlänge wie, insbesondere wie stark, beeinflusst wird.

Im Beispiel einer LED beschreibt die Wellenlängencharakteristik bevorzugt die Intensität des erzeugten Lichts in Abhängigkeit der Wellenlänge. Eine LED, die mit 700nm Emissionswellenlänge angegeben ist emittiert typischerweise auch bei 705nm oder 710nm Licht, allerdings typischerweise mit geringerer Intensität. LEDs können insbesondere gleiche angegebene Emissionswellenlänge aufweisen, sich aber z.B. in ihrer Wellenlängencharakteristik dahingehend unterscheiden, dass sie eine schmalere oder breitere Wellenlängencharakteristik aufweisen. Z.B. eine 700nm LED, die bei 750nm noch 50% der maximalen Emission aufweist im Vergleich zu einer, die bei 750nm nur noch 5% der maximalen Emission aufweist. Beispiele für Wellenlängencharakteristik sind in Bild 19 gezeigt und erläutert. Für Detektoren gilt entsprechendes.

Insbesondere ist in diesem Dokument unter Wellenlängencharakteristik eines Bauelements nicht nur die Charakteristik des eigentlichen Chips verstanden. Auch Filter, Farbstoffe, Vergussmaterialien, Montage- und Einbausituation, Einsatzumgebungen wie z.B. Temperatur und Alter sowie optisch wirksame Elemente wie Dünnschichten oder lichtbrechende Elemente erzeugen einen Einfluss auf die Wellenlängencharakteristik, der in diesem Zusammenhang soweit sinnvoll ebenfalls gemeint ist.

Im Zusammenhang mit einem Emitter Detektor Paar wird unter Wellenlängencharakteristik die gemeinsame Wirkung der Wellenlängencharakteristik des Emitters und des Detektors verstanden.

Einige Emitter können Licht unterschiedlicher Wellenlänge emittieren. Bekannt sind z.B. LEDs, die mehrere Chips für verschiedene Wellenlängen enthalten. Solche Emitter werden im Zusammenhang mit diesem Dokument bevorzugt als getrennte Emitter behandelt. In bevorzugter Variante wird dabei nur ein Emitter aktiviert. In alternativer vorteilhafter Variante werden mehrere Emitter gleichzeitig aktiviert. Dies ist z.B. günstig, wenn aufgrund geringer oder gut berechenbarer Überlappung der Wellenlängenbereiche die gleichzeitige Messung von mehreren Emitter Detektor Paaren eine parallele und somit schnellere Messung ermöglicht.

Einige Emitter sind in der Lage ihre Wellenlängencharakteristik zu verändern. Beispiele dafür sind z.B. LEDs, die bei unterschiedlichem Betriebsstrom unterschiedliche Wellenlängen emittieren. Ähnliches gibt es für Detektoren, die z.B. mit durchstimmbaren Filtern erhältlich sind. In bevorzugter Variante werden durchstimmbare Bauteile im Zusammenhang mit der vorliegenden Beschreibung so behandelt, dass die Wellenlängencharakteristik des Emitter Detektor Paares während der Zeitdauer, in der eine Teilmessung erfolgt zu einer festen Wellenlängencharakteristik gemittelt oder verrechnet wird. Die Zeitdauer einer Teilmessung ist dadurch definiert, dass in dieser Zeit aus dem Detektor keine zeitliche Abfolge von Messwerten gewonnen wird.

Ein durchstimmbarer Emitter oder Detektor kann somit virtuell zerlegt und auf den Grundgedanken dieser Erfindung zurückgeführt werden.

Unter **Richtcharakteristik** wird im Zusammenhang dieses Dokumentes insbesondere verstanden, welche Menge Licht in welche Richtung abgegeben oder empfangen wird. Die Zusammenhänge gelten grundsätzlich für Lichtemitter und Lichtdetektoren ähnlich, wie dem Fachmann bekannt ist, weswegen hier der Einfachheit wegen exemplarisch die Seite der Lichtemitter erläutert wird.

Die in diesem Dokument beschriebenen Lichtemitter emittieren das Licht in Abhängigkeit des Austrittswinkels in unterschiedlicher Intensität. Wichtig ist es, dass es sich bei den erfindungsgemäßen Ausführungsformen nicht um rotationssymmetrische Systeme handelt, wie man sie oft als Lichtemitter im Stand der Technik kennt. Bei der Richtcharakteristik handelt es sich deswegen um einen von 2 Winkeln abhängigen Wert, der die Stärke des in Richtung dieser Winkel abgesendeten Lichts charakterisiert. Beispielsweise eine prozentuale Angabe in Abhängigkeit des Orientierungswinkels in der Ebene der Lichtemitter und Lichtdetektoren und des Winkels zum Lot zu dieser Ebene, welches durch den Emitter oder Detektor verläuft.

Im Text wird die Bezeichnung Richtcharakteristik oft im Verhältnis zwischen Lichtemitter und Lichtdetektor gebraucht. In diesem Fall wird der Winkel in der Ebene vorteilhafterweise so angegeben, dass 0 Grad der Richtung auf das jeweilig andere Bauteil entspricht und Abweichungen von dieser Richtung im Bereich +-180 Grad erfasst werden.

Es kann vorteilhaft sein für eine vereinfachte Darstellung die Richtcharakteristik eines Lichtemitters auf einen Lichtdetektor oder umgekehrt als reine Funktion des Winkels zum Lot zur Emitter Detektor Ebene durch das Bauteil in Richtung des jeweiligen Partnerbauteils anzugeben. Noch stärker vereinfacht kann man dann von einer steilen oder flachen Richtcharakteristik in Richtung eines entsprechenden Partnerbauteils sprechen. Flach bedeutet insbesondere, dass relativ viel Licht so abgestrahlt, dass es zum Partnerbauteil gelangt ohne tiefere Schichten der Probe zu durchlaufen. Steil bedeutet insbesondere, dass das relativ viel Licht so abgestrahlt wird, dass es tiefere Schichten der Probe durchlaufen muss, um zum Detektor zu gelangen. Auch hier sind Emitter und Detektor bevorzugt vertauschbar. Weiterhin kann eine Richtcharakteristik auch im Paar zwischen Lichtemitter und Lichtempfänger verstanden werden.

Als Mittel zur Erzeugung einer Richtcharakteristik können intransparente Elemente z.B. aus Kunststoff oder Metall verwendet werden, die Licht abschatten. Diese können insbesondere auch schwarz sein oder schwarze Pigmente beinhalten, um Licht zu absorbieren. Weiterhin können Elemente verwendet werden, die Licht reflektieren. Dies können spiegelnde oder diffus reflektierende Oberflächen sein. Diese können insbesondere auch farbig sein, um für verschiedene Farben unterschiedliche Richtcharakteristik zu erzeugen. Die Oberflächen können auch gekrümmt sein. Weiterhin können transparente Materialien eingesetzt werden, um Licht zu leiten. Dies können insbesondere transparente Kunststoffe oder Gläser sein. Diese können auch Farbstoffe oder Farbpigmente enthalten. Insbesondere kann die Richtcharakteristik durch Einbringen von Körpern oder Pulvern, die das Licht streuen beeinflusst werden. Solche Körper können z.B. Glaskugeln, Farbpigmente, weiße Pigmente, weißes oder farbiges Pulver oder Paste, oder Luftblasen sein. Weiterhin kann die Richtcharakteristik durch Grenzflächen verschiedener Materialien und insbesondere durch deren Form oder Oberflächenbeschaffenheit beeinflusst werden. Beispielsweise wären hier Grenzflächen zwischen Gas und Glas, Gas und Kunststoff oder Glas und Kunststoff sowie verschiedener Gläser oder Kunststoffe zueinander denkbar. Insbesondere kann die Richtcharakteristik durch Linsen, Gitter, Fresnel-Linsen, Dünnschichten, Prismen oder Streuscheiben beeinflusst werden. Die Richtcharakteristik kann auch durch Lichtleiter beeinflusst werden. Weiterhin kann die Richtcharakteristik durch die Form, Größe und Orientierung des Emitters und/oder Detektors beeinflusst werden. Insbesondere ist dies durch die Form und Orientierung des Halbleiters möglich. Insbesondere ist dies auch durch einen Verguss des Halbleiters mit einem Kunststoff möglich. Insbesondere auch durch die Positionierung des Halbleiters relativ zu transparenten und intransparenten Materialelementen.

In vorteilhafter Ausgestaltung werden die intransparenten Materialien auch dazu genutzt dafür zu sorgen, dass nur Licht vom Emitter zum Detektor gelangt, welches durch die Probe gelaufen ist. Das intransparente Material bildet in vorteilhafter Variante eine Sperre für das Licht, die den Weg vom Emitter zum Detektor versperrt. In vorteilhafter Variante wird mindestens 50% besser mindestens 95% am besten jeder Weg versperrt, bei dem das Licht nicht mindestens durch die Probe, besser mindestens eine festgelegte Mindeststrecke durch die Probe laufen muss. In vorteilhafter Ausgestaltung beträgt diese Mindeststrecke mindestens 2% des Abstandes von Emitter zum Detektor, besser mindestens 20%, noch besser mindestens 50% am besten mindestens 80%. In vorteilhafter Variante wird diese Forderung zum Teil durch die Anordnung realisiert. In vorteilhafter Variante wird diese Forderung zum Teil durch das Auswertungsverfahren realisiert. In vorteilhafter Variante wird für die Berechnung davon ausgegangen, dass das Licht, welches die Grenzfläche von Probe und Vorrichtung mehr als 2-fach durchquert nicht berücksichtigt wird.

In einer vorteilhaften Variante existiert zumindest eine Paarung aus Lichtemitter und Lichtdetektor, bei der Licht nicht durch die Probe laufen muss, sondern auf dem Weg vom Lichtemitter zum Lichtdetektor zumindest teilweise an der Probenoberfläche reflektiert wird.

Unter Gruppe gleichartiger Paare oder Gruppe gleicher Paare oder Gruppe gleicher Lichtwege zwischen Emitter und Detektor wird im Zusammenhang dieses Dokumentes insbesondere folgendes verstanden. Für jede mögliche Kombination aus Emitter und Detektor kann eine Messung durchgeführt werden. Diese erfolgt, indem der Emitter aktiviert wird und Strahlung emittiert. In bevorzugter Ausgestaltung wird zu einem Zeitpunkt nur ein Emitter aktiviert. In bevorzugter Variante werden unterschiedliche Emitter nacheinander aktiviert. Die emittierte Strahlung durchläuft die Probe und gelangt zumindest zum Teil zu einem Detektor. In bevorzugter Ausführung sind mehrere Detektoren gleichzeitig aktiv. Das von einem Detektor erfasste Signal ist der Messwert des Emitter Detektor Paares aus dem aktivierten Emitter und dem zur Messung genutzten Detektor. Durch Variation der Emitter und Detektoren entsteht eine Vielzahl solcher Messwerte. Diese Messwerte werden unter bestimmten Bedingungen bevorzugt als eine Gruppe bezeichnet. Auch wenn in der Beschreibung teilweise explizit nur etwa gleiche Wellenlängencharakteristik und etwa gleicher Abstand genannt sind, sind bevorzugt auch andere Gruppenbildungsregeln möglich, die von der Ausgestaltung der Anordnung abhängen.

Für eine gleiche Wellenlängencharakteristik muss dazu z.B. nicht die Wellenlängencharakteristik des Emitters eines Emitter Detektor Paares mit der eines anderen übereinstimmen. Es genügt, bevorzugt wenn die aus Emitter und Detektor gemeinsam erzeugte Wellenlängencharakteristik hinreichend mit dem zu vergleichenden Emitter Detektor Paar übereinstimmt. Diese Übereinstimmung kann sich auch nur auf bestimmte Wellenlängenbereiche, die für die Analyse relevant sind beschränken.

Für eine gleiche Abstandscharakteristik muss nicht exakt der gleiche Abstand eingehalten werden. Wichtig ist, dass das von den am Detektor ankommenden Photonen durchlaufene Volumen der Probe zwischen den Paaren hinreichend ähnlich ist. Darauf hat auch die Richtcharakteristik einen Einfluss. Emitter und Detektorabstand bestimmen maßgeblich, wie tief das Licht, welches gemessen wird im Mittel in die Probe eindringt. Die Richtcharakteristik hat ebenso einen Einfluss darauf. Paare, die in eine Gruppe vergleichbarer oder gleicher Paare einsortiert werden, können also entweder hinreichend ähnlichen Emitter Detektor Abstand und hinreichend ähnliche Richtcharakteristik in Bezug auf die Emitter Detektor Paarung aufweisen, oder die Unterschiede in Richtcharakteristik und Abstand können sich kompensieren, so dass als Ergebnis eine hinreichende Vergleichbarkeit entsteht.

In einigen Fällen ist es vorteilhaft für die Zugehörigkeit zu einer Gruppe gleicher Paare zusätzlich vergleichbare Orientierung zu fordern.

Je nach Probe, Analyt und Messanordnung sind eine oder mehrere oder Kombinationen der obigen Bedingungen miteinander oder mit anderen Bedingungen vorteilhaft.

Unter **Probe** wird im Zusammenhang dieses Dokumentes allgemein ein zu untersuchendes Objekt verstanden. Dies soll insbesondere nicht ausschließen, dass es sich dabei um ein Lebewesen oder einen Menschen handelt. Insbesondere sind sowohl lebende, als auch nicht oder nicht mehr lebende zu untersuchende Objekte gemeint. Proben können unterschiedliche Zustände haben. Insbesondere sind feste, flüssige und gasförmige Proben möglich. Beispiele für eine Probe wären: der Handballen einer Person einschließlich der Hautschicht, eine Blut-, Urin- oder Gewebeprobe. Ein Stück Obst, ein Stück Kunststoff, ein Pressling aus Pulver (Tablette), eine Abgasprobe oder eine pürierte Menge an Gemüse sind ebenso Beispiele für eine Probe.

Unter Konzentration eines **Analyten** wird im Zusammenhang dieses Dokumentes eine Eigenschaft der zu untersuchenden Probe verstanden. Eine besonders typische Eigenschaft kann durch die Konzentration eines Stoffes in der Probe charakterisiert sein. Solche Stoffe sind beispielsweise Flavonoide, Cytochrom C Oxidase, Glucose, HbA1c, Fructose, Advanced glycation end products (AGE), Hämoglobin, Carboxyhämoglobin, Methhämoglobin, Synovitis, Laktat, Cholesterin. Im Rahmen des Dokumentes sind dabei auch Analyten gemeint, die nicht direkt, sondern indirekt über Zusammenhänge mit anderen Stoffen erkannt werden. Beispielsweise über Spaltprodukte, Abfallprodukte, Markersubstanzen, bei der Entstehung oder dem Abbau beteiligte Produkte. Insbesondere ist auch eine über mehrere Zusammenhänge z.B. aus mehreren anderen Substanzen ermittelbare Information gemeint. Weiterhin umfasst die Definition auch Eigenschaften, die über den rein chemischen Konzentrationsbegriff hinausgehen wie z.B. Mikrozirkulation der Gefäße, Hautfeuchtigkeit, Wassergehalt von Haut/Gewebe, Blutalkohol, Drogen, Zartheit von Fleisch, Frostbeständigkeit von Pflanzen, Reifegrad von Pflanzen, $NO_2$, Puls, Sauerstoffsättigung, Herzfrequenz. Insbesondere werden unter Konzentration eines Analyten also auch Konzentrationen von Stoffen in einem Teil der Probe verstanden. Beispielsweise ist die Probe die lebende Hand eines lebenden Menschen. Der interessierende Teil der Probe ist in diesem Beispiel das Blut des Menschen. Die interessierende Konzentration ist die Konzentration des Alkohols im Blut. Auch eine örtliche Begrenzung ist mit gemeint. Als Beispiel wäre die Konzentration z.B. Feuchtigkeit bestimmter Stoffe in bestimmten Hautschichten z.B. Epidermis oder 0-0,5mm Tiefe zu nennen. Insbesondere sind auch Konzentrationsverteilungsmerkmale gemeint, die z.B. Gradienten oder Stufungen sein können, die gleichmäßig oder ungleichmäßig sein können oder gerichtet oder ungerichtet oder isotrop oder anisotrop sein können. Sowie der zeitliche Verlauf von Konzentrationen, der beispielsweise gleichförmig, periodisch steigend oder fallend verlaufen kann. Ebenfalls unter Konzentration eines Analyten verstanden werden deswegen auch aus einer oder mehreren der genannten Eigenschaften ableitbare Größen. Ein Beispiel für einen periodischen Konzentrationsverlauf und einer daraus ableitbaren Größe ist die Bestimmung der Herzfrequenz, die sich aus der Periodizität der Konzentrationsverteilung bestimmter Blutstoffe in einem Körperteil ermitteln lässt, sowie dem aus dieser und anderen Größen erschließbaren Näherungswert für den allgemeinen Gesundheitszustand der analysierten Person.

Unter **Emittern** werden im Rahmen dieser Druckschrift insbesondere Elemente verstanden, die Licht oder elektromagnetische Strahlung emittieren. Dabei sind insbesondere ultraviolette Strahlung, infrarote Strahlung, Röntgenstrahlung und Mikrowellen sowie Funkwellen und die Frequenzbereiche dazwischen eingeschlossen.

Beispiele sind insbesondere Glühlampen, Glimmlampen, lichtemittierende Halbleiter, lichtemittierende Dioden (LEDs), lichtemittierende Leiter, Laser, Laserdioden, Leuchtstoffröhren, OLEDs, RGB-LEDs, Röhren, Antennen, Leuchtstoffe, Flammen, Lichtbogen, Funken und weitere dem Fachmann bekannte Strahlungsquellen.

Unter **Detektoren** werden insbesondere Elemente verstanden, die die von den Emittern emittierte Strahlung detektieren und in ein Signal umwandeln können.

Beispiele sind insbesondere Fototransistoren, Fotodioden, Fotomultiplyer, Spektrometer, Fotowiderstände, Solarzellen, Röhren, Kamerachips, CCD-Kameras, Halbleiter, LEDs, Antennen, Lichtfeldsensoren, Pyrometer und Strahlungssensoren.

Unter **transparentem Material** werden insbesondere Materialien verstanden, die von der von den Emittern erzeugten Strahlung zumindest zum Teil, bevorzugt zum Großteil, durchquert werden können.

Beispiele sind klarer Kunststoff oder Glas, milchiger oder trüber Kunststoff oder Glas, farbiger Kunststoff oder Glas, transparente Materialien, Gas, für Funkwellen passierbarer Kunststoff.

Unter **intransparentem Material** werden insbesondere Materialien verstanden, die die von den Emittern erzeugte Strahlung zumindest zum Teil am Durchqueren des Materials hindern. Dies kann beispielsweise durch Absorption, Reflektion, Brechung oder Streuung erfolgen.

Beispiele sind Kunststoff oder Glas mit schwarzen oder farbigen Pigmenten, Metall, Keramik, Holz, leitfähiger Kunststoff z.B. bei Funkwellen.

Elemente mit Volumenanteilen oder Oberflächen, die transparent oder intransparent sind können auch insgesamt als transparentes oder intransparentes Material angesehen werden. Beispiele sind ein Glaselement, welches mit einer nicht transparenten Metallschicht oder Lackschicht überzogen wurde oder ein Metallelement welches mit Löchern versehen wurde um Licht durchzulassen.

Die Definitionen von transparentem und intransparentem Material können sich überschneiden. In einem solchen Fall gilt das für die jeweils betrachtete Wellenlängencharakteristik eher für die Strahlung durchlässige Material als das transparente Material und das eher undurchlässige Material als das intransparente. Insbesondere kann die Einstufung eines Materials von der Wellenlänge der Strahlung abhängen. Übergänge zwischen transparentem und intransparentem Material müssen keine scharfen Übergänge sein. Zur Vereinfachung werden die Sachverhalte ohne Einschränkung der Allgemeinheit bevorzugt an scharfen Übergängen erläutert. Ein Material kann jedoch z.B. durch Einbringung von Pigmenten oder chemische oder physikalische Reaktionen an einigen Stellen in seiner Transparenz beeinflusst werden. Beispiele sind die Entwicklung von Fotofilmen, das Einbringen von Pigmenten in Teile einer sonst homogenen Kunststoffschicht oder die Einbringung von lichtstreuenden Blasen in einen homogen durchsichtigen Körper durch Laser.

## Beschreibung der Figuren

In den Figuren sind vorteilhafte Ausgestaltungen der Erfindung dargestellt.

Fig. 1            Einfache Emitter-Detektor Anordnung
Fig. 2            Matrixförmige Emitter-Detektor Anordnung
Fig. 3            Matrixförmige Emitter-Detektor Anordnung - Darstellung der 24 kurzen Lichtwege
Fig. 4            Matrixförmige Emitter-Detektor Anordnung - Darstellung der 16 kurzen bis mittellangen Lichtwege
Fig. 5            Matrixförmige Emitter-Detektor Anordnung - Darstellung der 32 anderen mittellangen Lichtwege
Fig. 6            Matrixförmige Emitter-Detektor Anordnung - Darstellung der 12 langen Lichtwege
Fig. 7            Beispielhafter Querschnitt eines Vorrichtungsgehäuses
Fig. 8            Gehäuse mit Positionierhilfen zur Messung am Daumenballen der Hand
Fig. 9            Gehäuse mit Positionierhilfen zum gezielten Bewegen der Vorrichtung auf der Oberfläche des Messobjekts
Fig. 10           Beispielhafte Ausgestaltung der Vorrichtung
Fig. 10a          zeigt eine beispielhafte Anordnung von Emittern und Detektoren
Fig. 10b, c, d, e, f    zeigen Beispiele gleicher Emitter Detektor Paare
Fig. 11           Beschreibung der Elektronik - optischer Emitter Beschreibung der Elektronik - Auswerteschaltung optischer Detektor
Fig.13            Beschreibung einer Vorrichtung 1
Fig. 14           Beschreibung einer Vorrichtung 2
Fig. 15           Beschreibung einer Vorrichtung 3
Fig. 16           Vorrichtung mit mehreren Arrays
Fig. 17           Besonders vorteilhafte Variante einer Messschaltung
Fig.17a           Messung eines Lichtwegs
Fig.18,a,b,c,d,e  Richtcharakteristik durch rechteckige Anordnung
Fig.19a,b,c,d     Erhöhung der spektralen Auflösung durch Kombination unterschiedlicher optischer Emitter mit unterschiedlichen optischen Detektoren

Fig.1 Einfache Emitter-Detektor Anordnung

Fig.1 zeigt eine einfache Emitter-Detektor Anordnung, bei der Emitter 1 und mehrere optische Detektoren 2 nebeneinander angeordnet sind und sich auf einer Linie befinden.

Mit dieser Anordnung kann das aus der Probe zurückgestreute Licht an drei verschiedenen Stellen detektiert werden. Die Entfernung zum Emitter nimmt dabei zu. Die Lichtwege 3.10 bis 3.30 unterscheiden sich deutlich. 3.10 bezeichnet einen kurzen Lichtweg, 3.20 einen mittellangen Lichtweg und 3.30 einen langen Lichtweg.

Um an einer biologischen Probe, wie beispielsweise der Haut, die Inhomogenität der Probe erfassen zu können, sind bei dieser Anordnung mehrfache Messungen an unterschiedlichen Hautstellen notwendig. Dies erfordert bei dieser einfachen Emitter-Detektor Anordnung nicht nur zusätzliche Messvorgänge, die zeitlich nacheinander durchzuführen sind, das Messergebnis kann auch von physiologische Änderungen zwischen den Messungen beeinflusst werden.

Wird zur Lösung des Problems die Emitter-Detektor Anordnung in einer Vorrichtung mehrfach nebeneinander angeordnet, erhöht sich der Aufwand in dem Maß, in dem zusätzliche Messpunkte hinzukommen. Ein weiterer Nachteil besteht darin, dass die Messpunkte in Richtung der längsten Ausdehnung nicht sehr nah beieinander platziert werden können.

Fig. 2 Matrixförmige Emitter-Detektor Anordnung

Die Nachteile der in Fig.1 dargestellten Anordnung werden erfindungsgemäß durch eine zweidimensionale Emitter-Detektor Anordnung gelöst. Fig.2 zeigt ein Beispiel für eine solche Anordnung.

In vorteilhafter Weise ergeben sich dabei eine größere Anzahl verschiedener Lichtweglängen. So zeigt 3.10 einen kurzen Lichtweg, 3.15 einen kurzen bis mittellangen Lichtweg, 3.21 einen weiteren mittelangen Lichtweg und 3.30 einen langen Lichtweg. In der matrixförmigen Anordnung gelingt es die Anzahl benötigter Messwege mit einer kleineren Anzahl an Emittern 1 und optischen Detektoren 2 zu erreichen, als eine Vervielfältigung der Anordnung aus Fig.1 es ergeben hätte.

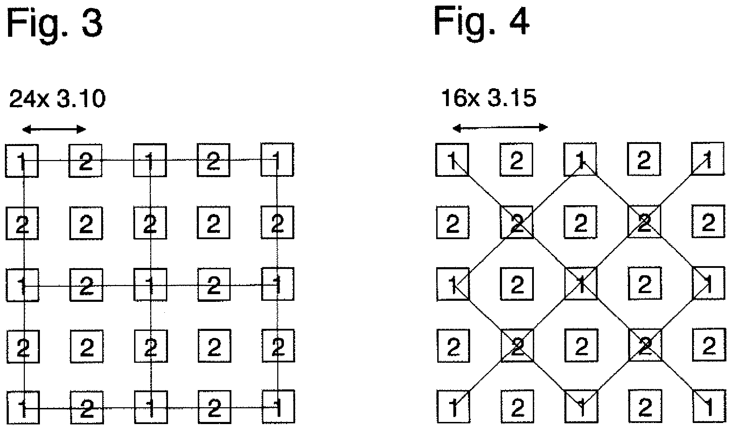

Fig.3 Matrixförmige Emitter-Detektor Anordnung - Darstellung der 24 kurzen Lichtwege

Fig.4 Matrixförmige Emitter-Detektor Anordnung - Darstellung der 16 kurzen bis mittellangen Lichtwege

Fig.5 Matrixförmige Emitter-Detektor Anordnung - Darstellung der 32 anderen mittellangen Lichtwege

Fig.6 Matrixförmige Emitter-Detektor Anordnung - Darstellung der 12 langen Lichtwege

Die Fig. 3 bis 6 zeigen, wie sich bei der beispielhaften Anordnung die Zahl der Lichtwege von Emitter 1 zu optischen Detektor 2 stark vervielfachen, während die Zahl der hierfür benötigten Emitter 1 und optischen Detektoren 2 deutlich weniger zunimmt. Die verschiedene Lichtweglängen (3.10, 3.15, 3.21, 3.30) sind in den Bildern 3 bis 6 zusammen mit der Häufigkeit ihres Vorkommens gezeigt. Insgesamt ergeben sich in der Summe mindestens die 84 in den Figuren gezeigten Lichtwege bei nur 9 Emittern und 14 optischen Detektoren.

Fig. 3 zeigt die Zahl der kurzen Lichtwege 3.10, die 24mal bei dieser Anordnung vorhanden ist. Fig. 4 zeigt die 16 kurzen bis mittellangen Lichtwege 3.15. Fig. 5 die 32 anderen mittellangen Lichtwege 3.21 und in Fig. 6 sind die 12 langen Lichtwege 3.30 zu sehen.

Fig.7 Beispielhafter Querschnitt eines Vorrichtungsgehäuses

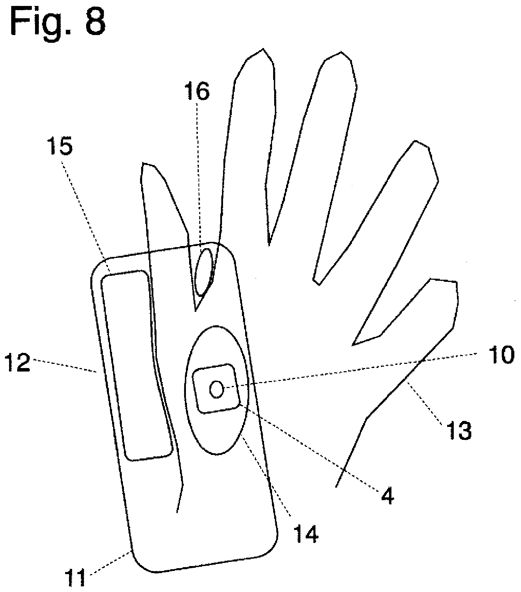

Fig.7 zeigt ein beispielhaftes Gehäuse für eine Vorrichtung zur Messung von Analyten am Daumenballen der Hand. Vorzugsweise nutzbar zur Messung von Karotinoiden. Das Gehäuse 12 ist stilisiert im Querschnitt dargestellt. Zur Durchführung der Messung wird es direkt auf den Daumenballen aufgelegt (siehe Fig.8). Im Hinblick auf die Form des Daumenballens besitzt das Gehäuse eine entsprechende Einwölbung 7.

Einwölbung 7 und Auswölbung 8 tragen in vorteilhafter Weise dazu bei, dass es nicht zum Verkratzen der Optoelektronik 4 kommt, wenn das Messgerät zwischen den Messungen z.B. auf einem Tisch abgelegt und dort hin und her geschoben wird. Einwölbung 7 und eine umlaufende oder punktuelle Auswölbung 8 tragen gleichzeitig dazu bei, dass während der Messung die Optoelektronik gegen Fremdlicht abgeschirmt ist.

Die Optoelektronik 4 befindet sich vorzugsweise in der Mitte der Einwölbung 7. Je nach Ausführung kann es einen lateralen Versatz zwischen Gehäuse 12 und der Optoelektronik 4 geben. Die Optoelektronik ist mit der Platine 6 verbunden, mit deren Hilfe die Signale aufgenommen und weiterverarbeitet werden können.

Zur Abdichtung gegenüber dem Eindringen von Feuchtigkeit und Staub wird zwischen dem Gehäuse 12 und der Optoelektronik 4 eine Vergussmasse 5 eingebracht. Bei einem Handmessgerät ist das Gehäuse vorzugsweise so gestaltet, dass es über den Handgriff 11 angefasst und während der Messung gehalten werden kann.

In einer vorteilhaften Variante der Vorrichtung wird der Start der Messung durch Betätigung einer Aktivierungstaste 10 ausgelöst. Vorteilhafter Weise ist die Aktivierungstaste 10 in einer Aktivierungsmulde 9 vertieft angeordnet, so dass ein ungewolltes Einschalten vermieden wird.

Wie in Fig.7 sichtbar ist die Aktivierungstaste vorzugsweise so angeordnet, dass daraus auf die Position der Optoelektronik geschlossen werden kann. Ein relativ präzises Auflegen des Messgeräts auf den Daumenballen der Hand ist somit leicht möglich. In vorteilhafter Variante wird zusätzlich über die Optoelektronik geprüft, ob Kontakt zum Prüfobjekt besteht. Eine Messung wird nur ausgelöst, wenn dieser Kontakt besteht.

In vorteilhafter Variante reagiert die Aktivierungstaste 10 erst bei einem gewissen Druck. Damit kann erreicht werden, dass die Messung erst gestartet wird, wenn die Vorrichtung ausreichend an das Prüfobjekt angedrückt wird.

Fig.8 Gehäuse mit Positionierhilfen zur Messung am Daumenballen der Hand

In Fig.8 ist beispielhaft eine Vorrichtung dargestellt, deren Gehäuse 12 mit Hilfe des Handgriffs 11 zur Messung auf den Daumenballen 14 aufgelegt wird. Mit Drücken der Aktivierungstaste 10 wird die Messung gestartet. Die Vorrichtung verfügt über Positionierhilfen, die das wiederholgenaue Auflegen der Optoelektronik 4 am Daumenballen 14 der Hand 13 unterstützt. Die seitliche Führung 15 stellt die Reproduzierbarkeit der Messposition in der einen Richtung, die Führung 16 die Reproduzierbarkeit in der anderen Richtung sicher. In einer vorteilhaften Variante der Erfindung sind die Führungen verstellbar, so dass sie auf den Benutzer oder das zu messende Objekt angepasst werden können.

In einer weiteren vorteilhaften Variante sind die Anschläge so ausgelegt, dass sie für unterschiedliche Messobjektgrößen auch ohne Verstellung gut geeignet sind.

In einer weiteren Variante sind die Führungen austauschbar oder können an das Gehäuse der Vorrichtung angebracht werden.

In einer vorteilhaften Variante können auch individuell auf einen Benutzer zugeschnittene Führungselemente verwendet werden. Z.B. indem ein Abdruck einer Hand in z.B. einer härtbaren Kunststoffmasse erstellt wird.

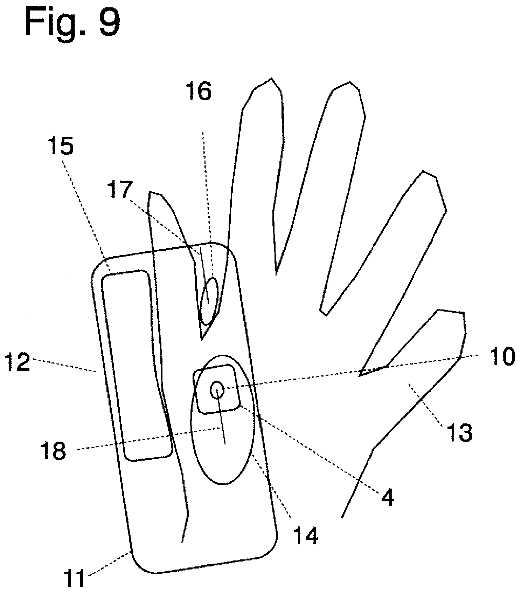

Fig.9 Gehäuse mit Positionierhilfen zum gezielten Bewegen der Vorrichtung auf der Oberfläche des Messobjekts

Die in Fig.9 beschriebenen Positionierhilfen 15 und 16 des beispielhaften Gehäuses 12 können auch dazu genutzt werden, dass die Optoelektronik 4 gezielt über die Oberfläche des Daumenballens 14 der Hand 13 bewegt werden kann, um auf diese Weise die Inhomogenität des Messobjekts möglichst genau zu erfassen. In einer vorteilhaften Variante der Erfindung ermöglichen sie das reproduzierbare Bewegen entlang einer Bahn 18. Dabei können nach Betätigung der Aktivierungstaste 10 entweder kontinuierlich Messwerte genommen werden, oder es können Messwerte an bestimmten Punkten der Messbahn 18 erfasst werden. Zu diesem Zweck kann sich z.B. das Tiefenführungselement 16 kontinuierlich oder in Schritten z.B. entlang eines Verschiebeweges 17 verschieben lassen. Messbahn und Verschiebeweg können eine Gerade oder eine Kurve im dreidimensionalen Raum darstellen.

In einer vorteilhaften Variante ist es auch möglich mehrere Messbahnen zu verwenden. Dies kann z.B. durch Verstellung weiterer Führungselemente geschehen.

Die während der Verschiebung der Optoelektronik 4 aufgenommen Daten können mit Hilfe einer Vorschrift so verrechnet werden, dass ein repräsentativer Wert der Inhomogenität des Messobjekts ermittelt wird.

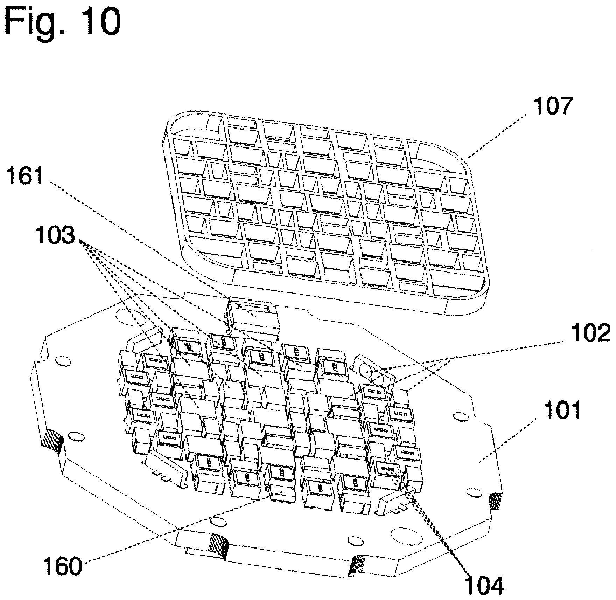

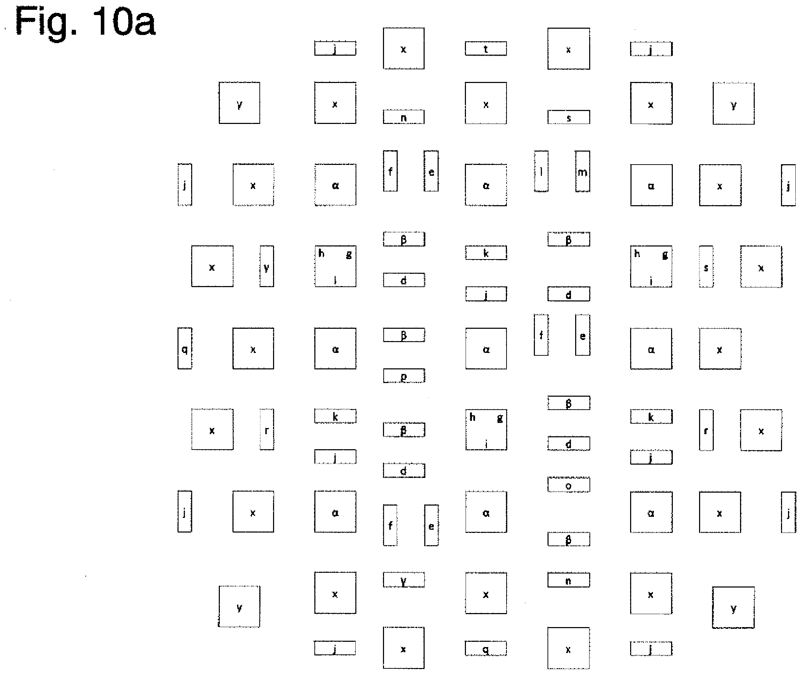

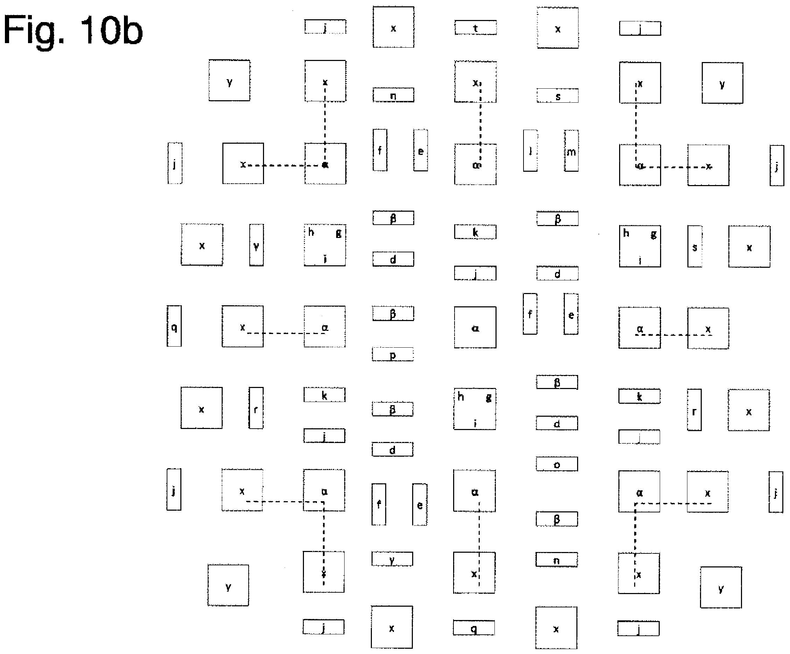

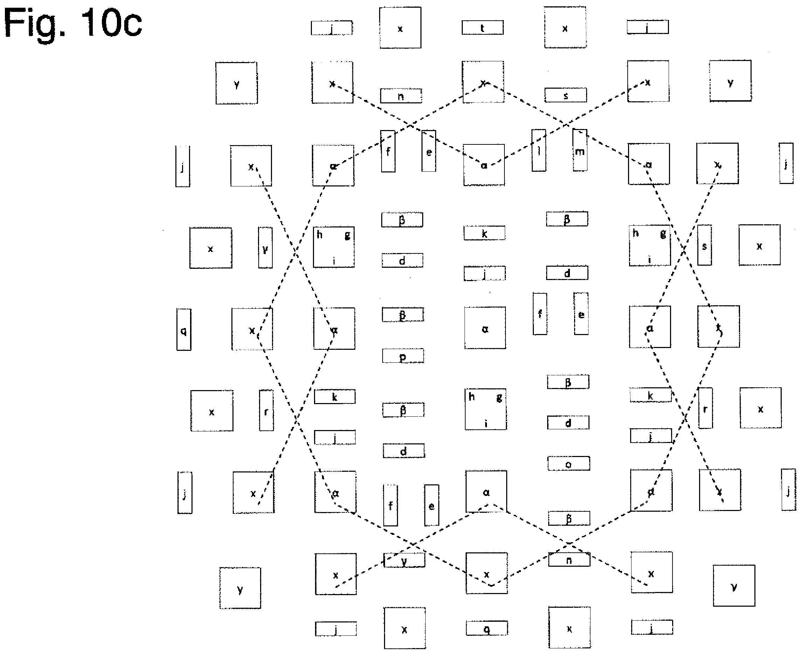

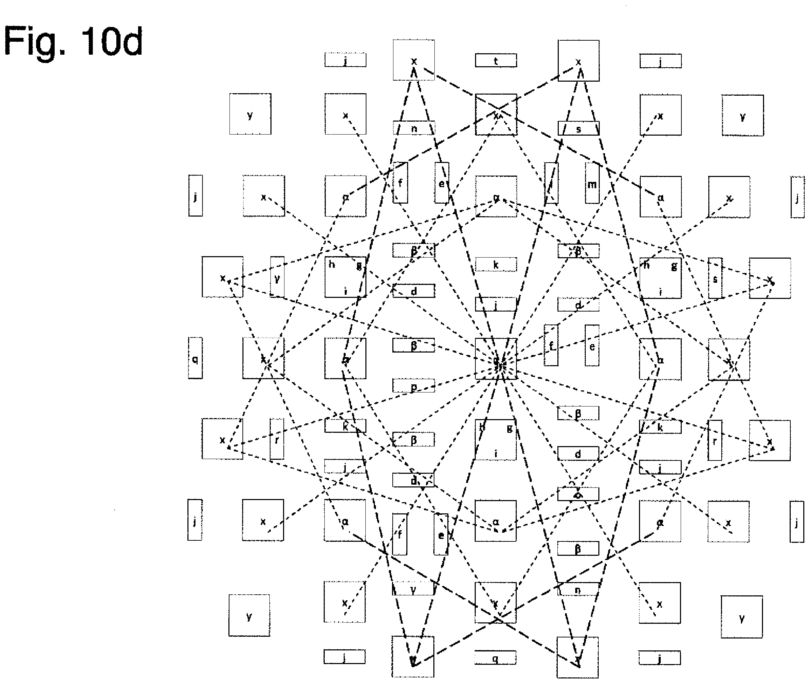

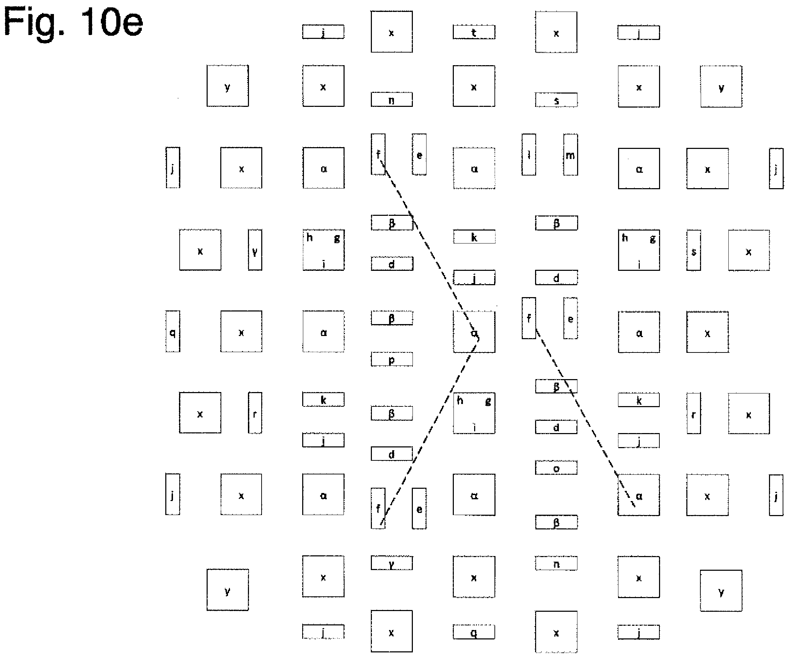

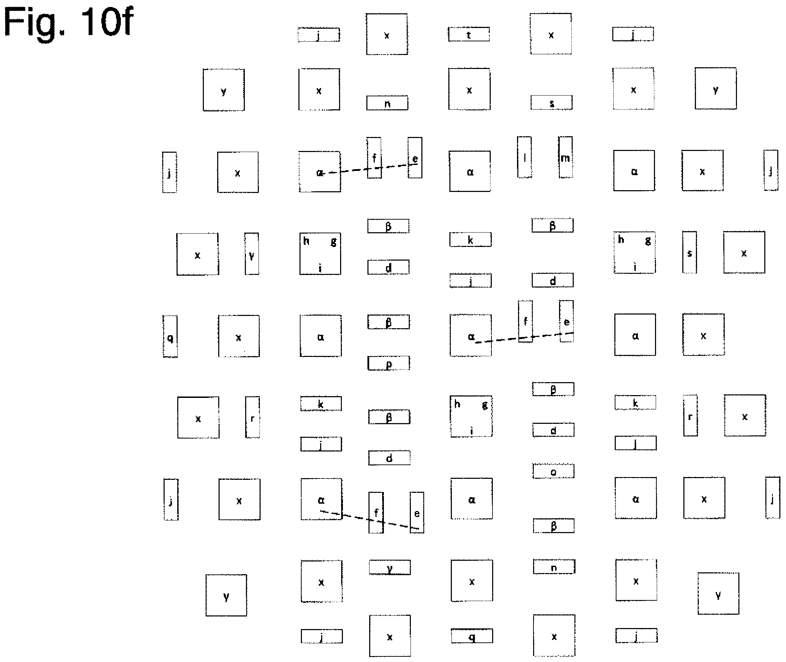

Fig. 10 Beispielhafte Ausgestaltung der Vorrichtung

Fig.10 zeigt beispielhaft eine erfindungsgemäße Ausgestaltung der Vorrichtung. Die optoelektronischen Bauteile befinden sich auf einem Trägermaterial oder einer Platine 101. Emitter 102 und optische Emitter Chips 104 sowie die optischen Detektoren 103 sind entsprechend der erfindungsgemäßen Ausgestaltung auf dem Trägermaterial 101 verteilt. Wegen der Einfachheit der Darstellung sind Detektor Chips nicht dargestellt. Ebenso sind nicht alle Emitter Chips gezeichnet. Weiterhin sind nur einige optische Emitter und Detektoren explizit mit Nummern als solche gekennzeichnet. Die nicht bezeichneten Bauteile sind ebenfalls Emitter oder Detektoren. Zur Messung zusätzlicher Parameter verfügt die Anordnung über einen Temperatursensor 160 und einen Feuchtigkeitssensor 161, so dass die Messbedingungen sehr gut erfasst und bei der Bestimmung der Konzentration des Analyten berücksichtigt werden können. Die Lichtsperre 107 besteht aus lichtundurchlässigen Material und ist wegen der besseren Übersichtlichkeit in Form einer Explosionszeichnung mit Abstand zur Vorrichtung gezeichnet. In der betriebsbereiten Version der Vorrichtung liegt die Lichtsperre 107 auf der Platine 101 auf. Die Größe und Form der einzelnen Kavitäten ist so gewählt, dass unter Berücksichtigung der Bauteilvolumens, sowie der Orientierung der Emitter und Detektoren eine gewünschte Abstrahl- und Detektionscharakteristik erreicht wird und das Licht in der Probe vorzugsweise an entsprechenden Lichtwegen propagiert. Wird die Lichtsperre, wie hier beispielhaft dargestellt, als separates Bauteil gefertigt, dann wird sie nachträglich auf die Anordnung der Optoelektronik aufgesteckt und die Kavitäten werden mit einem lichtdurchlässigen Material vergossen. Das transparente Material ist in der Zeichnung nicht dargestellt. Innerhalb jeweils einer Kavität befinden sich teilweise Emitter oder Detektoren, die jeweils eine Wellenlängencharakteristik aufweisen. Teilweise befinden sich innerhalb einer Kavität aber auch Emitter oder Detektoren mit mehr als einer Wellenlängencharakteristik, wie z.B. RGB-Emitter oder RGB-Detektoren oder mehrere einzelne Emitter und/oder Detektoren.

Fig. 10a zeigt eine beispielhafte Anordnung von Emittern und Detektoren, wie sie beispielsweise für die mechanische Anordnung aus Fig. 10 gewählt werden kann.

Emitter sind mit den Buchstaben a bis y gekennzeichnet. Emitter mit gleichen Buchstaben haben gleiche Wellenlängencharakteristik.

Die Emitter h, g und i sind nicht durch intransparentes Material getrennt.

Die Emitter x und y sind beispielsweise Emitter, die mehrere Wellenlängencharakteristika aufweisen können. Sie Können Licht unterschiedlicher Wellenlänge emittieren.

Die Emitter y strahlen Licht mit einer anderen Richtcharakteristik ab, als die Emitter x.

Die Detektoren sind mit den griechischen Buchstaben α, β und γ gekennzeichnet.

Der Detektor α weist beispielsweise fünf verschiedene Wellenlängencharakteristika auf.

Der Detektoren β und γ weisen unterschiedliche Wellenlängencharakteristik auf. Durch die rechteckige Geometrie ist die Richtcharakteristik nicht rotationssymmetrisch.

Fig. 10 b zeigt eine beispielhafte Gruppe gleicher Emitter Detektor Paare. In diesem Beispiel wurden etwa gleiche Wellenlänge, etwa gleichlange Lichtwege und vergleichbare Richtcharakteristik als Merkmal für die Gruppenzugehörigkeit gewählt. Es sind vier verschiedene Orientierungen der Verbindungslinie vom Emitter zum Detektor erkennbar, so dass in jedem 90 Grad Winkelbereich ein Lichtweg realisiert ist.

Fig. 10 c zeigt eine beispielhafte Gruppe gleicher Emitter Detektor Paare. In diesem Beispiel wurden etwa gleiche Wellenlänge, etwa gleichlange Lichtwege und vergleichbare Richtcharakteristik als Merkmal für die Gruppenzugehörigkeit gewählt. Es sind acht verschiedene Orientierungen der Verbindungslinie vom Emitter zum Detektor erkennbar. Es ist erkennbar, dass im Vergleich zu Fig. 10b eine feinere Stufung der Richtungen erfolgt.

Fig. 10 d zeigt eine beispielhafte Gruppe gleicher Emitter Detektor Paare. In diesem Beispiel wurden etwa gleiche Wellenlänge und etwa gleichlange Lichtwege als Merkmal für die Gruppenzugehörigkeit gewählt. Es sind vierzig Lichtwege vom Emitter zum Detektor erkennbar. Es ist erkennbar, dass die Anordnung so gewählt wurde, dass unter Berücksichtigung der Orientierung eine geringe Überlappung angestrebt wurde. Die 16 Emitter-Detektor Paarungen mit dem mittleren Detektor analysieren ein ringförmiges Areal um den Mittelpunkt der Vorrichtung, bei dem die Orientierung der Messung jeweils zu dieser Mitte gerichtet ist. Die verbleibenden 24 gezeigten Paarungen decken dieses ringförmige Gebiet ebenfalls ab, weisen aber eine Orientierung auf, die im Wesentlichen quer zu den 16 erstgenannten Paaren verläuft. Die Mehrfachnutzung der Emitter und Detektoren ist in diesem Beispiel ebenfalls gut erkennbar. Gerade durch die Verwendung längerer Abstände, wie sie durch eine Auswerteschaltung mit hoher Dynamik möglich werden, entsteht eine starke Zunahme an möglichem Emitter Detektor Paarungen. Im der Figur 10 d sind die Verbindungslinien der Emitter Detektor Paare mit unterschiedlich gestrichelten Linien gezeigt. Damit soll ein Beispiel dafür gegeben werden, dass Gruppen gleicher Emitter Detektor Paare auch in mehrere Untergruppen aufgeteilt werden können. Im Allgemeinen gilt, dass die Messwerte eines Emitter Detektor Paares natürlich auch in mehrere Gruppen einfließen können.

Fig. 10 e zeigt eine beispielhafte Gruppe gleicher Emitter Detektor Paare. In diesem Beispiel wurden etwa gleiche Wellenlänge, etwa gleichlange Lichtwege und vergleichbare Richtcharakteristik als Merkmal für die Gruppenzugehörigkeit gewählt. Es sind drei verschiedene Orientierungen der Verbindungslinie vom Emitter zum Detektor erkennbar. Es sind drei unterschiedliche Messorte erkennbar. Dies ist ein Beispiel dafür, dass die Anordnungen nicht symmetrisch sein müssen.

Fig. 10 f zeigt eine beispielhafte Gruppe gleicher Emitter Detektor Paare. In diesem Beispiel wurden etwa gleiche Wellenlänge, etwa gleichlange Lichtwege und vergleichbare Orientierung als Merkmal für die Gruppenzugehörigkeit gewählt.

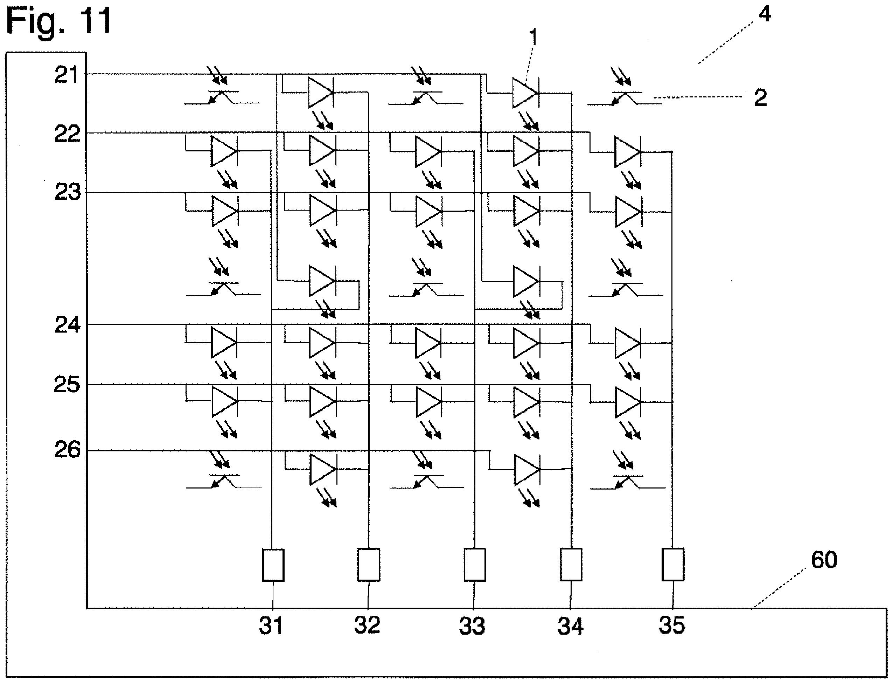

Fig. 11 Beschreibung der Elektronik - optischer Emitter

Zur Auswertung der mit der optoelektronischen Vorrichtung 4 durchgeführten Messung, deren Emitterseite in diesem Bild gezeigt ist, ist es in vorteilhafter Weise sinnvoll, die verschiedenen optischen Emitter 1 nacheinander anzuschalten, um die Messdaten der verschiedenen Lichtwege separat erfassen zu können. Insbesondere, wenn nur eine Lichtquelle gleichzeitig aktiviert ist bietet eine Matrixanordnung, wie sie in Fig.11 gezeigt ist, unerwartete Vorteile in der Ansteuerung. Von Matrixanordnungen ist bekannt, dass weniger Ansteuerleitungen von der Steuerungselektronik 60 benötigt werden.

Im Fall der beschriebenen Anordnung der Optoelektronik entsteht jedoch ein unerwarteter Vorteil bei der Verdrahtung, da die matrixförmige Anordnung weniger Leiterbahnplatz benötigt und sich die optischen Elemente 1 und 2 so dichter zusammenpacken lassen und bessere Messergebnisse liefern.

Das in Fig.11 gezeigte Beispiel ist so angeordnet, dass auf den LED-Zeilensignalen 21 bis 26 jeweils 0V ausgegeben werden mit Ausnahme von einem Zeilensignal, welches auf positivem Pegel liegt. Die Spaltensignale 31 bis 35 liegen alle auf positivem Pegel mit Ausnahme von einem Signal, welches auf 0V liegt.

Die Lichtquelle am Schnittpunkt des Zeilensignals auf positivem Pegel und des Spaltensignals auf 0V leuchtet.

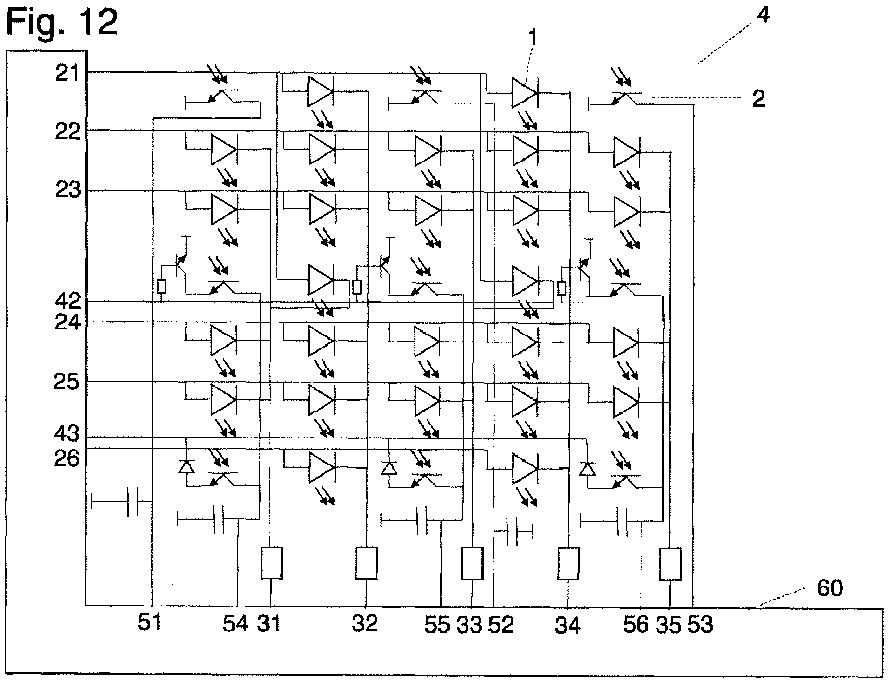

Fig.12 Beschreibung der Elektronik - Auswerteschaltung optischer Detektor

Fig.12 zeigt eine Auswerteschaltung der optoelektronischen Vorrichtung 4 mit verschiedenen Schaltungsvarianten.

In vorteilhafter Variante ist die Auswertung der lichtempfindlichen Detektoren 2 oder auch der anderen im System eingesetzten optischen Emitter 1 matrixförmig organisiert. Dabei können mehrere optische Detektoren an einer Selektionsleitung 42 oder 43 angeschlossen sein.

In vorteilhafter Variante sind die an die Selektionsleitung 42 angeschlossenen Transistoren räumlich in die Matrix integriert. In besonders vorteilhafter Variante sind sie auf der Platinenrückseite (also auf der Seite, auf der keine optischen Bauelemente liegen) angeordnet.

In alternativer Variante sind statt Transistoren Dioden verwendet, wie es die Leitung 43 zeigt.

Das analoge Signal mehrerer optischer Detektoren kann in vorteilhafter Variante auf einer gemeinsamen Leitung 54, 55, 56 gesammelt und gemessen werden. In vorteilhafter Variante kann die Messung erfolgen, indem die zeitliche Dauer der Ladung oder Entladung einer Kapazität gemessen wird.

In besonders vorteilhafter Variante wird die Lade-/Entladezeit auf ein Vielfaches der halben Netzfrequenzperiode ausgelegt. Die meisten Leuchtmittel erzeugen Licht in einer Frequenz, die der doppelten Netzfrequenz entspricht. Beträgt die Messung bei 50Hz Netzfrequenz ein Vielfaches von 10ms, so werden Störeinflüsse durch flackerndes netzbetriebenes Licht minimiert.

In vorteilhafter Variante erfolgt die Entladung über eine Zeitspanne, die zumindest näherungsweise mit der Periodizität möglicherweise vorhandenen Kunstlichts abgeglichen ist. So kann z.B. an Orten mit 50Hz Wechselstrom die Zeitspanne ein Vielfaches von 10ms sein, da typischerweise beide Halbwellen der Netzspannung einen Lichtimpuls erzeugen. An Orten mit 60Hz gilt entsprechendes. Durch Wahl der Zeitspanne in der Nähe eines geeigneten Vielfachen beider Frequenzen lässt sich ein weltweit gut einsetzbares System schaffen.

In besonders vorteilhafter Variante kann die beschriebene Schaltung direkt an einen Mikrocontroller angeschlossen werden. Dieser kann in besonders vorteilhafter Variante die komplette Funktion der Steuerungselektronik 60 übernehmen.

Dazu werden die LED Zeilensignale 21 bis 26 und LED Spaltensignale 31 bis 35 an digitale IO-Ports des Controllers angeschlossen. Ebenso die Detektor Zeilensignale.

Der Controller erzeugt nun diese Signale so, dass genau eine LED aktiviert ist und eine Gruppe optischer Detektoren aktiviert sind.

Anschließend können die Leitungen 51 bis 56 über einen im Controller zuschaltbaren Widerstand auf z.B. positives Potential gelegt werden. Durch im Controller integrierte AD-Wandler kann jetzt bereits ein grober Wert des Stromes durch den optischen Detektor bestimmt werden.

Über ein Setzen der Leitungen 51 bis 56 auf positives Potential ohne Widerstand kann der Kondensator auf definiertes Potential gebracht werden.

Alternativ oder zusätzlich kann das Potential am Kondensator über AD-Wandler oder DA-Wandler und Komperatoren gemessen werden.

Werden die Leitungen 51 bis 56 anschließend auf hochohmiges Potential geschaltet, so kann mittels AD-Wandler oder mittels Comperator oder durch das Überschreiten einer Schaltschwelle eines digitalen Eingangs die Veränderung der Spannung am Kondensator festgestellt werden. Über die Messung der dafür nötigen Zeit kann auf den Strom durch den Detektor geschlossen werden.

Durch Festlegen der Schwelle für den Comperator kann die Zeit auf die oben beschriebenen günstigen Ladezeiten angepasst werden. Ist die Ladezeit geringer, so kann eine oder mehrere weitere Messung oder Messungen durchgeführt werden, wobei die Ergebnisse der Messungen anschließend gemittelt werden können, um ein Ergebnis zu erhalten, welches von flimmerndem Kunstlicht wenig beeinflusst wird.

In vorteilhafter alternativer Variante wird jeder Detektor einzeln ausgewertet, was den Vorteil einer schnelleren Messung aufweist. Dies ist bei den Detektoreingängen 51, 52 und 53 gezeigt.

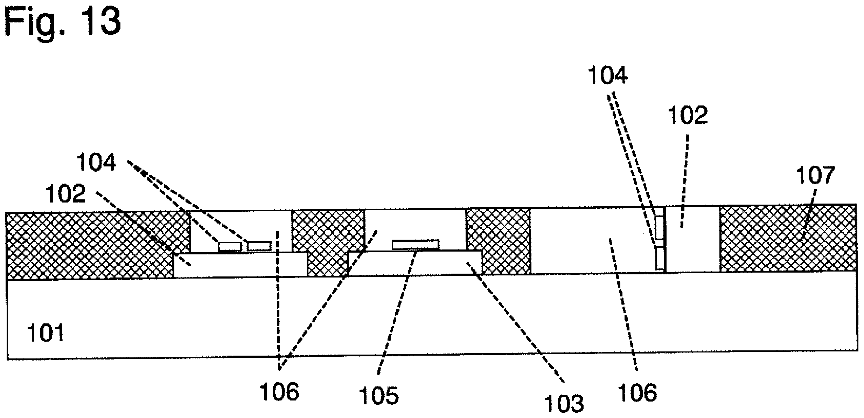

Fig.13 Beschreibung einer Vorrichtung 1

Die Fig.13 zeigt einen Ausschnitt eines Schnitts durch die Vorrichtung.

Auf das Trägermaterial 101, z.B. FR4 oder Keramik, sind optoelektronische Bauelemente aufgebracht, z.B. in Form von aufgelöteten SMD Bauelementen. Zu diesen Bauteilen gehören optische Emitter 102 (z.B. LEDs) und optische Detektoren 103.

Die optischen Emitter 102 weisen einen oder mehrere Chips 104 auf, die Licht abstrahlen. Dieses Licht wird von dem optischen Detektorchip 105 empfangen.

Damit das Licht von den Emittern zu den Detektoren gelangen kann wird transparentes Material 106 verwendet. Dabei kann es sich z.B. um Kunststoff handeln.

Damit das Licht nicht direkt vom Emitter zum Detektor gelangen kann, sondern durch die zu messende Probe laufen muss, kann nicht transparentes Material 107 eingesetzt werden.

Es kann jedoch auch vorteilhaft sein, wenn Detektor und Emitter nicht durch ein nicht transparentes Material getrennt werden. So kann z.B. an der Oberfläche der Probe reflektiertes Licht analysiert werden.

In vielen Fällen ist es wichtig zu beeinflussen, auf welchen Wegen das Licht durch die zu analysierende Probe läuft. Zu diesem Zweck kann z.B. die Orientierung der Emitterchips 104 oder der Detektorchips 105 angepasst werden. Im Bild ist beispielsweise eine senkrechte Orientierung beim rechten Emitterchip 104 gezeigt.

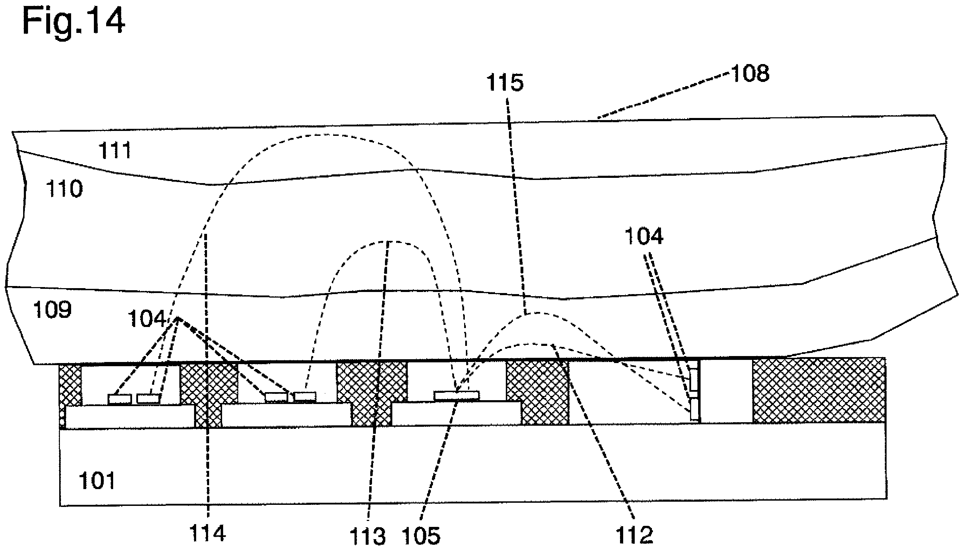

Fig.14 Beschreibung einer Vorrichtung 2

Fig.14 zeigt eine beispielhafte Ausführung der Vorrichtung mit aufgelegter zu messender Probe 108.

Die zu messende Probe ist in diesem Beispiel ein Stück menschliche Haut. Diese weist 3 Schichten auf. Die äußere Schicht 109, eine weiter Richtung Körperinneres liegende mittlere Schicht 110 und eine noch weiter innen liegende innere Schicht 111.

Diese Schichten weisen verschiedene Eigenschaften auf. Durch die optische Messung sollen Konzentrationen von Analyten in den verschiedenen Schichten gemessen oder abgeschätzt werden.

Durch die unterschiedlichen Abstände der Emitter Chips 104 zum optischen Detektor Chip 105 ergeben sich unterschiedliche Lichtwege, wie man an den Lichtwegen 113 und 114 sieht.

Die im Bild gezeigten Lichtwege repräsentieren jeweils nur einen typischen Lichtweg. In der Realität setzt sich der am Detektor ankommende Lichtstrom aus einer Vielzahl unterschiedlicher Wege zusammen, die verschiedene Länge aufweisen und verschieden wahrscheinlich sind. Die Wege ergeben sich durch Brechung, Streuung, Absorption und Reflektion des Lichts im Gewebe.

Für die relevanten Lichtwege wird die Lichtmenge erfasst, die vom Emitter zum Detektor gelangt. Durch Vergleich der Messergebnisse verschiedener Lichtwege kann auf die Eigenschaften der Probe in verschiedener Tiefe geschlossen werden. Auf diesem Weg kann auch auf Eigenschaften der verschiedenen Schichten z.B. verschiedener Hautschichten geschlossen werden.

Die Messungen können an gleichen oder unterschiedlichen Orten wiederholt werden, um die Messgenauigkeit zu erhöhen.

Zur Berechnung der Konzentrationen von Analyten an einem bestimmten Ort der Probe werden die Messergebnisse z.B. linear oder nichtlinear gewichtet und anschließend summiert oder anders verknüpft. Das Ergebnis wird anschließend nochmal linear oder nichtlinear gewichtet. Die Gewichtungsfaktoren oder Gewichtungsfunktionen werden geeignet gewählt, um den gewünschten Konzentrationswert am gewünschten Probenort zu ermitteln.

Es kommt vor, dass die Abstände zwischen Detektoren und Emittern nicht kurz genug gemacht werden können, um Lichtwege zu erzeugen, von denen nur ein kleiner Teil die äußerste Schicht verlässt.

Auch in anderen Messsituationen ist es erstrebenswert die Lichtwege so zu beeinflussen, dass sich ein für das Ergebnis günstiger Lichtweg ergibt. Das Beispiel einer Messung für die äußere Schicht 109 soll den allgemeinen Fall hier nicht einschränken.

Die Erfindung zeigt mehrere Wege auf, dieses Problem zu lösen.

In Fig.14 wird vom rechtesten Emitter 104 Licht abgestrahlt, welches besonders flach durch die Probe läuft. Dies ist als Lichtweg 112 markiert. Der flache Lichtweg wird durch die Orientierung des Emitterchips erreicht.

Eine wichtige Rolle spielt auch die Position des Emitterchips. Lichtweg 115 zeigt eine alternative Position, die einen anderen Lichtweg ergibt.

Natürlich kann zusätzlich oder alternativ auch die Orientierung des Detektorchips geändert werden.

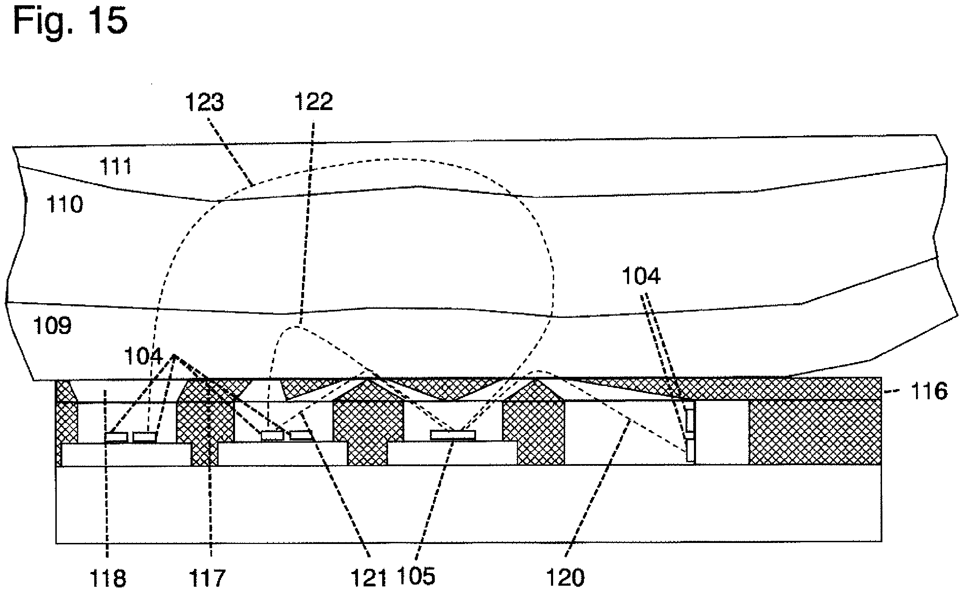

Fig.15 Beschreibung einer Vorrichtung 3

Fig.15 zeigt eine andere Vorrichtung. Bei dieser Vorrichtung werden Elemente 116 eingesetzt, die die Empfangs- und/oder Abstrahlcharakteristik und/oder Richtcharakteristik von Emitterchip 104 und Detektorchip 105 gezielt beeinflussen.

Diese Elemente können z.B. aus lichtdurchlässigen 118 und lichtundurchlässigen Materialien 117 bestehen. Sie können die Richtcharakteristik beeinflussen, indem die lichtundurchlässigen Materialien als Blende wirken.

Weiterhin kann die Richtcharakteristik beeinflusst werden, indem das Licht gebrochen oder gespiegelt oder gebeugt wird.

Die Oberfläche des Elementes zur Optimierung der Richtcharakteristik muss nicht zwangsläufig eben sein. Die Richtcharakteristik kann auch durch Linsen, Gitter, oder gewölbte Oberflächen oder Spiegel beeinflusst werden.

Lichtweg 121 und 122 zeigen, dass Licht dabei auch auf mehreren Wegen vom Emitter 104 zum Detektor 105 gelangen kann.

Lichtweg 123 zeigt, dass durch gezielte Orientierung des Lichtstrahls weg vom optischen Partner besonders tiefe Messwege erreicht werden können und dabei die Schichten 109, 110, 111 der Probe durchlaufen werden. Auch eine Orientierung des Lichtstrahls senkrecht in die Probe hinein führt zu tiefen Lichtwegen.

Lichtweg 120 zeigt, dass durch eine geeignete Orientierung der Richtcharakteristik in Richtung auf den optischen Partner besonders flache Lichtwege erreicht werden können und das Licht dabei nur einen geringen Teil der Schicht 109 durchläuft.

Fig.16 Vorrichtung mit mehreren Arrays

Fig.16 zeigt eine vorteilhafte Variante der Vorrichtung.

In dieser Variante werden mehrere Emitter-Detektor Arrays eingesetzt. Im gezeigten Beispiel das Array 140 und das Array 141.

Die gezeigte Anordnung ist beispielhaft. Im allgemeinen Fall könnten auch mehr als 2 Emitter-Detektor Arrays verwendet werden. Die Arrays müssen auch nicht zwangsläufig flach sein, sondern können auch gewölbt sein. Es kann sich auch um ein einzelnes gewölbtes Array handeln.

Die Anordnung kann auch variabel, beweglich oder an die Messung anpassbar gestaltet werden. Im Beispiel ist ein einfaches Gelenk 143 gezeigt, durch welches die beiden Gehäuseteile 144 und 145 schwenkbar verbunden sind. Die Bewegung kann jedoch auch anders realisiert sein. Insbesondere kann die zu messende Probe 142 durch die Bewegung eingeklemmt oder gegriffen werden. Die relative Position der Gehäuseteile 144 und 145 zueinander kann durch Sensoren erfasst werden, was die Auswertung erleichtert.

Lichtwege, die nicht durch die Probe hindurchverlaufen, wie 150 können genutzt werden, um festzustellen, wie die Probe 142 gegriffen ist.

Lichtweg 146 erfasst sowohl Gewebeteile der äußeren Schicht 109 als auch signifikante Teile der mittleren Schicht 110.

Lichtweg 148 dagegen erfasst kaum Anteile aus der mittleren Schicht 110. Durch Vergleich der beiden Lichtwege können die Eigenschaften der mittleren Schicht gut bestimmt werden.

Der Lichtweg 147 zeigt einen diagonalen Verlauf durch das Messobjekt. Dadurch kann ein längerer Lichtweg durch die äußeren Schichten erreicht werden.

Die in Fig.16 gezeigten geraden Linien sollen nicht darüber hinwegtäuschen, dass ein großer Teil des Lichts nicht den direkten Weg nimmt, sondern der Richtcharakteristik von Emitter und

Detektor folgt und einer gebogenen Kurve folgt. Die Richtcharakteristik kann auch in dieser Anordnung geeignet gewählt werden.

Lichtweg 149 zeigt, dass auch Lichtwege genutzt werden können, die nicht komplett in der zu messenden Probe verlaufen.

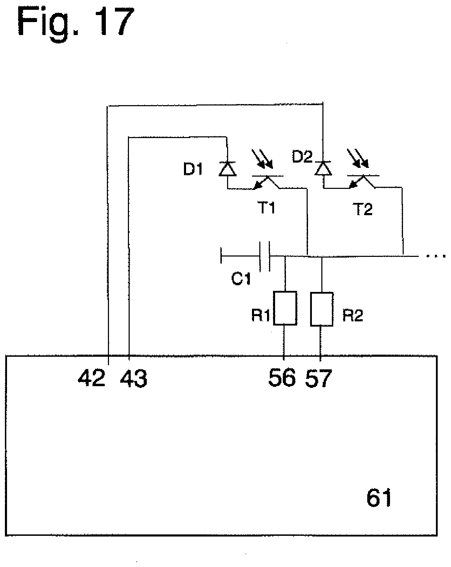

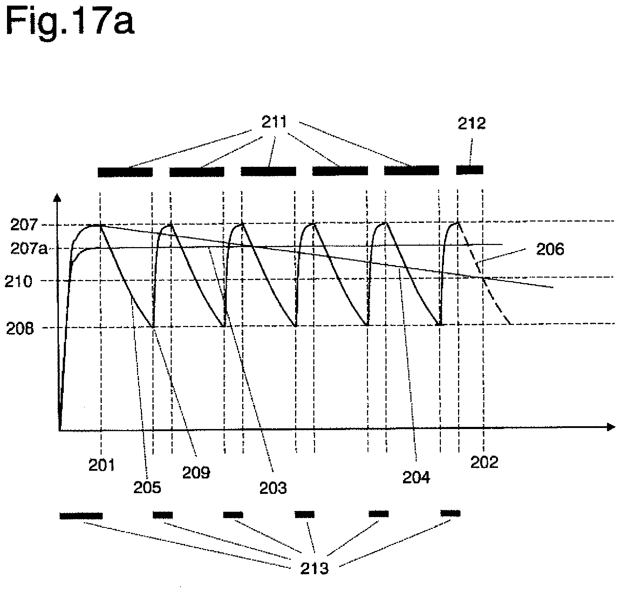

Fig.17 Besonders vorteilhafte Variante einer Messschaltung

Fig.17 zeigt eine besonders vorteilhafte Variante der Messschaltung.

Diese lässt sich für einen oder mehrere Fototransistoren verwenden. Zur Vereinfachung sind hier mit T1 und T2 nur zwei gezeigt. Die Selektion erfolgt wieder über die Selektionssignale 42 und 43. Über diese wird dafür gesorgt, dass kein Strom über den optischen Detektor fließt, wenn dieser nicht ausgewählt ist. Die Signale 42 u. 43 können direkt vom Mikrocontroller 61 hochohmig geschaltet werden, oder sie können über zusätzliche Bauteile den Stromfluss verhindern. Z.B. über die Dioden D1 und D2, die bei geeignetem Pegel an 42, 43 den Stromfluss sperren. Alternativ können auch andere Bauteile statt der Dioden eingesetzt werden, wie z.B. Transistoren. Weiterhin kann bei Verwendung nur eines Detektors pro Messeingang 56 auch das Detektorbauteil direkt an Betriebsspannung angeschlossen werden, so dass D1 und D2 entfallen.

In vorteilhafter Variante existiert ein C1, welches die natürliche Kapazität von Detektor und Schaltung erhöht. Diese Kapazität wird beim Messen geladen und entladen.

Prinzipiell kann die Schaltung auch mit umgekehrtem Stromverlauf realisiert werden. Laden und Entladen sind dann vertauscht. Der Einfachheit halber wird hier nur eine Variante beschrieben.

Der Messzyklus beginnt, indem C1 über Ausgang 56 oder 57 über R1 oder R2 aufgeladen wird.

In einer vorteilhaften Variante wird über 57 geladen und über 56 der Spannungspegel auf C1 gemessen. Besonders hohe Helligkeiten können in diesem Schritt gut erfasst werden. Er wird im Folgenden als Mode D bezeichnet. R2 bildet dabei mit z.B. T1 einen Spannungsteiler, so dass die Höhe des Stromflusses durch T1 durch Messung an 56 erfasst werden kann.

In vorteilhafter Variante kann 57 entfallen, indem 56 gleichzeitig oder sehr schnell hintereinander zum Aufladen und Messen genutzt wird.

In vorteilhafter Variante können R1 oder R2 entfallen.

In vorteilhafter Variante können R1 und R2 entfallen, wenn Mode D nicht genutzt wird. Alternativ bieten viele Mikrocontroller einen im Controller integrierten R1 und/oder R2.

In vorteilhafter Variante wird der Stromfluss während der Messzeit mehrfach zyklisch gemessen, um den Einfluss flackernder Lichtquellen (z.B. mit Netzfrequenz) herauszurechnen, was z.B. durch Mittelung erfolgen kann.

Für hohe Helligkeiten wird in vorteilhafter Variante Mode Z angewendet. Bei diesem wird die Kapazität zunächst geladen. Zu Beginn der Messzeit werden 56 und 57 auf hochohmig geschaltet, so dass sich C1 über z.B. T1 entlädt. Über 56 wird festgestellt, ob eine bestimmte Entladeschwelle erreicht ist. Wenn ja, dann wird C1 über 56 oder 57 wieder aufgeladen. Dieser Vorgang wird während der Messzeit zyklisch wiederholt. Die Zahl der Zyklen und die Gesamtzeit der Entladung werden erfasst. Über die Anzahl Zyklen und die dazu nötige Entladezeit kann der Stromfluss durch den Detektor und somit die Helligkeit ermittelt werden.

Bei sehr geringen Helligkeiten wird Mode A angewendet. Dabei wird die Spannung an 56 zum Beginn des Messzeitraumes und am Ende des Messzeitraumes gemessen. Um die Genauigkeit zu steigern kann dies auch mehrfach erfolgen. Aus der Spannungsdifferenz kann auf den Stromfluss durch das Detektorelement geschlossen werden.

Zwischen Mode A und Mode Z ist Mode M angesiedelt. In diesem Modus werden mehrere Entladezyklen durchgeführt, wie in Mode Z. Der letzte Entladezyklus wird jedoch in seiner Anfangs- und Endspannung gemessen wie in Mode A. Durch geeignete Verrechnung lässt sich der Stromfluss so noch genauer ermitteln.

In vorteilhafter Variante wird die Spannung zu Beginn der Entladung nicht gemessen. Sie kann auch aus den Randbedingungen der Aufladung abgeschätzt werden.

In vorteilhafter Variante wird die Endspannung über einen Comparator überwacht, wie er oft in Mikrocontroller integriert ist.

Die Messung von Entladezeiten zum Messen von Stromfluss ist eine bekannte Methode. Die hier erläuterte Kombination der Verfahren hat jedoch den Vorteil, dass alle Verfahren so modifiziert sind, dass sie die gleiche Messzeit abdecken und darüber mitteln. Dies ist zur Unterdrückung von Fehlern durch flackendes Kunstlicht für die hier beschriebene Vorrichtung sehr wichtig und vorteilhaft.

Weiterhin entstehen besondere Vorteile durch den hohen Dynamikbereich, der sich aus der Kombination der Messverfahren Mode D, Mode Z, Mode M und Mode A ergibt. Der hohe Dynamikbereich erlaubt es sowohl sehr kurze Lichtwege, als auch lange Lichtwege mit der gleichen Messschaltung zu erfassen. Weiterhin ist die Schaltung ohne großen Aufwand auch auf mehrere Detektoren anwendbar, so dass eine matrixförmige Detektoranordnung realisierbar ist.

Vorteilhaft ist weiterhin, dass zu Beginn der Messung keine Information über die zu erwartende Helligkeit vorliegen muss. Verfahren, die mit Umschaltung von Verstärkern arbeiten haben vergleichsweise den Nachteil, dass zunächst im unempfindlichsten Messbereich gemessen werden muss. Anschließend wird bei geringem Signal in einen empfindlicheren Bereich geschaltet und nochmal gemessen. In der beschriebenen matrixförmigen Detektoranordnung wäre dies ein großer Nachteil, da die Messzeit zum Ausmitteln von flackerndem Licht eine feste Länge aufweist und somit 2 oder mehr Messzeiten benötigt werden. Die Messung wird damit signifikant langsamer und es können in der zur Verfügung stehenden Gesamtmesszeit nur eine geringere Anzahl an Lichtwegen gemessen werden. Diesen Nachteil weist die erfindungsgemäße Ausführung nicht auf. Zu Beginn der Messung muss der zu messende Helligkeitswert nicht bekannt sein. Die Messung nach Mode D, Mode A, Mode Z und Mode M beginnen alle gleich. Aus den während der Messung anfallenden Daten kann dynamisch der korrekte Mode ausgewählt werden, ohne dass die Messung neu begonnen werden muss.

Fig.17a **Messung eines Lichtwegs**

Fig.17a zeigt den Ablauf der Messung eines Lichtweges. In vorteilhafter Anwendung wird jeweils eine Lichtquelle aktiviert und die in Bild17a gezeigte Messung für alle Detektoren, die sich keinen Eingang 56 (Fig.17) teilen gleichzeitig durchgeführt. Anschließend wird die Lichtquelle deaktiviert und die nächste Lichtquelle aktiviert und erneut gemessen. Sind alle Lichtquellen gemessen, so werden die Detektoren über die Ausgänge 42,43 (Bild 17) umgeschaltet und es werden erneut alle Lichtquellen gemessen. Die Reihenfolge kann dabei aber auch anders sein.

Jede einzelne Messung läuft ab wie in Fig.17a gezeigt. In einem ersten Aufladeschritt 213 wird die Kapazität C1 geladen. Über AD-Wandler wird die Spannung an 56 bestimmt (Bild 17). Ist diese auf geringem Niveau 207a, so wird Mode D durchgeführt. Die Spannung wird zyklisch im Zeitraum vom Messungsbeginn 201 bis zum Messungsende 202 gemessen und das Ergebnis gemittelt. Kurve 203 zeigt den Spannungsverlauf bei gleichbleibender Helligkeit. Mode D eignet sich für extrem helle Situationen.

Ist die Spannung zum Zeitpunkt 201 hoch genug, so wird nicht Mode D, sondern Mode A, Z oder M verwendet, die dem gleichen Ablauf unterliegen. Die Ladespannung 207 wird gespeichert. Am Ende der Messung wird die Spannung 210 an 56 (Fig. 17) nochmals gemessen und gespeichert.

Die Differenz dieser Spannungen ist die Entladespannung für Mode A, die im Zeitraum zwischen 201 und 202 erfolgt ist. Wurde während der Messung an 56 (Fig. 17) niemals der Schwellwert 208 unterschritten, so liegt Mode A vor. Die zugehörige Kurve ist als 204 eingezeichnet. Aus dieser Spannung kann direkt auf geringe Helligkeiten am Detektor geschlossen werden. Ggf. wird eine Linearisierung angewendet. Mode A eignet sich für sehr dunkle Situationen.

Kurve 205 zeigt, wie der Spannungsverlauf in Mode Z und Mode M aussieht. Die Spannung an C1 sinkt ab, bis an 56 (Fig. 17) die Unterschreitung der Schwelle 208 festgestellt wird. Die dazu benötigte Zeitspanne 211 wird gespeichert und es wird eine Aufladung z.B. über 56 oder 57 (Fig. 17) veranlasst. Nach Abschluss der Aufladung wird eine erneute Entladung durchgeführt. Dabei wird die Anzahl der Zyklen gezählt, die Entladedauern 211 werden gemessen und die Entladespannungshübe von 207 zu 208 werden für die Auswertung erfasst.

Bei hoher Zyklenzahl wird nur aus Zyklenzahl oder in vorteilhafter Variante aus Zyklenzahl und der Summe der Entladezeiten 211 auf die Helligkeit am Detektor geschlossen. Dies ist Mode Z. Er ist für helle Situationen geeignet.

Bei geringer Zyklenzahl z.B. im Bereich zwischen 1 und 200 Zyklen, wird Mode M verwendet. Bei diesem wird die Höhe des letzten Zyklus durch Messung der Spannung 210 am Messungsende 202 und Differenzbildung zu 207 ermittelt. Die Höhe des letzten Zyklus wird zur Summe der Höhe der vorhergehenden Zyklen addiert. Dieser Wert wird zur Summe der Entladezeiten 211 plus der Entladezeit 212 in Beziehung gesetzt. Ggf. erfolgt vor der Summenbildung jeweils eine Linearisierung. Auf diesem Weg lässt sich die Helligkeit für mittelhelle Situationen sehr genau bestimmen.

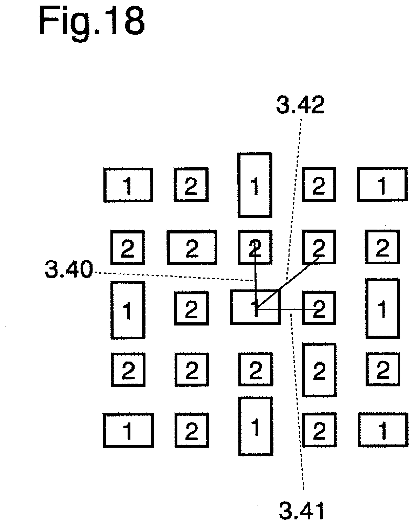

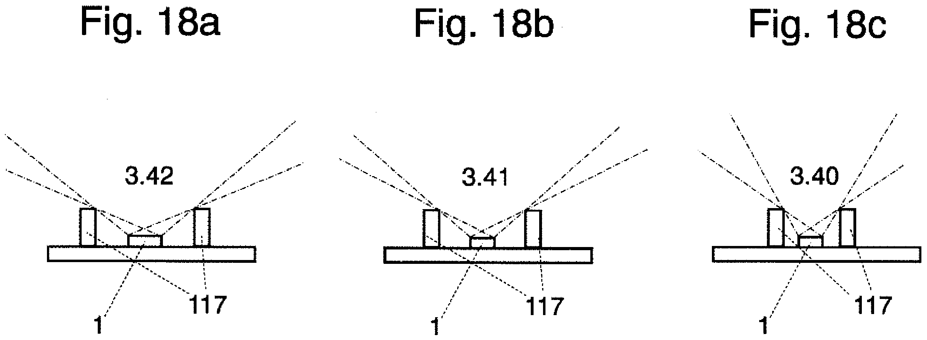

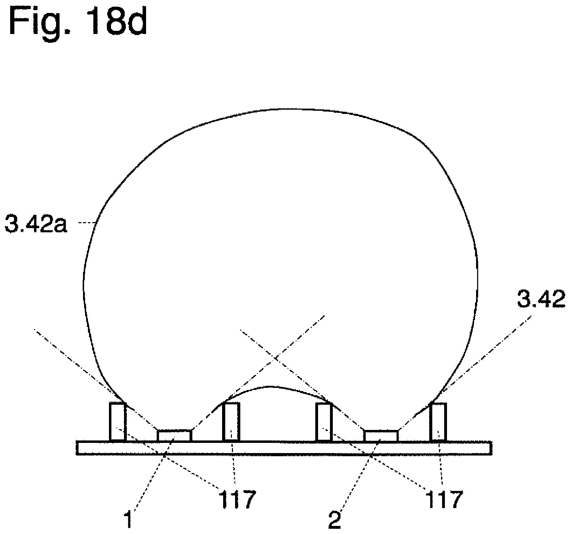

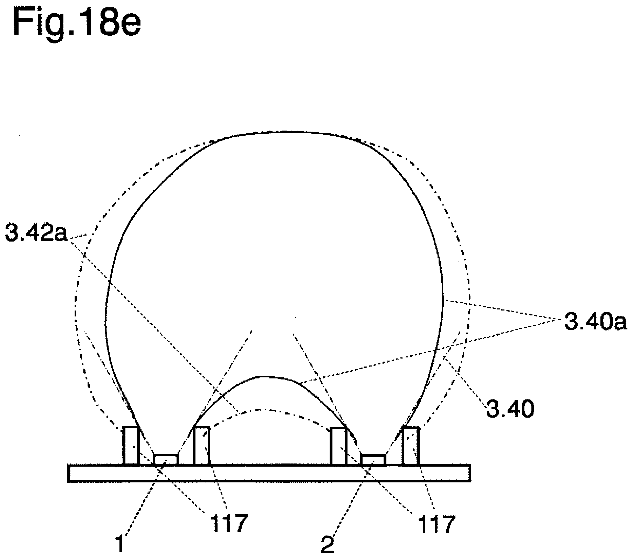

Fig. 18,a,b,c,d,e Richtcharakteristik durch rechteckige Anordnung

Fig.18 zeigt eine Anordnung mit rechteckiger Gestaltung der Lichtsperre am Beispiel des optischen Emitters 1 in der Mitte der Vorrichtung. Die optischen Detektoren 2 können vom Licht über verschiedene Lichtwege 3.40, 3.41, 3.42 mit unterschiedlicher Richtcharakteristik erreicht werden.

Lichtweg 3.41 ist in Fig.18b in einem Schnitt entlang der in Fig. 18 gezeigten Lichtwegrichtung dargestellt. Die strichpunktierten Linien zeigen die begrenzende Wirkung der Lichtsperre 117 auf den Lichtaustritt und auch den Halbschattenbereich.

Lichtweg 3.40 ist in Fig.18c in einem Schnitt entlang der in Fig. 18 gezeigten Lichtwegrichtung dargestellt. Die strichpunktierten Linien zeigen die begrenzende Wirkung der Lichtsperre 117 auf den Lichtaustritt und auch den Halbschattenbereich.

Lichtweg 3.42 ist in Fig.18a in einem Schnitt entlang der in Fig. 18 gezeigten Lichtwegrichtung dargestellt. Die strichpunktierten Linien zeigen die begrenzende Wirkung der Lichtsperre 117 auf den Lichtaustritt und auch den Halbschattenbereich.

Im Vergleich von Fig.18b und 18c erkennt man, dass der Lichtaustrittswinkel in Fig.18c geringer ist und das Licht 3.40 steiler in die Probe gesendet wird. Damit ist weniger Lichtanteil vorhanden, der das Gewebe lediglich in den flachen Schichten durchläuft. Dies wird durch die enger stehenden Seitenwände der rechteckigen Form erreicht.

Im Vergleich von Fig.18b und 18a erkennt man, dass der Lichtaustrittswinkel 3.42 in Fig.18a gegenüber 3.41 breiter ist und das Licht sowohl steil, als auch flach in die Probe gesendet wird. Damit ist mehr Lichtanteil vorhanden, der das Gewebe lediglich in den flachen Schichten durchläuft. Dies wird zum einen durch die weiter stehenden Seitenwände der rechteckigen Form erreicht. Zum anderen wird dies erreicht, weil der Schnitt durch den Emitterchip entlang der Diagonalen eine größere Breite der lichtemittierenden Fläche ergibt. Auch bei kreisförmiger Form der Lichtsperre lässt sich die richtungsabhängige Variation in der Abstrahlcharakteristik erreichen, indem ein eckiger Emitterchip verwendet wird.

Durch Vergleich der Signale unterschiedlicher Richtcharakteristik und durch Vergleich von Signalen unterschiedlicher Weglängen lassen sich nicht nur Absorptions- und Streuanteil auf zwei Wegen berechnen und somit exakter bestimmen, sondern es ist auch möglich Rückschlüsse über Gewebe zu gewinnen, welches in den verschieden tiefen Gewebeschichten abweichendes Absorptions- und Streuverhalten zeigt.

Fig. 18d veranschaulicht den analysierten Volumenbereich von Lichtweg 3.42, der auch flache Lichtanteile enthält. Die durchgezogene Linie 3.42a symbolisiert eine Grenze, des hinreichend stark von Photonen durchfluteten Raumes. Tatsächlich muss eher mit einer Aufenthaltswahrscheinlichkeit der Photonen gerechnet werden, die nach außen langsam abnimmt. Die feste Grenze dient nur dazu die Zusammenhänge zeichnerisch leichter zu verdeutlichen. Der Bereich in der Nähe des unteren Bogens von 3.42a ist dabei besonders wichtig. Dort ist die Photonendichte auch besonders hoch. Gerade in diesem Bereich erkennt man die relevanten Unterschiede zu Fig. 18e.

An dieser Figur soll weiterhin die Tatsache erläutert werden, dass der eingezeichnete Aufenthaltswahrscheinlichkeitsbereich für Photonen sich nicht allein auf die emittierten Photonen bezieht, sondern nur auf diejenigen, die vom Emitter emittiert und vom Detektor detektiert werden. Diese Photonen nehmen alle etwas unterschiedliche Wege. Somit durchstrahlen sie einen Volumenbereich, der in der Probe liegt und sich im Wesentlichen über der Verbindungslinie zwischen Emitter und Detektor befindet. Allerdings kommt immer auch eine seitliche Abweichung von dieser Verbindungslinie vor. Der durchstrahlte Volumenbereich wird als Lichtweg bezeichnet und ist in der Figur 18d mit 3.42a bezeichnet und sein Rand ist mit durchgezogener Linie dargestellt. Wobei nochmal angemerkt ist, dass der Bereich quasi weich ausläuft und somit keinen festen Rand besitzt. Oft entstehen etwa bananenförmige Volumenbereiche mit höherer Photonenaufenthaltsdichte im Bereich der kürzeren Wege von Emitter zu Detektor.

In Fig. 18e ist eine Richtcharakteristik mit engerem Abstrahlwinkel gezeichnet. Der untere Bogen 3.40a verläuft nicht so flach, wie der zum Vergleich strichpunktiert eingezeichnete Bogen 3.42a aus Fig. 18d. Der Einfluss der flachen Gewebeschichten auf das Messsignal ist in 3.40a also geringer. Durch den Vergleich beider Signale ist der Einfluss der flachen und der tiefen Schichten trennbar.

Damit die mathematischen Verfahren zur Trennung dieser Einflüsse Messungenauigkeiten und Rauschanteile nicht zu stark verstärken ist diese Methode in besonders vorteilhafter Ausgestaltung in Kombination mit mehrfach redundanten Lichtwegen zu kombinieren. Beispielsweise durch eine in Fig. 18 gezeigte matrixartige Anordnung.

In vorteilhafter Anordnung können sowohl Emitter als auch optische Detektoren die benötigte Richtcharakteristik realisieren.

In einer bevorzugten Ausführung werden die produktionsbedingten Unterschiede in der Abstrahlcharakteristik der Lichtquelle durch Verfüllen der Emitter-Kavitäten mit einem lichtdurchlässigen aber diffusen Material optimiert.

In einer vorteilhaften Variante ergibt sich durch die rechteckige Formgebung eine besonders platzsparende Anordnung, die auf geringer Fläche eine besonders große Zahl Lichtemitter und Lichtdetektoren beherbergen kann.

In einer vorteilhaften Variante wird die Variation der Abstrahlcharakteristik in Abhängigkeit der Abstrahlrichtung nicht oder nicht nur durch die Form der Lichtsperre erreicht, sondern zumindest teilweise durch die Form und Lage des Emitters oder Detektors selbst. Beispiele dafür sind die Wahl eines rechteckigen Emitters oder einer nicht horizontalen Anordnung der Emitterfläche.

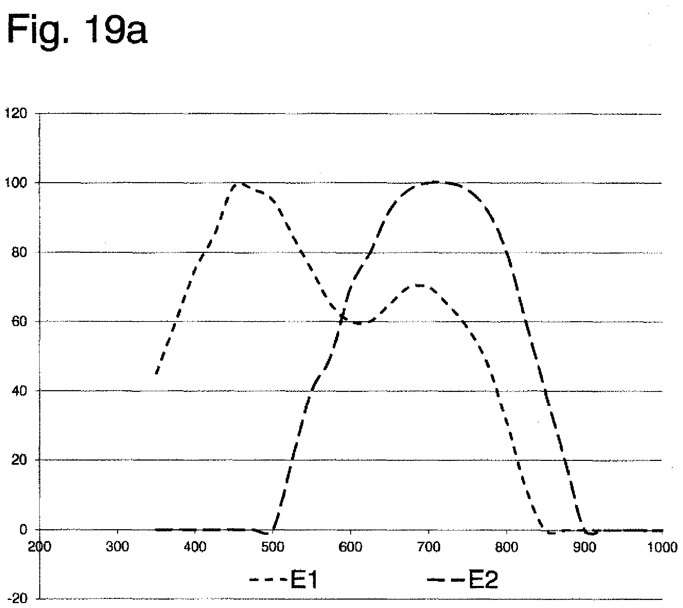

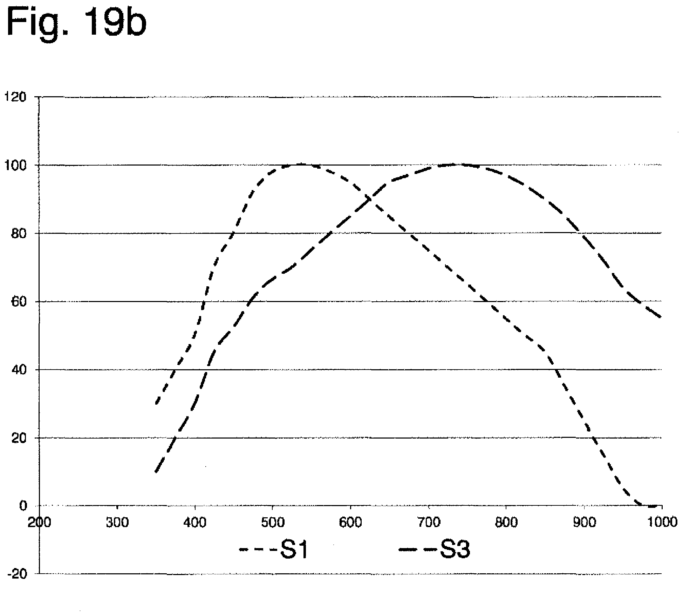

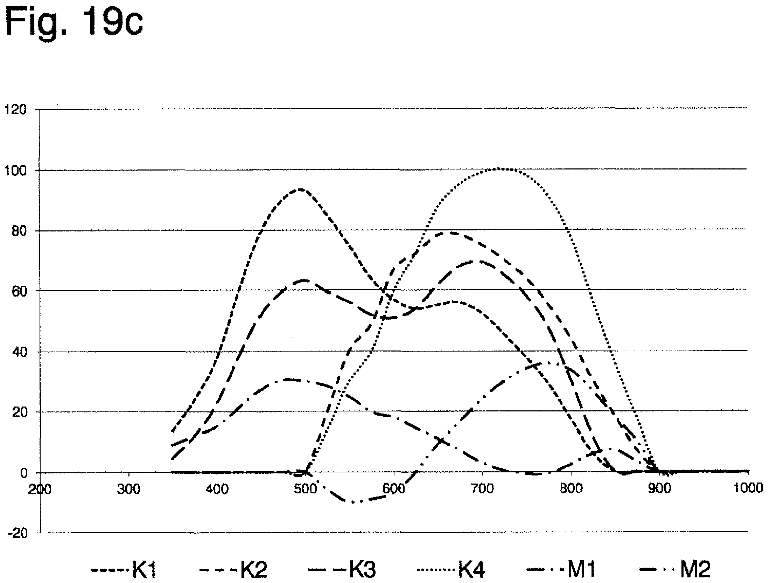

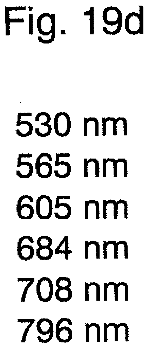

Fig. 19a,b,c,d Erhöhung der spektralen Auflösung durch Kombination unterschiedlicher optischer Emitter mit unterschiedlichen optischen Detektoren

In der vorliegenden Erfindung wird die spektrale Auflösung der Anordnung erhöht, indem unterschiedliche Lichtemitter mit unterschiedlichen Lichtdetektoren gezielt kombiniert werden. Dies ist in den Fig. 19 a bis d gezeigt.

In Fig. 19a sieht man die relativen Spektren der beiden Emitter E1 und E2. Erfindungsgemäß werden hier gezielt auch breitbandige Emitter genutzt. Breitbandige Emitter eignen sich in vorteilhafter Ausgestaltung gut zur Erfassung allgemeiner Eigenschaften der Probe, die nicht auf einen speziellen Stoff gerichtet sind. Zusätzlich kommen schmalbandige Emitter zum Einsatz. In vorteilhafter Variante werden die schmalbandigen Emitter zur Detektion spezieller Stoffe genutzt. Fig. 19b zeigt die Wellenlängencharakteristik der Detektoren S1 und S3. Es ist erkennbar, dass das Spektrum von E2 gut zum Detektor S3 passt und E1 gut zu S1 passt. In bekannter Lösungsvariante würden somit zwei unterschiedliche Wellenlängen gemessen werden, da E2 mit S3 und E1 mit S1 gemessen würde. Fig 19c zeigt die erfindungsgemäße Optimierung der Anordnung. Dabei werden zusätzlich zu den genannten Messwegen K1:E1->S1 und K4:E2->S3 die Wege K3:E1->S3 und K2:E2->S1 erfasst. Die Ergebnisse sind in K1 bis K4 visualisiert. Die Kurven zeigen dabei die Empfindlichkeit des Messweges in Abhängigkeit von der Wellenlänge. Durch Anwendung der weiter hinten in dieser Schrift erläuterten Auswertealgorithmen erhält man außerdem M1 und M2 als Ergebnisse von Verknüpfungen der Messergebnisse von K1 bis K4. Fig. 19d zeigt eine Tabelle die die Schwerpunkte der Kurven K1 bis K4 und M1 und M2 sortiert nach Wellenlänge auflistet. Durch die Kombination der Variation der Wellenlängencharakteristik bei Lichtemitter und Lichtdetektor ist eine erkennbar feinere Wellenlängenauflösung erreicht worden.

**Vorteilhafte spektrale Auslegung**

In vorteilhafter Ausgestaltung werden mindestens 3 unterschiedliche spektrale Charakteristika bei den Lichtemittern realisiert.

In vorteilhafter Ausgestaltung werden mindestens 2 oder 3 unterschiedliche spektrale Charakteristika bei den Lichtdetektoren realisiert.

In vorteilhafter Ausgestaltung ergibt sich in Kombination von Lichtemittern und Lichtdetektoren eine größere Anzahl nutzbarer unterschiedlicher Wellenlängencharakteristika, als es Emitter mit unterschiedlicher Wellenlängencharakteristik gibt und als es Detektoren mit unterschiedlicher Wellenlängencharakteristik gibt.

In vorteilhafter Ausgestaltung ergibt sich in Kombination von Lichtemittern und Lichtdetektoren eine größere Anzahl nutzbarer unterschiedlicher Wellenlängencharakteristika, als es Emitter und Detektoren mit unterschiedlicher Wellenlängencharakteristik gibt.

In vorteilhafter Variante wird die Anzahl nutzbarer Wellenlängenkombinationen erhöht, indem die Detektoren an eine Messschaltung mit besonders hohem Dynamikbereich angeschlossen werden.

In vorteilhafter Ausgestaltung ergeben sich auch Kombinationen aus Emitter und Detektorpaaren, die trotz unterschiedlicher spektraler Charakteristik des Lichtemitters in Kombination mit dem Lichtdetektor zu ähnlichen spektralen Gesamtcharakteristika führen, wie andere Paarungen. Diese Paarungen werden in vorteilhafter Variante genutzt, um gemäß der anderen Abschnitte dieser Beschreibung eine bessere Variation von Lichtwegorientierungen und Lichtweglängen sowie unterschiedlicher Messorte zu realisieren.

**Vorteilhafte geometrische Anordnung**

In vorteilhaften Anordnungen emittieren die Lichtquellen verschiedene Wellenlängen.

Im folgenden bezeichnet LEDxx-yy die LED am Kreuzungspunkt des Zeilensignals yy mit dem Spaltensignal xx aus Fig. 11.

Die im Beispiel gezeigte Anordnung ist symmetrisch und regelmäßig, was jedoch keine notwendige Voraussetzung ist. Vorteilhafte Anordnungen können auch unsymmetrisch und unregelmäßig sein, da sich dadurch z.B. einen größere Anzahl Lichtweglängen erreichen lässt.

Ist eine Wellenlänge besonders wichtig, so kann diese z.B. auf die Positionen LED32-22, LED34-22, LED32-24 und LED24-34 gelegt werden, da sich so eine große Menge an kurzen Lichtwegen ergibt. In vorteilhafter Variante liegt an dieser oder vergleichbarer Stelle eine LED mit einer Wellenlänge, wie sie entweder der zu messende Stoff absorbiert oder reflektiert. In alternativer vorteilhafter Variante liegt an dieser oder vergleichbarer Stelle eine LED, die eine Abschätzung der allgemeinen Absorption erlaubt und nicht im spektralen Bereich liegt, in dem ein zu messender Stoff absorbiert oder reflektiert.

In vorteilhafter Anordnung ist die Position von Emittern der beschriebenen Wellenlänge derart, dass der kürzeste Abstand eines Emitters in dieser Position zu den Detektoren mindestens 2-fach auftritt. In vorteilhafter Alternative, dass die kürzesten Abstände sich weniger als 25% in ihrer Länge unterscheiden. Alternativ auch um 50% oder 80%. In besonders vorteilhafter Anordnung unterscheiden sich die Lichtwege des Lichtes dieser Wellenlänge in ihrer Orientierung.

Vergleichbares gilt für Anordnungen, bei denen Emitter und Detektor vertauscht sind.

Für bestimmte Wellenlängen ist es wichtig besonders kurze Wege durch das zu messende Objekt zu haben. Dies liegt zum einen daran, dass Licht bestimmter Wellenlängen in der Haut, die ein häufiges Messobjekt darstellt stark absorbiert wird. Zum anderen liegt es daran, dass kurze Weglängen vor allem Lichtanteile messen, die nicht tief in das zu messende Objekt eingedrungen sind. Will man Stoffe bestimmen, die vor allem in diesen oberflächennahen Schichten liegen, so muss man für diese Stoffe charakteristische Wellenlängen ggf. mit kurzen Lichtweglängen messen.

In vorteilhaften Varianten werden aber auch Konzentrationen tieferer Schichten im Messobjekt bestimmt, indem der Einfluss der oberflächennahen Schichten abgezogen wird, wozu er zuerst durch Messungen mit kurzer Lichtweglänge bestimmt werden muss. Alternativ dazu werden Lichtwege miteinander verrechnet, die aufgrund unterschiedlicher Abstrahlcharakteristik unterschiedlich tief ins Gewebe eingedrungen sind.

Die im Beispiel gezeigte Anordnung hat für die LED31-22 beispielsweise eine besonders kurze Lichtweglänge.

In einer vorteilhaften Variante der Erfindung werden Emitter, die in einem Wellenlängenbereich emittieren, für den die Erfassung mit kurzer Lichtweglänge vorteilhaft ist an Positionen angeordnet, die einen kurzen Abstand zum Detektor aufweisen. In besonders vorteilhafter Variante so, dass Emitter und Detektor direkt benachbart liegen.

In vorteilhafter Variante besetzen LEDs wichtiger Wellenlängen mehrere entsprechende dem Detektor benachbarte Positionen. Im Beispiel könnte dies LED31-22, LED33-24 und LED35-22 sein.

In alternativer vorteilhafter Variante besetzen LEDs verschiedener Wellenlängen die direkten Nachbarpositionen, um für viele Wellenlängen Messungen mit kurzem Lichtweg durchführen zu können. So könnten im Beispiel LED33-23 670nm, LED31-21 660nm, LED33-24 650nm, LED33-21 640nm, LED33-22 630nm und LED33-25 620nm emittieren.

In vorteilhafter Anordnung sind die Emitter so um die Detektoren angeordnet, dass die räumliche Anordnung in der Fläche mehreren nächsten Nachbarn eines Detektors einen kurzen Lichtweg ermöglicht. In vorteilhafter Variante unterscheiden sich die Lichtwege in ihrer Richtung und Orientierung in vorteilhafter Variante um mindestens 85° in alternativer Variante um mindestens 40° und in anderer alternativer Variante um mehr als 20°, in alternativer Variante um mehr als 10° oder 5° oder um mehr als die Abweichung aufgrund von Fertigungstoleranzen von einer Linienanordnung.

Vergleichbares gilt für Anordnungen, bei denen Emitter und Detektor vertauscht sind. In diesem Text wird deswegen teilweise von optischem Bauteil gesprochen, welches sowohl ein Emitter für Licht, als auch ein Detektor für Licht sein kann und seinem Partner. Der Partner eines optischen Bauteils ist bei Detektoren jeweils ein Bauteil, welches Licht emittieren kann und bei Emittern ein Bauteil welches Licht detektieren kann. Das Licht legt während der Messung den Weg zwischen dem Bauteil und seinem Partner zurück, wobei in der Regel der Anteil des Lichtes gemessen wird, der diesen Weg findet. Unter Verbindungslinie von einem Bauteil zu seinem Partner wird die Linie vom Schwerpunkt der lichtemittierenden Fläche zum Schwerpunkt der lichtsensitiven Fläche verstanden. Der Abstand eines optischen Bauteils zu seinem Partner ist als die Länge dieser Linie zu sehen, wenn nicht der Abstand zwischen den Gehäusen gemeint ist.

In besonders vorteilhafter Anordnung werden die Konzepte kombiniert.

Beispielsweise wird eine besonders wichtige Wellenlänge auf die, Positionen LED31-22, LED33-22, LED35-22, LED31-24 LED33-24, LED35-24 positioniert, während die vergleichbaren Positionen LED31-23, LED33-25, LED35-23 von einer etwas weniger wichtigen Wellenlänge belegt werden, die aber doch noch so wichtig ist, dass redundante Messung gewünscht ist. Und die ebenfalls vergleichbaren Positionen LED31-25 LED33-23, LED35-25 von 3 weiteren unterschiedlichen Wellenlängen belegt werden.

Die Anzahl der Lichtweglängen für die jeweilige Wellenlänge kann durch die zweidimensionale Anordnung also auf die von der Messung benötigten Bedürfnisse abgestimmt werden.

Erfindungsgemäß wird eine Anordnung verwendet, bei der sich die Lichtwege als Kombination der Positionen der Emitter und der Detektoren ergeben, die nicht nur in einer Linie oder einer erkennbaren Näherung einer Linie angeordnet sind.

Erfindungsgemäß ergeben sich so Lichtwege, die nicht parallel sind, sondern sich in vorteilhafter Weise um mindestens 3°, 10°, 25° oder 40° voneinander in ihrer Orientierung unterscheiden.

Erfindungsgemäß ergeben sich durch die Anordnung Lichtwege, bei denen die Anzahl gleichlanger Lichtwege einer Wellenlänge sich von der Anzahl gleichlanger Lichtwege einer anderen Wellenlänge unterscheidet. Unter gleichlang soll dabei nicht nur exakt gleiche Länge verstanden werden, sondern auch im Rahmen der Messung tragbare Unterschiede abdecken, die sich z.B. durch Fertigungsungenauigkeiten oder den minimal technisch sinnvoll realisierbaren Abstand nebeneinander zu platzierender Bauteile ergeben.

Als gleichlang wird insbesondere verstanden, wenn die Abstandsunterschiede kleiner sind als 30% oder 20% oder 10% der maximalen Lichtweglänge.

Insbesondere ergeben sich in vorteilhafter Variante mehr als 2 annähernd gleichlange Lichtwege einer Wellenlänge.

In vorteilhafter Variante werden Detektoren und Emitter so angeordnet, dass die Verteilung der Lichtweglängen für die verschiedenen Wellenlängen unterschiedlich ist. Dadurch ergeben sich Wellenlängen, deren räumliche Auflösung der Lichtweglängen im Bereich der Wege die kürzer sind, als ein Maximalabstand, bei dem aufgrund zu großem Abstand eine Nutzung nur eingeschränkt sinnvoll ist, eine feinere Weglängenauflösung zustande kommt, als bei anderen Wellenlängen. In vorteilhafter Variante ergeben sich für eine Wellenlänge um einen Faktor kleiner 0,9 oder vorteilhafter Weise um kleiner 0,8 oder kleiner 0,6 oder kleiner 0,3 feinere Abstände als bei einer anderen Wellenlänge.

Insbesondere ergibt sich in vorteilhafter Anordnung mindestens ein Lichtwegabstand zwischen zwei Lichtwegen bei einer Wellenlänge der kleiner ist als 100% der Detektorgröße, Emittergrö-ße oder dem Abstand zweier Bauteile dieser beiden Kategorien. In besonders vorteilhafter Anordnung ergibt sich 50% der Detektorgröße oder in noch vorteilhafterer Anordnung 20%, 10% oder sogar 5%.

In vorteilhafter Variante lassen sich durch die beschriebene Anordnung Lichtweglängen erreichen, die sich um folgende Faktoren von den Abständen der Detektoren zueinander unterscheiden. In vorteilhafter Variante sind die Lichtweglängenunterschiede einer Wellenlänge kleiner als der doppelte Detektorabstand, in besonders vorteilhafter Variante kleiner als der Detektorabstand und in ganz besonders vorteilhafter Variante kleiner als 55% des Detektorabstandes.

In vorteilhafter Variante treten bei größeren Lichtweglängen viele feine Abstufungen in den Lichtweglängen auf.

In typischer Umsetzung der Erfindung ist die Anzahl signifikant unterschiedlicher Lichtweglängen größer als die Zahl der Dektoren und größer als die Zahl der Emitter.

In typischer Ausgestaltung der Erfindung beträgt die Anzahl der Lichtwege das Produkt aus Emitter Anzahl und Detektoren Anzahl.

In vorteilhafter Ausgestaltung sind dabei nicht alle Lichtwege einer Wellenlänge von signifikant unterschiedlicher Länge.

Um die Durchdringung der Probe noch besser beeinflussen zu können sind in vorteilhafter Variante der Erfindung mehrere matrixartige Sensoranordnungen verwendet. Ein Beispiel einer solchen Anordnung ist in Fig. 16 und der zugehörigen Figurenbeschreibung erläutert.

**Abstrahlcharakteristik der Emitter und Detektionscharakteristik der Detektoren**

Zur Erfassung der Inhomogenität einer Proben, insbesondere wenn es sich um biologisches Gewebe handelt, spielt die Abstrahlcharakteristik des Lichts in die Probe und die Detektionscharakteristik des Lichts, das aus der Probe kommend vom Detektor gemessen wird, eine gro-ße Rolle. Da in Abhängigkeit vom Einstrahlungswinkelbereich das Licht an unterschiedlich langen Lichtwegen in der Probe propagiert, kann über die Anzahl von Einstrahlwinkelbereichen pro Lichtquelle die Lichtwegvarianz beeinflusst werden. Eine höhere Varianz bildet die Inhomogenitätsbedingungen der Probe besser ab.

Bei Untersuchungen konnte überraschender Weise festgestellt werden, das ein nicht rotationssymmetrischer Abstrahl- oder Detektionswinkel oft zu einer besseren Berücksichtigung der Inhomogenität der Probe und zu einer höheren Genauigkeit der Konzentrationsbestimmung des Analyten führt.

In einer vorteilhaften Ausführung werden an einem Einstrahlungsort für jede Lichtquelle jeweils mindestens zwei unterschiedliche Vorzugswinkelbereiche der Einstrahlung und am Detektionsort für jeden Detektor mindestens zwei unterschiedliche Vorzugswinkelbereiche für die Detektion des aus der Probe heraustretenden Licht realisiert.

In einer weiteren vorteilhaften Ausführung erfolgt die Realisierung unterschiedlicher Vorzugswinkelbereiche durch gezielte Gestaltung einer, das direkte Übersprechen des Lichts vom Emitter zum Detektor verhindernden Lichtsperre. Als Bauteil schirmt die Lichtsperre nach ihrem Aufbringen die Emitter und Detektoren in vorteilhafter Variante untereinander vollständig ab. Emitter und Detektor befinden sich in vorteilhafter Variante in einer jeweils eigenen Kavität mit gewollt zugeschnittener Geometrie.

Bei einer bevorzugten Ausführung besitzt die Lichtsperre eine eckige Geometrie und bei einer noch vorteilhafteren Ausführung eine rechteckige Geometrie. Bei rechteckiger Geometrie können in vorteilhafter Variante grob zwei unterschiedliche Vorzugswinkelbereiche mit einer Emission von Photonen in Winkeln vorzugsweise zwischen 5°-175° zur Einstrahl- und Detektionsfläche eingestellt werden. Die Packungsdichte der von der Lichtsperre umschlossenen Emitter und Detektor Anordnung, ist bei erfindungsgemäßer eckiger Gestaltung höher als bei runder Geometrie der Lichtsperre.

Fig. 18 zeigt eine Anordnung mit rechteckiger Gestaltung der Lichtsperre am Beispiel des optischen Emitters 1 in der Mitte Vorrichtung. Weitere Erläuterung und vorteilhafte Ausgestaltungen dazu finden sich in der Figurenbeschreibung.

## Ausführung der Lichtsperre

Bei einer bevorzugten Ausführung wird die Lichtdichtheit an der Kontaktstelle von Lichtsperre und Platine der Emitter und Detektoren durch gezieltes Erwärmen der Platine erreicht. Eine aus Kunststoff gefertigte Lichtsperre schmilzt/erweicht im Bereich der Kontaktfläche dabei und verschließt so einen möglichen Spalt zwischen Kontaktfläche und Lichtsperre. In vorteilhafter Ausführung sind auf der Platine dazu Leiterbahnen angeordnet, die ein Aufheizen der entsprechenden Stelle ermöglichen. Eine beispielhafte Ausführung ist in Fig. 10 gezeigt und in der Figurenbeschreibung erläutert.

Bei einer anderen bevorzugten Lösung werden die Emitter und Detektoren mit einem lichtdurchlässigen Material zunächst komplett vergossen und anschließend dünne Schlitze eingebracht (z.B. mit Hilfe eines feinen Sägeblatts), die im Vergussmaterial bis auf den Boden der Emitter-Detektor Ebene gehen. Die Schlitze werden anschließen mit einem lichtdichten Material vergossen.

Beschreibung: Fig 13 Beschreibung einer Vorrichtung 1

Die Fig. 13, 14 und 15 zeigen Beispiele einer vorteilhaften Ausgestaltung der Lichtsperre im Schnitt. Weitere Erläuterungen und vorteilhafte Ausgestaltungen finden sich in der Figurenbeschreibung.

## Spektrale Analyse der Probe

Um die zu detektierende Substanz zu detektieren wird Licht einer oder mehrere Wellenlängen verwendet, welche von der Substanz absorbiert oder reflektiert werden. In einigen Fällen wird die Substanz auch mit einer Wellenlänge angeregt und emittiert daraufhin Licht anderer Wellenlänge, welches detektiert wird. Zum Beispiel bei Lumineszenz und Raman Streuung, die ebenfalls zur Detektion genutzt werden können.

Da in der Probe in der Regel eine Vielzahl von Substanzen vorhanden ist muss der Einfluss von Substanzen, die eine optische Auswirkung im Bereich derjenigen Wellenlängen haben, die für die zu analysierende Substanz relevant ist, kompensiert werden. Zu diesem Zweck werden weitere Wellenlängen zur Messung verwendet, aus denen Korrekturwerte bestimmt werden können. Diese Korrekturwerte können entweder direkt mit Konzentrationen eines oder mehrerer anderer Stoffe im Zusammenhang stehen, oder es können Wellenlängen sein, die neben den zur Detektion des Stoffes liegenden Wellenlängen angesiedelt sind und die die optische Charakteristik aller Stoffe der Probe in der Nähe der zu analysierenden Wellenlänge erfassen. Die Algorithmische Verarbeitung ist im Kapitel über den Auswertealgorithmus beschrieben.

Im Stand der Technik findet man Anordnungen, in denen bei zwei oder mehr Wellenlängen gemessen wird. Dabei werden z.B. mehrere Lichtemitter verschiedener Wellenlänge genutzt. Zu jeder Sorte Emitter werden Detektoren eingesetzt, die für die Wellenlänge des Lichtemitters optimiert sind.

In der vorliegenden Erfindung wird die spektrale Auflösung der Anordnung erhöht, indem unterschiedliche Emitter mit unterschiedlichen Detektoren gezielt kombiniert werden.

Dies ist in Fig. 19 a bis d gezeigt. Die Figurenbeschreibung enthält Hinweise zur vorteilhaften Ausgestaltung und zu Anwendungsverfahren, sowie Beispielhafte Ausgestaltungen.

In vorteilhafter Ausgestaltung ergeben sich auch Kombinationen aus Emitter und Detektorpaaren, die trotz unterschiedlicher spektraler Charakteristik des Lichtemitters in Kombination mit dem Lichtdetektor zu ähnlichen spektralen Gesamtcharakteristika führen, wie andere Paarungen. Diese Paarungen werden in vorteilhafter Variante genutzt, um gemäß der anderen Abschnitte dieser Beschreibung eine bessere Variation von Lichtwegorientierungen und Lichtweglängen sowie unterschiedlicher Messorte zu realisieren.

**Auswerteschaltung**

Um viele Emitter-Detektor Paare, zur Verbesserung von Messgenauigkeit und Wiederholgenauigkeit nutzen zu können, bedarf es einer hohen Dynamik der Detektoren, da dann sowohl eine große Anzahl wie eine geringe Anzahl von Photonen korrekt detektierbar und vom Rauschen unterscheidbar sind.

In vorteilhafter Weise führt die erfindungsgemäße hohe Dynamik dazu, dass dann bei gleicher Anzahl von Emittern und Detektoren eine größere Zahl von Emitter-Detektor Paaren zur Erfassung der Inhomogenität der Probe zur Verfügung steht. Somit kann insgesamt mit einer geringeren Anzahl an Bauteilen ein reproduzierbareres Messergebnis gewonnen werden.

**Vorteilhafte Auswertungsschaltung für die Detektoren**

Um die für die Erfindung charakteristisch hohe Anzahl optoelektronischer Bauelemente mit geringem technischen Aufwand anschließen zu können sind verschiedene Ausgestaltungen der Auswerteschaltung vorteilhaft. Einige Beispiele dazu sind in Fig. 12 gezeigt. In Fig. 12 und in ihrer Beschreibung werden verschiedene Varianten gezeigt. In vorteilhafter Ausgestaltung wird nur eine dieser Varianten verwendet. Die Figurenbeschreibung erläutert Beispiele und vorteilhafte Varianten.

In vorteilhafter Variante werden als Detektoren Bauelemente eingesetzt, die Daten über eine digitale Schnittstelle übertragen. In vorteilhafter Variante ist dies ein Bussystem, wie beispielsweise I$^2$C oder SPI. Dies erlaubt in vorteilhafter Ausgestaltung eine einfachere Verdrahtung und höhere Packungsdichte.

In vorteilhafter Variante der Erfindung wird die von den Detektoren gemessene Lichtmenge erfasst, indem Kapazitäten geladen und entladen werden. Vorteilhafte Ausgestaltungen und Verfahren sowie Beispielhafte Implementierungen und Verfahren sind in Fig.17 gezeigt und in der Figurenbeschreibung erläutert.

Vorteilhaft an der in Fig 17 beschriebenen Ausgestaltung ist, dass zu Beginn der Messung keine Information über die zu erwartende Helligkeit vorliegen muss. Verfahren, die mit Umschaltung von Verstärkern arbeiten haben vergleichsweise den Nachteil, dass zunächst im unempfindlichsten Messbereich gemessen werden muss. Anschließend wird bei geringem Signal in einen empfindlicheren Bereich geschaltet und nochmal gemessen. In der beschriebenen matrixförmigen Detektoranordnung wäre dies ein großer Nachteil, da die Messzeit zum Ausmitteln von Flackerndem Licht eine feste Länge aufweist und somit 2 oder mehr Messzeiten benötigt werden. Die Messung wird damit signifikant langsamer und es können in der zur Verfügung stehenden Gesamtmesszeit nur eine geringere Anzahl an Lichtwegen gemessen werden. Diesen Nachteil weist die erfindungsgemäße Ausführung nicht auf. Zu Beginn der Messung muss der zu messende Helligkeitswert nicht bekannt sein. Die Messung nach den in der Figurenbeschreibung erläuterten Modi (Mode D, Mode A, Mode Z und Mode M) beginnen alle gleich. Aus den während der Messung anfallenden Daten kann dynamisch der korrekte Mode ausgewählt werden, ohne dass die Messung neu begonnen werden muss. Dies ist von erheblichen Vorteil, weil beispielsweise bei Messungen an menschlicher Haut die Absorption infolge unterschiedlichen Hauttyps oder unterschiedlicher Konzentrationen der Analyten stark variiert. Da z.B. bei Hauttyp 5 die Lichtabschwächung durch die Haut weitaus stärker ist als bei Hauttyp 1, verändert sich für die Messung das Signal-Rauschverhältnis. In Abhängigkeit von diesen Bedingungen wird erfindungsgemäß in einem Mode gemessen, der auch bei größerem Emitter-Detektor Abstand eine exakte Bestimmung des zu messenden Analyten ermöglicht. Eine Variation der Belichtungszeiten zur Verbesserung des Signal-Rauschverhältnisses ist durch die erfindungsgemäße Gestaltung der Messschaltung deshalb in vorteilhafter Variante nicht nötig.

Die beschriebene Schaltungsvariante kann auch in alternativen Varianten umgesetzt werden, bei denen z.B. Lade- durch Entladevorgänge getauscht werden.

In alternativer vorteilhafter Variante wird ein Strom durch den Detektor zunächst verstärkt und in vorteilhafter Variante analog-digital gewandelt.

In vorteilhafter Variante wird die Helligkeitsmessung mehrfach durchgeführt, um Schwankungen, wie sie z.B. durch periodische oder gleichförmige physiologische Veränderungen entstehen erfassen und/oder kompensieren zu können. Auch Änderungen der Mess- oder Umgebungsbedingungen können so ausgeglichen oder auch gezielt genutzt werden. Ein Beispiel ist die ungewollte Erwärmung des Sensors während der Messung, deren Einfluss bei mehrfacher Messung besser kompensiert werden kann. Die Erwärmung kann aber auch gezielt erfolgen, um z.B. eine Verschiebung der Wellenlängencharakteristik zu erzielen.

EP 4 005 469 B1

**Verfahren zur genaueren Bestimmung der Messwerte durch Verwendung zusätzlicher Information**

Eine besonders vorteilhafte Variante der Erfindung besteht darin Fehler der Vorrichtung zu kompensieren, indem Umwelteinflüsse mit zusätzlichen Sensoren erfasst werden. Insbesondere bieten sich folgende Sensoren an:

- Temperatur
- Feuchtigkeit der Probe an ihrer Oberfläche insbesondere der Kontaktfläche zur Emitter Detektor Anordnung
- Feuchtigkeit des Probeninneres (z.B. über Hochfrequenz- oder kapazitive Messungen)
- Luftspalte zwischen Probe und Emitter-Detektor Anordnung (z.B. über Kapazitätsmessung, Andruckmessung, Triangulation)
- Rauigkeit der Probenoberfläche (z.B. durch optische oder kapazitive Sensoren, z.B. Fingerprintsensor)
- Farbauffälligkeiten der Probenoberfläche
- Leitfähigkeit des Gewebes des Nutzers (verschiedene Verfahren) Druck, der beim Auflegen der Anordnung auf die Probe, z.B. Haut, ausgeübt wird

In vorteilhafter Variante wird die Information dieser Sensoren und der optischen Messwerte der Vorrichtung dazu genutzt eine Abschätzung über die Messunsicherheit oder Standardabweichung zu treffen. Dazu können auch die Messwerte vergangener Messungen und ihr zeitlicher Abstand genutzt werden. Insbesondere können auch statistische Informationen über Messwerte und die Verteilung der Messwerte genutzt werden.

In vorteilhafter Variante wird die abgeschätzte Messunsicherheit oder Standardabweichung genutzt, um die Anzahl der benötigten Messungen zur Erreichung einer angestrebten Genauigkeit zu ermitteln.

In vorteilhafter Variante wird dem Endkunden das Ergebnis in Stufen angezeigt. Dies verhindert, dass der Kunde aus Messschwankungen falsche Schlüsse z.B. über seinen Gesundheitszustand zieht. Messergebnisse, die z.B. eine Konzentration repräsentieren werden dabei Kategorien zugeordnet.

Vorteilhafter Weise geht das letzte Ergebnis dieses Nutzers (im Folgenden wird erläutert, wie der Nutzer von anderen Nutzern unterschieden wird) in die Entscheidung zur Einordnung des Messergebnisses in eine Kategorie ein. In vorteilhafter Variante kann damit die Häufigkeit von durch Messunsicherheiten verursachten Veränderungen der für den Nutzer sichtbaren Messwerte im zeitlichen Verlauf reduziert werden. In vorteilhafter Variante wird dadurch die Sichtbarkeit (oder Wahrnehmung) relevanter Änderungen des Messwertes für den Nutzer nicht signifikant verzögert.

In vorteilhafter Variante können eine oder mehrere zusätzliche Messungen durchgeführt werden, wenn die Messdaten, die Historie der Messdaten oder weitere Informationen darauf hindeuten, dass die Entscheidung welche Ergebnisstufe dem Nutzer angezeigt werden soll zu stark durch Messunsicherheiten beeinflusst wird. In vorteilhafter Variante werden beispielsweise zusätzliche Messungen durchgeführt, wenn die Messwerte im Grenzbereich zwischen zwei Ausgabestufen liegen.

In vorteilhafter Variante können die genannten zusätzlichen Sensoren genutzt werden, um günstige Umgebungsbedingungen für die Messung zu schaffen. So können z.B. Temperatur und Feuchtesensoren genutzt werden, um über Stellelemente günstige Bedingungen für die Messung zu schaffen. Z.B. kann die Vorrichtung oder die Oberfläche der Optoelektronik geheizt werden, um eine Temperaturdrift durch Kontakt zur Probe zu reduzieren/vermeiden. Feuchtigkeit kann z.B. durch einströmende Luft, die durch kleine Auslässe in der Fläche der Emitter-Detektor Anordnung ausgeblasen wird in einen günstigen Bereich gebracht werden. Der Abstand der Probe zur Emitter-Detektor Anordnung kann z.B. verringert werden, indem Luft zwischen der Oberfläche der Optoelektronik und Probe abgesaugt wird und die Probe damit praktisch angesaugt wird. Ein ungünstiger Messort kann verändert werden, indem dem Nutzer signalisiert wird einen anderen Messort zu verwenden, oder indem dem Nutzer die Qualität des Messortes angezeigt wird.

Einige der genannten Verfahren zu Verbesserung der Messsituation sind in alternativer Variante auch ohne entsprechende zusätzliche Sensoren anwendbar.

Kalibriermessungen auf die jeweiligen Hautbedingungen eines Nutzers können erfolgen, indem die Vorrichtung mechanisch über die Haut bewegt wird und permanent Messergebnisse mit mehreren matrixartig angeordneten Emittern und Receivern erzeugt werden, so dass über den gewünschten Messraum eine 2- oder 3-dimensionale Topographie von Messwerten entsteht. Bei einer nachfolgenden Messung, bei der die Vorrichtung nicht unbedingt mehr bewegt werden muss, kann der neue Messort in das topografische Bild eingepasst werden, so dass z.B. der früher bestimmte Karotenoidwert mit dem neu bestimmten Karotenoidwert verglichen werden kann. Veränderungen sind dadurch präziser und früher zu bestimmen, weil bei inhomogener Verteilung eines Stoffes, z.B. der Karotenoide, gleiche Ortskonzentrationen miteinander verglichen werden. Da jede nachfolgende Messung meist nicht immer am gleichen Messort stattfindet, muss die tatsächlich gemessene Veränderung dem Nutzer nicht direkt mitgeteilt werden. Es könnte sein, dass die vorherige Messung an einem Ort mit niedriger oder höherer Konzentration stattgefunden hat und die Veränderung nur durch den neuen Messort hervorgerufen wird. Die Ausgabe des neuen Messwertes könnte den Nutzer zu falscher Einschätzungen verleiten. Der ausgegebene Messwert kann deshalb beispielsweise mit Bezug auf frühere Messungen als arithmetisches Mittel berechnet sein, so dass sich für den Nutzer vielleicht keine zahlenmäßige Veränderung zu früheren Messungen ergibt. Mit einer weiteren Anzeige in Form eines Pfeiles der aufsteigende, absteigende oder gleichbleibende Tendenz signalisiert, wird der tatsächlich ermittelten Veränderung in vorteilhafter Variante jedoch Rechnung getragen.

Die Genauigkeit der Veränderungsbestimmung kann noch dadurch erhöht werden, das die 2- oder 3-D Topografie beispielsweise die Karotenoidkonzentrationen bezogen auf unterschiedliche Tiefen der Haut enthält. So sind beispielsweise Konzentrationsgradienten für die Karotenoide in der Haut bekannt. Wird mit Hilfe der bekannten SRR Methode die Konzentrationsbestimmung in einzelnen Hautschichten durchgeführt (vgl. US 7,139,076 B1) so kann es möglich sein, dass sich Veränderungen nicht in allen Hautschichten zeigen. Der Vergleich kann sich deshalb auf bestimmte Hautschichten beziehen, deren Konzentrationshöhe zeitlich oder physiologisch einer anderen Lage vorausgehen. Aus dieser Kenntnis können für den Nutzer weiterführende Informationen abgeleitet werden, die ihm helfen eine von ihm bewusst oder unbewusst herbeigeführte Änderung der Lebensweise (Schlafdauer, Ernährung, Alkoholkonsum) auf eine konkrete Handlung zurückzuführen. Der dadurch eintretende Lerneffekt hilft ihm sich in Zukunft entsprechend zu verhalten, was beispielsweise im Sinne der Prävention (Minderung des Risikos in Zukunft zu erkranken) vorteilhaft ist.

Das Zurückführen von gemessener Konzentration auf bestimmte Verhaltensweise kann darüber hinaus verbessert werden, wenn neben den reinen Hautmesswerten noch weitere Informationen verarbeitet werden, die der Nutzer beispielsweise in Form eines Fragebogens schriftlich beantwortet. Dieser Fragebogen kann als 2-d oder 3-d Topografie ausgewertet werden, wobei diese Topografie mit der oben beschriebenen 2-d oder 3-d Messtopografie in örtlichen Bezug gebracht wird, indem beide beispielsweise überlagert werden. Dabei können Spitzen auf Täler oder Spitzen auf Spitzen treffen, so dass aus dieser Konstellation ein spezifischer Zusammenhang ermittelbar ist.

## Ankopplung

In einer bevorzugten Ausführung werden Emitter umlaufend am Rand der Emitter-Detektor Verteilung platziert. Der Vergleich der Messwerte ermöglicht die Überprüfung der korrekten Ankopplung der gesamten Emitter-Detektor Anordnung auf der Probe. Bei Messungen an der Handinnenfläche kann dadurch festgestellt werden, ob aufgrund der Wölbung des Daumenballens eine flächige Emitter-Detektor Anordnung bei der Messung rundherum in gleicher Weise aufliegt. Lokal unterschiedliche Ankopplungsbedingungen beeinflussen das Messergebnis in bestimmten Anwendungen nachhaltig.

Überraschender Weise konnte durch Vergleich von Emitter Detektor Paaren, bei denen der Emitter im Randbereich angeordnet ist, festgestellt werden, dass für Paare mit gleichen Abstand die Festlegung von Schwellwerten für das empfangene Licht in vielen Anwendungen ausreicht, um eine vollständige Ankopplung der Emitter-Detektor Anordnung von einer unvollständigen Ankopplung zu unterscheiden. In vorteilhafter Ausgestaltung der Erfindung wird die Ankopplung der Probe messtechnisch überwacht. In vorteilhafter Ausgestaltung durch Schwellwerte für das auf ausgesuchten Lichtwegen gemessene Licht.

In einer vorteilhaften Ausführung erfolgt die Überprüfung der Ankopplung zu Beginn der Messprozedur. Bei unvollständiger Ankopplung wird in vorteilhafter Variante sofort die Messung abgebrochen, was zur Einsparung unnötiger Messzeit führt. Der Nutzer wird über die Ausgabe einer Nachricht aufgefordert die Messung zu wiederholen.

In alternativer Variante erfolgt keine Wiederholungsmessung. Es kann bei der Auswertung der Messung das Licht aus dem betroffenen Randzonenbereich unberücksichtigt bleiben, so dass nur Lichtwege Berücksichtigung finden, für die die Ankopplung korrekt war.

Führt das unvollständige Auflegen der Emitter-Detektor Anordnung zu einem regelrechten Lichtspalt zwischen Gewebeoberfläche und Emitter-Detektor Oberfläche, der zum Eindringen von Fremdlicht führt, wird bei einer bevorzugten Ausführung die Bestimmung durch Messung des Helligkeitswertes bei abgeschalteten Emittern der Vorrichtung realisiert.

Die Überprüfung der Ankopplung betrifft aber nicht nur das unvollständige Auflegen der Emitter-Detektor Anordnung auf der Probe, sondern auch veränderte Ankopplungsbedingungen infolge partieller Feuchtigkeits- oder Fettbildungen auf der Hautoberfläche, lokaler Verunreinigungen, Pigmentstörungen, Verletzungen, Narben etc. Je mehr gleiche Lichtwege miteinander verglichen werden können, umso mehr Ankopplungsprobleme können erkannt und entweder durch Nichtberücksichtigung betroffener Teilmessungen oder durch gezielte Neumessung eliminiert werden. Erfindungsgemäß verbessern sich damit sowohl die Messgenauigkeit wie die Wiederholgenauigkeit der Messung.

In einer bevorzugten Ausführung werden zur Überprüfung der Feuchtigkeitsbedingungen auf der Probenoberfläche Feuchtesensoren eingesetzt, die auf der Fläche der Vorrichtung angebracht sind, die zur Messung Kontakt mit der Probe hat.

## Temperaturdrift der Lichtquelle

Werden LEDs als Emitter verwendet, so verschiebt sich deren optisches Spektrum allein durch die Erwärmung des Emitters beim Anschalten der LED. Beim Messen an biologischen Gewebe erfolgt eine Verschiebung infolge der Körpertemperatur durch Auflegen der Vorrichtung auf die Haut. Je höher der Temperaturgradient umso stärker ist die Temperaturdrift. Mit einer Temperierung der Emitter-Detektor Anordnung kann der Temperaturgradient reduziert werden. Mit Hilfe von Messungen wurde festgestellt, dass bereits einige zusätzlich angebrachte Leiterbahnen, die als Heizfläche genutzt werden, ausreichen, um die Temperatur auf der Ebene der Emitter und Detektoren mit dem Start der Messung so zu erhöhen, dass sie bei in vivo Messungen der Körpertemperatur des Menschen entspricht. Überraschender Weise konnte dabei festgestellt werden, das bereits mit dieser Maßnahme die Wiederholgenauigkeit der Messung verbessert wird.

**Verfahren zur Berechnung physiologischer Parameter oder Stoffkonzentrationen**

Das Verfahren dient dazu Stoffkonzentrationen wie z.B. die Karotenoidkonzentration in der menschlichen Haut oder anderen Proben zu bestimmen. Alternativ können auch physiologische Parameter bestimmt werden, wie z.B. Grad in dem die Haut vor Alterung oder vor Sonneneinstrahlung geschützt ist.

Insbesondere können auch Stoffkonzentrationen an bestimmten Stellen der Haut (in der Fläche der Hautoberfläche) und auch in der Tiefe (senkrecht zur Oberfläche) oder auch relativ zu bestimmen Markerpunkten in der Haut bestimmt werden. Solche Markerpunkte können künstlich gesetzte Punkte sein (auf die Haut oder in die Haut eingebrachte Markierungen sichtbarer oder unsichtbarer Natur) oder natürliche Marker darstellen. Ein Beispiel für natürliche Marker sind z.B. die Schichtengrenzen zwischen Epidermis und Dermis oder eine durch das Gewebe verlaufende Ader.

Insbesondere können die zu bestimmenden Werte nicht nur ortsaufgelöst erfasst werden, sondern auch als Mittelwert.

Im Folgenden wird eine besonders bevorzugte Variante eines Berechnungssystems vorgestellt. Für den Fachmann ist erkennbar, dass vielfältige Möglichkeiten der Umstellung der Berechnungsreihenfolge oder des Ergänzens oder Auslassens von Berechnungsschritten möglich sind, ohne den Grundgedanken des Verfahrens zu verlassen.

Das Berechnungsverfahren verwendet Daten, welche von den beschriebenen Detektoren stammen. Diese sind im wesentlichen Helligkeitswerte sowie Werte anderer Sensoren (z.B. Temperatursensoren und Nutzereingaben), die z.B als Korrekturfaktoren genutzt werden.

Schritt 1: Vorverarbeitungskorrektur

Die üblichen Vorverarbeitungsschritte können auf die Messwerte und auf virtuelle Messwerte, die im Anschluss erläutert werden angewendet werden. Im Folgenden werden diese beiden Wertearten als YS0 bezeichne. Typische Beispiele für Vorverarbeitungsschritte sind die Kompensation von Dunkelwerten, Offsetkorrektur oder getakteter Betrieb, die Berücksichtigung verschiedener Verstärkungsfaktoren oder eine Linearitätskorrektur. Insbesondere ist auch eine Korrektur anhand von bekannten oder geschätzten physikalischen Eigenschaften der Probe möglich. Diese können gemessen, tabelliert oder aus physikalischen Modellen abgeleitet sein. Insbesondere können auch andere Werte, insbesondere Messwerte in die Korrekturgleichungen einfließen.

Die erhaltenen Werte sollen im Folgenden als YS1 bezeichnet werden.

Zur Vereinfachung der Schreibweise werden die verschiedenen YS1 Werte im Folgenden zusammenfassend beschrieben. Gemeint ist natürlich mit YS1 jeder genommene Messwert, so dass YS1 repräsentativ ist für z.B. die Werte:

YS1     Helligkeit aufgenommen von Fotodiode 1 wenn LED 1 leuchtet

YS1     Helligkeit aufgenommen von Fotodiode 2 wenn LED 1 leuchtet

YS1     Helligkeit aufgenommen von Fotodiode 1 wenn LED 2 leuchtet

YS1     Helligkeit aufgenommen von Fotodiode 2 wenn LED 2 leuchtet

YS1     Helligkeit aufgenommen von Fotodiode 10 wenn LED 20 leuchtet

YS1     Temperatur der LED2

YS1     Temperatur der Haut

YS1     Helligkeit aufgenommen von Fotodiode 1 wenn LED 1 leuchtet gemessen zum Zeitpunkt 1

YS1     Helligkeit aufgenommen von Fotodiode 1 wenn LED 1 leuchtet gemessen zum Zeitpunkt 2 und weitere

Schritt 2: erste Gewichtung

Die aus Schritt 1 erhaltenen Informationen können gewichtet werden, dies geschieht im einfachsten Fall durch Multiplikation mit einer Konstanten.

Eine Gewichtung kann auch eine nichtlineare Funktion beinhalten. Besonders vorteilhaft ist eine Gewichtung durch Polynome. Besonders vorteilhaft sind Polynome 4. Grades der Form

$$YS2 = K3*YS1^3 + K2*YS1^2 + K1*YS1 + K0$$

Weiterhin vorteilhaft ist die Gewichtung über Exponentialfunktionen der Form:

$$YS2 = K4* K5^{(YS1*K6)}$$

Aus der Literatur bekannt sind für ähnliche Anwendungszwecke auch logarithmische Funktionen:

$$YS2 = K7 * LOG (K8 * YS1; \text{zur Basis } K9)$$

In besonders vorteilhafter Variante wird eine abschnittsweise definierte lineare Funktion verwendet. Zwischen den gespeicherten Stützstufen kann linear interpoliert werden.

Die Funktionen können durch Modellierung des Problems ausgewählt werden. In vielen Fällen werden jedoch die Wahl der Funktion als auch die Parameter bestimmt, indem eine Menge an Messwerten von Probanden oder Phantomen gewonnen wird, für die das korrekte Ergebnis (z.B. durch ein Referenzverfahren) bekannt ist. Anschließend probiert ein Computer iterativ durch, welche Kombinationen von Algorithmen und Konstanten sich zu einer guten Abbildung der gemessenen Messwerte auf die korrekten Ergebnisse eignen.

Dieses Verfahren gilt in vorteilhafter Variante auch für die folgenden Schritte.

Die Konstanten können für jeden Messwert andere Werte annehmen. Die Schreibweise von K ist hier vereinfacht dargestellt, indem Indizes an K... weggelassen sind.

Eine wichtige Variante der Gewichtung stellt eine zeitliche Filterung dar. Insbesondere können durch den Einsatz von Tiefpassfiltern Messwerte günstig gewichtet werden. Dazu geht der Messzeitpunkt und die Historie der Ausgangswerte YS1 in vorteilhafter Variante in die Berechnung ein. Zu diesem Zweck wird die Historie ausgewählter Werte in vorteilhafter Variante gespeichert. Weitere typische Varianten beinhalten Diffusionsgleichungen, die die Diffusion von Stoffen durch das Gewebe und das daraus resultierende zeitliche Verhalten von Konzentrationen beschreiben können. Diese werden oft durch Varianten von Exponentialfunktionen beschrieben aber auch anders z.B. durch Differentialgleichungen.

Schritt 3: Kompensation

Die aus Schritt 2 gewonnenen Werte für YS2 werden nun miteinander verrechnet, so dass Störgrößen oder Signale wegfallen oder hervorgehoben werden.

Es werden dazu einige neue Werte YS3 gebildet.

Die Werte YS3 werden in einer vorteilhaften Variante durch Summation ermittelt:

$$YS3 = K101*YS2_{Index1} + K102*YS2_{Index2} + .... Kxxx * YS2_{Index N}$$

In einer alternativen vorteilhaften Variante werden die YS3 durch Produktbildung ermittelt:

$$YS3 = YS2_{Index1}{}^{K201} * YS2_{Index2}{}^{K202} * .... * YS2_{Index N}{}^{Kxxx}$$

Dabei kann es oft vorkommen, dass für die zu ermittelnden YS3 verschiedene YS2 Berechnungen eingesetzt werden. Also, dass die YS2 mit teilweise verschiedenen Verarbeitungsfunktionen und unterschiedlichen Konstantenwerten berechnet werden.

Die YS3 werden in vorteilhafter Variante so bestimmt, dass sie bestimmte Eigenschaften der zu untersuchenden Probe bezüglich bestimmter Orte oder Wellenlängen repräsentieren.

Eine weitere vorteilhafte Variante ist der Einsatz eines neuronalen Netzes zur Bestimmung von YS3.

Eine weitere vorteilhafte Variante ist die Bestimmung von YS3 Werten durch statistische Verfahren. Diese sind z.B. Mittelwertbildung oder Medianberechnung. Es können jedoch auch Clusteringverfahren auf Vektoren aus mehreren YS2 angewendet werden. Ein Beispiel ist die beiliegend beschriebene Erkennung und Gewichtung von Ausreißern auf Basis bekannter Standardabweichungen. Auch Kalman oder Wiener Filter.

Eine besonders vorteilhafte Variante bezieht die geometrischen Größen der Emitter-Detektor Anordnung in die Verarbeitung ein. Dazu gehören Abstände der Orte von Lichtaussendung und Lichtempfang sowie die erwarteten Lichtwege der durch die Probe laufenden Photonen. Da die vom Licht durchlaufenen Volumina von Streuung und Absorption abhängen und dies wieder ermittelte Parameter sein können ist eine Abhängigkeit der Volumina von YS0 und YS3 vorteilhaft. Insbesondere ist es oft vorteilhaft zu berechnen, welche Volumina das Licht einer LED/Lichtempfänger-Kombination gemeinsam haben und welche nicht und daraus die Mittelungsfaktoren zu bestimmen.

Verfahren der klassischen Bildverarbeitung können ebenso angewendet werden.

In besonders vorteilhafter Variante werden YS2, die zu einer Gruppe vergleichbarer Emitter Detektor Paare gehören je zu mindestens einem YS3 zusammengefasst. In vorteilhafter Variante sind diese YS3 virtuelle Messwerte, wie in Schritt 4 erläutert. In vorteilhafter Variante werden diese virtuellen Messwerte anschließend in einem weiteren Schritt 3 zusammengefasst um daraus den auszugebenden Konzentrationswert zu ermitteln.

Schritt 4: Gewinnung virtueller Messwerte

Einige der YS3 werden nun zu virtuellen Messwerten erklärt. Damit können Sie die Schritte 1 bis 4 ein weiteres Mal durchlaufen.

Dies ist ein durchaus vorteilhaftes Verfahren, da somit immer wieder gleichartige Berechnungen als Basis dienen können um das Berechnungsergebnis zu gewinnen. Insbesondere ist dadurch eine automatische Bestimmung der Berechnungsfunktionen und der in der Berechnung einzusetzenden Konstanten deutlich erleichtert.

In vielen Fällen müssen mehrere Effekte kompensiert werden, um das Endergebnis zu erhalten.

Beispielsweise werden in einem ersten Durchlauf der Schritte 1-4 unerwünschte Effekte wechselnder Durchblutung des Probanden kompensiert.

Durch die Rückführung der Ergebnisse YS3 zu weiteren YS0 Werten können in einem 2. Durchlauf der Schritte 1 bis 4 Messabweichungen aufgrund variierender Oberflächenankopplung ausgeglichen werden.

In einem dritten Durchlauf der Schritte 1 bis 4 können anschließend die allgemeinen Gewebeabsorptionen von der Absorption einer interessanten Substanz (z.B. Karotinoide) separiert werden.

In einem vierten Durchlauf der Schritte 1 bis 4 können abschließend eine oder mehrere so berechnete Konzentrationswerte einer interessanten Substanz zu einem physiologischen Wert verrechnet werden. Diese kann z.B. eine Empfehlung für zukünftiges Verhalten sein wie z.B.

- Der Fitnesswert der Haut beträgt X (wobei X aus einer Skala von 1 bis 10 stammt)

- Die Wahrscheinlichkeit, dass Tomaten in der nächsten Mahlzeit die Gesundheit um einen bestimmten Wert fördern liegt bei xxx%

Besonders für solche Aussagen ist auch die Verarbeitung von eingegebenen oder anderweitig erfassten Lebensumständen als YS0 Werte vorteilhaft.

Um die Schritte 1 bis 4 mehrfach anzuwenden ist abschließend noch festzulegen, welche YS3 Werte an den Nutzer ausgegeben werden und welche nur als Zwischenvariablen dienen.

In vorteilhafter Variante werden einige der Verfahren und Konstanten sowie die Anzahl der verwendeten YS3 vom Menschen so vorgegeben, dass das bekannte System (z.B. die menschliche Haut und die verwendete Vorrichtung) beschrieben werden. Die übrigen Konstanten ermittelt ein Computer z.B. anhand von einer Messung mit Probanden oder Phantomen, bei denen per Referenzverfahren gewonnene korrekte Werte vorliegen. Die Verfahren dazu können PLS (Parital Least Squares), Hauptkomponentenanalyse oder genetische Algorithmen oder systematisches probieren sein.

In vorteilhafter Variante kann das System auch auf YS3 Zwischenergebnisse trainiert werden, die durch eine Referenzmessung gewonnen werden. Dies können z.B. die Streu- oder Absorptionskoeffizienten von Haut sein, die Anhand von Proben gemessen wurden. Diese Größen sind für Messungen an menschlicher Haut z.B. die Karotinoidmessung bekanntermaßen wichtig und deswegen eine sinnvolle Größe, um sie als Zwischenvariable festzulegen und deren Erkennung separat zu trainieren, bevor der eigentliche Ausgabewert trainiert wird. Während des Trainiervorganges des Ausgabewertes können deswegen auch Verfahren und Gruppen von Konstanten festgehalten werden, während andere zur gezielten iterativen Modifikation verfügbar sind.

In einer vorteilhaften Variante repräsentieren die YS3 Konzentrationswerte eines Stoffes in einer bestimmten Tiefe der Probe, insbesondere einer Hautprobe.

Das beschriebene Auswertungsverfahren bietet entscheidende Vorteile für die beschriebene Vorrichtung. Fig. 10 zeigt eine beispielhafte Ausgestaltung einer Vorrichtung. Diese besteht aus über 100 Lichtemittern und über 40 Lichtdetektoren. Weiterhin wird diese für eine Messung mehrfach ausgewertet, so dass über 20000 Messwerte entstehen. Eine manuelle Erstellung eines Auswertealgorithmus für eine solch große Messwertezahl ist ohne die Verwendung eines Grundgerüstes, wie oben beschrieben kaum möglich. Durch Verwendung des oben beschriebenen Algorithmengerüsts ist die Bestimmung von Algorithmus und Konstanten durch computergestützte Optimierungsverfahren jedoch möglich.

**Patentansprüche**

1. Vorrichtung zur Bestimmung einer Konzentration eines Analyten in einer Probe, wobei die Vorrichtung umfasst:

   mindestens drei Emitter (1, 102, 104, E1, E2, d bis t, x, y) in einer flächigen Anordnung, nicht auf einer Linie, und mindestens drei Detektoren (2, 103, 105, S1, S3, $\alpha$, $\beta$, $\gamma$) in einer flächigen Anordnung, nicht auf einer Linie, wobei die Emitter (1, 102, 104, E1, E2, d bis t, x, y) optische Emitter sind und mindestens drei unterschiedliche Wellenlängencharakteristika aufweisen und wobei die Detektoren optische Detektoren sind,
   **dadurch gekennzeichnet, dass**
   die Vorrichtung Mittel umfasst, die bewirken, dass von den Emittern emittierte Photonen nicht rotationssymmetrisch emittiert und/oder dass von den Detektoren erfasste Photonen nicht rotationssymmetrisch detektiert werden; oder
   die Vorrichtung Mittel umfasst, die bewirken, dass mehrere der mindestens drei Detektoren vom Licht eines der mindestens drei Emitter über verschiedene Lichtwege mit unterschiedlicher Richtcharakteristik erreicht werden; oder
   die Vorrichtung Mittel umfasst, die bewirken, dass ein erster der mindestens drei Emitter in Bezug auf einen ersten der mindestens drei Detektoren eine steilere Richtcharakteristik aufweist als der erste Emitter in Bezug auf einen zweiten der mindestens drei Detektoren.

2. Vorrichtung gemäß Anspruch 1, **dadurch gekennzeichnet, dass** die zumindest drei Emitter und die zumindest drei Detektoren auf einer zweidimensionalen Fläche verteilt sind.

3. Vorrichtung gemäß Anspruch 2, **dadurch gekennzeichnet, dass** es sich bei der zweidimensionalen Fläche um eine elektronische Leiterplatte handelt.

4. Vorrichtung gemäß einem der voranstehenden Ansprüche 2 bis 3, **dadurch gekennzeichnet,**

   **dass** die mindestens drei Emitter und mindestens drei Detektoren auf der zweidimensionalen Fläche derart verteilt sind,
   **dass** sich eine Gruppe gleicher Emitter-Detektor-Paare mit vier oder acht verschiedenen Orientierungen von Verbindungslinien vom Emitter zum Detektor ergibt,
   wobei die gleichen Emitter-Detektor-Paare der vorgenannten Gruppe etwa gleiche Wellenlänge, etwa gleichlange Lichtwege und vergleichbare Richtcharakteristik aufweisen.

5. Vorrichtung gemäß einem der voranstehenden Ansprüche 2 bis 4, **dadurch gekennzeichnet,**
   **dass** die mindestens drei Emitter und mindestens drei Detektoren auf der zweidimensionalen Fläche derart verteilt sind, dass die Vorrichtung umfasst:
   Emitter-Detektor-Paare mit gleichem Emitter-Detektor-Abstand und gleicher Wellenlängencharakteristik, bei denen bei virtueller Kreuzung derer direkten Verbindungslinien in der zweidimensionalen Fläche ein spitzer Winkel entsteht, welcher maximal 10° beträgt.

6. Vorrichtung gemäß einem der voranstehenden Ansprüche 2 bis 5, **dadurch gekennzeichnet,**

   **dass** die mindestens drei Emitter und mindestens drei Detektoren auf der zweidimensionalen Fläche derart verteilt sind, dass die Vorrichtung gleiche Emitter-Detektor-Paare umfasst, und
   **dass** alle gleichen Emitter-Detektor Paare einem flächenbasierten Abstandskriterium genügen, sodass sich eine Fläche eines der gleichen Emitter-Detektor-Paare, die sich durch die Punkte auf der zweidimensionalen Fläche ergibt, deren Abstand zur Verbindungslinie vom jeweiligen Emitter zum Detektor nicht mindestens 1 mm beträgt, zu mindestens 50 % mit einer solchen Fläche eines anderen gleichen Emitter-Detektor-Paars nicht überlappt.

7. Vorrichtung gemäß einem der voranstehenden Ansprüche 2 bis 5, **dadurch gekennzeichnet,**

**dass** die mindestens drei Emitter und mindestens drei Detektoren auf der zweidimensionalen Fläche derart verteilt sind, dass die Vorrichtung gleiche Emitter-Detektor-Paare umfasst, und

**dass** mehrere der gleichen Emitter-Detektor Paare einem flächenbasierten Abstandskriterium genügen, sodass sich eine Fläche eines der gleichen Emitter-Detektor-Paare, die sich durch die Punkte auf der zweidimensionalen Fläche ergibt, deren Abstand zur Verbindungslinie vom jeweiligen Emitter zum Detektor nicht mindestens 3 mm beträgt, zu mindestens 95 % mit einer solchen Fläche eines anderen gleichen Emitter-Detektor-Paars nicht überlappt.

8. Vorrichtung gemäß einem der voranstehenden Ansprüche 6 bis 7, **dadurch gekennzeichnet, dass** die gleichen Emitter-Detektor-Paare einen etwa gleichen Abstand vom Emitter zum Detektor und eine etwa gleiche Wellenlängencharakteristik aufweisen.

9. Vorrichtung gemäß einem der voranstehenden Ansprüche 6 bis 7, **dadurch gekennzeichnet, dass** die gleichen Emitter-Detektor-Paare einen etwa gleichen Abstand vom Emitter zum Detektor, eine etwa gleiche Wellenlängencharakteristik und gleiche Charakteristika von Emissions- und Detektionswinkel aufweisen.

10. Vorrichtung gemäß einem der voranstehenden Ansprüche 2 bis 9, **dadurch gekennzeichnet,**
**dass** die mindestens drei Emitter und mindestens drei Detektoren auf der zweidimensionalen Fläche derart verteilt sind, dass die Vorrichtung umfasst:
Emitter-Detektor-Paare mit gleichem Emitter-Detektor-Abstand und gleicher Wellenlängencharakteristik, bei denen bei virtueller Kreuzung derer direkten Verbindungslinien in der zweidimensionalen Fläche ein spitzer Winkel von 30° bis 45° oder von >45° oder von nahezu 90° entsteht.

11. Vorrichtung gemäß einem der voranstehenden Ansprüche 1 bis 10, **dadurch gekennzeichnet, dass** die Vorrichtung eingerichtet ist zum messtechnischen Überwachen einer Ankopplung der Probe an die Vorrichtung, durch Vergleich von Messwerten für Emitter-Detektor-Paare, bei denen der Emitter im Randbereich angeordnet ist.

12. Vorrichtung gemäß dem voranstehenden Anspruch 11, **dadurch gekennzeichnet, dass** das messtechnische Überwachen durch Schwellwerte für auf ausgesuchten Lichtwegen gemessenes Licht erfolgt.

13. Vorrichtung gemäß einem der voranstehenden Ansprüche 4 bis 12 soweit zurückbezogen auf einen der Ansprüche 2 bis 3, **dadurch gekennzeichnet,**

**dass** die Vorrichtung umlaufend am Rand der Emitter-Detektor-Verteilung platzierte Emitter aufweist und
**dass** die Vorrichtung dazu eingerichtet ist, über einen Vergleich der Messwerte des von den im Randbereich angeordneten Emittern ausgesendeten Lichts eine korrekte Ankopplung der Vorrichtung auf der Probe zu überprüfen.

14. Vorrichtung gemäß einem der voranstehenden Ansprüche 11 bis 13, **dadurch gekennzeichnet, dass** die Vorrichtung eingerichtet ist, über eine aus den Emittern und den Detektoren bestehende Optoelektronik zu prüfen, ob ein Kontakt zur Probe besteht und eine Messung nur auszulösen, wenn ein Kontakt zur Probe besteht, und/oder eingerichtet ist, die Ankopplung der Probe an die Vorrichtung zu Beginn einer Messprozedur zu überprüfen und die Messprozedur abzubrechen, wenn die Ankopplung der Probe unvollständig ist.

15. Vorrichtung gemäß einem der voranstehenden Ansprüche 1 bis 14, **dadurch gekennzeichnet, dass** die Vorrichtung eingerichtet ist, eine Auswertung durchzuführen, bei welcher nur Lichtwege Berücksichtigung finden, für die eine korrekte Ankopplung vorliegt.

16. Vorrichtung gemäß einem der voranstehenden Ansprüche 4 bis 15 soweit zurückbezogen auf einen der Ansprüche 2 bis 3, **dadurch gekennzeichnet,**
**dass** für eine Gruppe von Paaren von Emittern und Detektoren etwa gleicher Wellenlängencharakteristik und etwa gleichen Abstands in jedem Winkelbereich von 70 Grad einer in der Ebene der Emitter und Detektoren angelegten Winkelskala mindestens eine Verbindungslinie eines Paares der Gruppe vom Emitter zum Detektor und/oder vom Detektor zum Emitter existiert, deren Orientierung im angegebenen Winkelbereich liegt.

17. Vorrichtung gemäß einem der voranstehenden Ansprüche 1 bis 16, **dadurch gekennzeichnet,**
**dass** die Vorrichtung Mittel zur Erzeugung einer Richtcharakteristik aufweist, wobei die Mittel zur Erzeugung einer Richtcharakteristik intransparente Elemente aufweisen, die aus intransparentem Material bestehen und Licht abschatten.

18. Vorrichtung gemäß einem der voranstehenden Ansprüche, **dadurch gekennzeichnet,**
**dass** die Vorrichtung zumindest ein intransparentes Material aufweist, welches eine Lichtsperre bildet, welche zu mindestens 50% oder zu mindestens 95% verhindert, dass Licht von einem der Emitter zu einem der Detektoren gelangt, ohne durch die Probe zu laufen.

19. Vorrichtung gemäß einem der voranstehenden Ansprüche, **dadurch gekennzeichnet, dass** die Vorrichtung Mittel zur Erzeugung einer Richtcharakteristik aufweist, wobei die Richtcharakteristik durch Linsen beeinflusst wird.

20. Vorrichtung gemäß einem der voranstehenden Ansprüche, **dadurch gekennzeichnet, dass** die Vorrichtung weiter umfasst:
Sensoren weiterer Umgebungsbedingungen und/oder physikalischer oder chemischer Parameter und/oder von Lebensumständen eines zur Probe gehörigen Organismus.

21. Vorrichtung gemäß einem der voranstehenden Ansprüche, **dadurch gekennzeichnet,**

   **dass** die Emitter und Detektoren als Halbleiterbauelemente ausgestaltet sind,
   wobei die Halbleiterbauelemente nicht weiter als 6 mm oder weniger als 1,5 mm von der Probe entfernt sind,
   wenn sich die Vorrichtung in ihrer Messposition befindet.

22. Vorrichtung gemäß einem der voranstehenden Ansprüche, **dadurch gekennzeichnet,**

   **dass** die Vorrichtung eingerichtet ist, eine Auswertung durchzuführen,
   wobei bei der Auswertung Temperatur und Luftfeuchte der Umgebung, Temperatur eines Sensors und/oder der Probe berücksichtigt werden.

23. Vorrichtung gemäß einem der voranstehenden Ansprüche, **dadurch gekennzeichnet,**
**dass** die Vorrichtung eingerichtet ist, über ein Messverfahren Werte zu gewinnen und bei der Weiterverarbeitung der Werte Informationen mit zu verarbeiten, die die Probe und den zur Probe gehörenden Organismus betreffen.

24. Vorrichtung gemäß einem der voranstehenden Ansprüche, **dadurch gekennzeichnet,**
**dass** jeder der Emitter in mindestens zwei verschiedenen Gruppen gleicher Emitter-Detektorpaare Mitglied ist, wobei gleiche Emitter-Detektorpaare etwa gleichen Emitter-Detektor-Abstand und etwa gleiche Wellenlängencharakteristik aufweisen.

25. Vorrichtung gemäß einem der voranstehenden Ansprüche, **dadurch gekennzeichnet,**
**dass** jeder der Detektoren in mindestens sechs verschiedenen Gruppen gleicher Emitter-Detektorpaare Mitglied ist, wobei gleiche Emitter-Detektorpaare etwa gleichen Emitter-Detektor-Abstand und etwa gleiche Wellenlängencharakteristik aufweisen.

26. Vorrichtung gemäß einem der voranstehenden Ansprüche, **dadurch gekennzeichnet,**
**dass** die Vorrichtung so klein und kompakt ist, dass die Vorrichtung in einen Alltagsgegenstand integriert oder auch als tragbares Taschengerät eingesetzt werden kann.

27. Vorrichtung gemäß einem der voranstehenden Ansprüche, **dadurch gekennzeichnet,**
**dass** die Vorrichtung aus lichtdurchlässigen und lichtundurchlässigen Materialien bestehende Elemente umfasst, die eine Abstrahlcharakteristik eines ersten der mindestens drei Emitter beeinflusst, indem die lichtundurchlässigen Materialien als Blende wirken, sodass Licht von dem ersten Emitter zu einem ersten der mindestens drei Detektoren auf mehreren Lichtwegen gelangt.

28. Vorrichtung gemäß einem der voranstehenden Ansprüche 4 bis 27 soweit zurückbezogen auf Anspruch 3, **dadurch gekennzeichnet,**

   **dass** die Vorrichtung eine auf der elektronischen Leiterplatte aufliegende Lichtsperre aufweist, welche aus lichtundurchlässigem Material besteht und Kavitäten bildet,
   wobei sich in zumindest einer der Kavitäten mehrere Emitter mit mehr als einer Wellenlängencharakteristik befinden.

**29.** Vorrichtung gemäß dem voranstehenden Anspruch 28, **dadurch gekennzeichnet,**
**dass** sich in zumindest einer anderen der Kavitäten ein Emitter mit einer Wellenlängencharakteristik befindet.

**30.** Vorrichtung gemäß einem der voranstehenden Ansprüche, **dadurch gekennzeichnet,**
**dass** Emitter und Detektoren derart angeordnet sind, dass eine Verteilung von Lichtweglängen für verschiedene Wellenlängen unterschiedlich ist.

**31.** Vorrichtung gemäß einem der voranstehenden Ansprüche 4 bis 30 soweit zurückbezogen auf einen der Ansprüche 2 bis 3, **dadurch gekennzeichnet,**

**dass** die Probe ein biologisches Gewebe mit Blutgefäßen und Hautrillen ist und Inhomogenität aufweist und
**dass** die mindestens drei Emitter und mindestens drei Detektoren derart auf der zweidimensionalen Fläche verteilt und orientiert sind,
**dass** bei einem ersten Auflegen der Vorrichtung auf die Probe für eine Erstmessung Licht entlang von Lichtwegen propagiert, welche die Inhomogenität der Probe in unterschiedlichen Vorzugsrichtungen durchlaufen und bei einem erneuten Auflegen der Vorrichtung auf die Probe mit anderem Winkel als beim ersten Auflegen für eine Wiederholungsmessung sich eine Orientierung, in welcher das Licht das biologische Gewebe durchläuft, nicht signifikant ändert,
sodass sich bei der Wiederholungsmessung ein Messergebnis ergibt, welches vergleichbar ist zur Erstmessung.

**32.** Vorrichtung gemäß einem der voranstehenden Ansprüche, **dadurch gekennzeichnet, dass** die Vorrichtung eingerichtet ist, Lichtwege unterschiedlicher Länge zwischen den Emittern und den Detektoren zu verwendet, für die jeweiligen Lichtwege Messwerte aufzunehmen und durch Verrechnung der Messwerte dieser Lichtwege miteinander auf die Eigenschaften der Probe in Abhängigkeit der Tiefe unter einem Punkt der Oberfläche zu schließen.

**33.** Verfahren zur Bestimmung einer Sauerstoffsättigung in der Probe unter Verwendung der Vorrichtung mit den Merkmalen der voranstehenden Ansprüche 1, 2 und 4.

**Claims**

**1.** An apparatus for determining a concentration of an analyte in a sample, the apparatus comprising:

at least three emitters (1, 102, 104, E1, E2, d to t, x, y) in a planar arrangement, not in a line, and at least three detectors (2, 103, 105, S1, S3, $\alpha$, $\beta$, $\gamma$) in a planar arrangement, not in a line, wherein the emitters (1, 102, 104, E1, E2, d to t, x, y) are optical emitters and have at least three different wavelength characteristics and wherein the detectors are optical detectors,
**characterized in that**
the apparatus comprises means which cause photons emitted by the emitters to be emitted non-rotationally-symmetrically and/or that photons detected by the detectors are detected non-rotationally-symmetrically;
or the apparatus comprises means which cause a plurality of the at least three detectors to be reached by the light of one of the at least three emitters via different light paths with different directional characteristics;
or the apparatus comprises means which cause a first of the at least three emitters to have a steeper directional characteristic with respect to a first of the at least three detectors than the first emitter has with respect to a second of the at least three detectors.

**2.** Apparatus according to claim 1, **characterized in that** the at least three emitters and the at least three detectors are distributed on a two-dimensional surface.

**3.** Apparatus according to claim 2, **characterized in that** the two-dimensional surface is an electronic circuit board.

**4.** Apparatus according to one of the preceding claims 2 to 3, **characterized in that** the at least three emitters and at least three detectors are distributed on the two-dimensional surface in such a way, that a group of similar emitter-detector pairs with four or eight different orientations of connecting lines from the emitter to the detector is formed, the similar emitter-detector pairs of the aforementioned group having approximately the same wavelength, approximately the same length of light paths and comparable directional characteristics.

**5.** Apparatus according to one of the preceding claims 2 to 4, **characterized in that** the at least three emitters and at least three detectors are distributed on the two-dimensional surface in such a way that the apparatus comprises: emitter-detector pairs with a similar emitter-detector distance and a similar wavelength characteristic, in which, when their direct connecting lines virtually cross in the two-dimensional surface, an acute angle is formed which is at most 10°.

6. Apparatus according to one of the preceding claims 2 to 5, **characterized in that** the at least three emitters and at least three detectors are distributed on the two-dimensional surface such that the apparatus comprises similar emitter-detector pairs, and that all similar emitter-detector pairs satisfy an area-based distance criterion, so that an area of one of the similar emitter-detector pairs, which is formed by those points on the two-dimensional surface that do not have a distance of at least 1 mm from the connecting line from the respective emitter to the detector, does not overlap by at least 50% with such an area of another similar emitter-detector pair.

7. Apparatus according to one of the preceding claims 2 to 5, **characterized in that** the at least three emitters and at least three detectors are distributed on the two-dimensional surface such that the apparatus comprises similar emitter-detector pairs, and that several of the similar emitter-detector pairs satisfy an area-based distance criterion so that an area of one of the similar emitter-detector pairs formed by the points on the two-dimensional surface that do not have a distance of at least 3 mm to the connecting line from the respective emitter to the detector, does not overlap by at least 95% with such an area of another similar emitter-detector pair.

8. Apparatus according to one of the preceding claims 6 to 7, **characterized in that** the similar emitter-detector pairs have an approximately same distance from the emitter to the detector and an approximately same wavelength characteristic.

9. Apparatus according to one of the preceding claims 6 to 7, **characterized in that** the similar emitter-detector pairs have an approximately same distance from the emitter to the detector, an approximately same wavelength characteristic and similar characteristics of emission and detection angle.

10. Apparatus according to one of the preceding claims 2 to 9, **characterized in that** the at least three emitters and at least three detectors are distributed on the two-dimensional surface such that the apparatus comprises: emitter-detector pairs with a similar emitter-detector distance and a similar wavelength characteristic, in which an acute angle of 30° to 45° or of >45° or of almost 90° is formed when their direct connecting lines virtually cross in the two-dimensional surface.

11. Apparatus according to one of the preceding claims 1 to 10, **characterized in that** the apparatus is configured for measurement-based monitoring of a coupling of the sample to the apparatus, by comparison of measured values for emitter-detector pairs in which the emitter is arranged in the rim region

12. Apparatus according to the preceding claim 11, **characterized in that** the measurement-based monitoring is carried out by using threshold values for light measured on selected light paths.

13. Apparatus according to one of the preceding claims 4 to 12 insofar as related back to one of claims 2 to 3, **characterized in that** the apparatus has emitters placed circumferentially at the rim of the emitter-detector distribution and **in that** the apparatus is configured to check correct coupling of the apparatus to the sample by comparing the measured values of the light emitted by the emitters arranged in the rim region.

14. Apparatus according to one of the preceding claims 11 to 13, **characterized in that** the apparatus is configured to check via optoelectronics consisting of the emitters and the detectors whether there is contact with the sample and to trigger a measurement only if there is contact with the sample, and/or is configured to check the coupling of the sample to the apparatus at the start of a measurement procedure and to abort the measurement procedure if the coupling of the sample is incomplete.

15. Apparatus according to one of the preceding claims 1 to 14, **characterized in that** the apparatus is configured to carry out an evaluation in which only light paths for which a correct coupling is present are taken into account.

16. Apparatus according to one of the preceding claims 4 to 15 insofar as related back to one of claims 2 to 3, **characterized in that** for a group of pairs of emitters and detectors of approximately the same wavelength characteristic and approximately the same distance in each angular range of 70 degrees of an angular scale applied in the plane of the emitters and detectors there is at least one connecting line of a pair of the group from the emitter to the detector and/or from the detector to the emitter, which is oriented within the specified angular range.

17. Apparatus according to one of the preceding claims 1 to 16, **characterized in that** the apparatus comprises means for generating a directional characteristic, wherein the means for generating a directional characteristic comprise non-transparent elements which consist of non-transparent material and which block light.

18. Apparatus according to one of the preceding claims, **characterized in that** the apparatus comprises at least one non-transparent material forming a light barrier which prevents at least 50% or at least 95% of the light of one of the emitters from reaching one of the detectors without passing through the sample.

19. Apparatus according to one of the preceding claims, **characterized in that** the apparatus has means for generating a directional characteristic, the directional characteristic being influenced by lenses.

20. Apparatus according to one of the preceding claims, **characterized in that** the apparatus further comprises: sensors of further environmental conditions and/or physical or chemical parameters and/or life circumstances of an organism belonging to the sample.

21. Apparatus according to one of the preceding claims, **characterized in that** the emitters and detectors are designed as semiconductor devices, the semiconductor devices being no further than 6 mm or less than 1.5 mm away from the sample when the apparatus is in its measuring position.

22. Apparatus according to one of the preceding claims, **characterized in that** the apparatus is configured to carry out an evaluation, the evaluation taking into account the temperature and humidity of the environment, the temperature of a sensor and/or the sample.

23. Apparatus according to one of the preceding claims, **characterized in that** the apparatus is configured to obtain values via a measuring method and, during further processing of the values, to process information which is related to the sample and the organism belonging to the sample.

24. Apparatus according to one of the preceding claims, **characterized in that** each of the emitters is a member of at least two different groups of similar emitter-detector pairs, wherein similar emitter-detector pairs have approximately the same emitter-detector distance and approximately the same wavelength characteristics.

25. Apparatus according to one of the preceding claims, **characterized in that** each of the detectors is a member of at least six different groups of similar emitter-detector pairs, wherein similar emitter-detector pairs have approximately the same emitter-detector distance and approximately the same wavelength characteristics.

26. Apparatus according to one of the preceding claims, **characterized in that** the apparatus is so small and compact that the apparatus can be integrated into an everyday object or can also be used as a portable pocket apparatus.

27. Apparatus according to one of the preceding claims, **characterized in that** the apparatus comprises elements consisting of translucent and opaque materials which influence an emission characteristic of a first of the at least three emitters, **in that** the opaque materials act as a mask so that light from the first emitter reaches a first of the at least three detectors on several light paths.

28. Apparatus according to one of the preceding claims 4 to 27 insofar as related back to claim 3,

   **characterized in that** the apparatus has a light barrier which is arranged on the electronic printed circuit board, consists of opaque material and forms cavities,
   a plurality of emitters with more than one wavelength characteristic being located in at least one of the cavities.

29. Apparatus according to the preceding claim 28, **characterized in that** an emitter with one wavelength characteristic is located in at least one other of the cavities.

30. Apparatus according to one of the preceding claims, **characterized in that** emitters and detectors are arranged in such a way that a distribution of light path lengths is different for different wavelengths.

31. Apparatus according to one of the preceding claims 4 to 30 insofar as related back to one of claims 2 to 3, **charac-**

**terized in that** the sample is a biological tissue with blood vessels and skin grooves and has inhomogeneity and that the at least three emitters and at least three detectors are distributed and oriented on the two-dimensional surface in such a way that when the apparatus is first placed on the sample for a first measurement, light propagates along light paths, which pass through the inhomogeneity of the sample in different preferred directions and, when the apparatus is placed on the sample again at a different angle than when it was first placed for a repeat measurement, an orientation in which the light passes through the biological tissue does not change significantly, so that the repeat measurement produces a measurement result which is comparable to the first measurement.

**32.** Apparatus according to one of the preceding claims, **characterized in that** the apparatus is configured to use light paths of different lengths between the emitters and the detectors, to record measured values for the respective light paths and to draw conclusions about the properties of the sample as a function of the depth below a point on the surface by mathematically processing the measured values of these light paths.

**33.** Method for determining an oxygen saturation in the sample using the apparatus having the features of the preceding claims 1, 2 and 4.

**Revendications**

**1.** Dispositif pour déterminer une concentration d'un analyte dans un échantillon, le dispositif comprenant : au moins trois émetteurs (1, 102, 104, E1, E2, d à t, x, y) selon une disposition plane, mais non en ligne, et au moins trois détecteurs (2, 103, 105, S1, S3, $\alpha$, $\beta$, y) selon une disposition plane, mais non en ligne, dans lequel les émetteurs (1, 102, 104, E1, E2, d à t, x, y) sont des émetteurs optiques et ont au moins trois caractéristiques de longueur d'onde différentes, et dans lequel les détecteurs sont des détecteurs optiques,
**caractérisé en ce que** :

le dispositif comprend des moyens qui provoquent l'émission sans symétrie de rotation de photons émis par les émetteurs, et/ou **en ce que** les photons détectés par les détecteurs sont détectés sans symétrie de rotation ; ou
le dispositif comprend des moyens qui provoquent l'atteinte d'une pluralité des détecteurs au nombre d'au moins trois par la lumière d'au moins l'un des émetteurs au nombre d'au moins trois par l'intermédiaire de trajets de lumière différents ayant des caractéristiques de directivité différentes ; ou
le dispositif comprend des moyens qui amènent un premier des émetteurs au nombre d'au moins trois à avoir une caractéristique de directivité en plus forte pente par rapport à un premier des détecteurs au nombre d'au moins trois que ne l'est celle du premier émetteur par rapport à un deuxième des détecteurs au nombre d'au moins trois.

**2.** Dispositif selon la revendication 1, **caractérisé en ce que** les émetteurs au nombre d'au moins trois et les détecteurs au nombre d'au moins trois sont répartis sur une surface en deux dimensions.

**3.** Dispositif selon la revendication 2, **caractérisé en ce que** la surface en deux dimensions est constituée par une carte de circuits imprimés électronique.

**4.** Dispositif selon l'une des revendications 2 à 3 qui précèdent, **caractérisé en ce que** les émetteurs au nombre d'au moins trois et les détecteurs au nombre d'au moins trois sont répartis sur la surface en deux dimensions, de telle sorte que soit obtenu un groupe de paires émetteur-détecteur similaires avec quatre ou huit orientations différentes de lignes de liaison de l'émetteur au détecteur, les paires émetteur-détecteur similaires du groupe précédemment mentionné ayant approximativement la même longueur d'onde, approximativement la même longueur de trajets de lumière et des caractéristiques de directivité comparables.

**5.** Dispositif selon l'une des revendications 2 à 4 qui précèdent, **caractérisé en ce que** les émetteurs au nombre d'au moins trois et les détecteurs au nombre d'au moins trois sont répartis sur la surface en deux dimensions, de telle sorte que le dispositif comprenne : des paires émetteur-détecteur avec une distance émetteur-détecteur similaire et une caractéristique de longueur d'onde similaire, dans lequel, lorsque leurs lignes de liaison directes se croisent virtuellement dans la surface en deux dimensions, il est formé un angle aigu qui est d'au plus 10°.

**6.** Dispositif selon l'une des revendications 2 à 5 qui précèdent, **caractérisé en ce que** les émetteurs au nombre d'au moins trois et les détecteurs au nombre d'au moins trois sont répartis sur la surface en deux dimensions, de telle sorte que le dispositif comprenne des paires émetteur-détecteur similaires, et **en ce que** toutes les paires émetteur-détecteur similaires satisfont à un critère de distance basé sur la surface, de telle sorte qu'une surface de l'une des paires émetteur-détecteur similaires, qui est constituée par les points sur la surface en deux dimensions qui n'ont pas une distance d'au moins 1 mm par rapport à la ligne de liaison de l'émetteur au détecteur respectifs, ne chevauche pas d'au moins 50% une telle surface d'une autre paire émetteur-détecteur similaire.

7. Dispositif selon l'une des revendications 2 à 5 qui précèdent, **caractérisé en ce que** les émetteurs au nombre d'au moins trois et les détecteurs au nombre d'au moins trois sont répartis sur la surface en deux dimensions, de telle sorte que le dispositif comprenne des paires émetteur-détecteur similaires, et **en ce que** plusieurs des paires émetteur-détecteur similaires satisfont à un critère de distance basé sur la surface, de telle sorte qu'une surface de l'une des paires émetteur-détecteur similaires, qui est constituée par les points sur la surface en deux dimensions qui n'ont pas une distance d'au moins 3 mm par rapport à la ligne de liaison de l'émetteur au détecteur respectifs, ne chevauche pas d'au moins 95% une telle surface d'une autre paire émetteur-détecteur similaire.

8. Dispositif selon l'une des revendications 6 à 7 qui précèdent, **caractérisé en ce que** les paires émetteur-détecteur similaires ont approximativement la même distance de l'émetteur au détecteur et approximativement la même caractéristique de longueur d'onde.

9. Dispositif selon l'une des revendications 6 à 7 qui précèdent, **caractérisé en ce que** les paires émetteur-détecteur similaires ont approximativement la même distance de l'émetteur au détecteur et approximativement la même caractéristique de longueur d'onde, et des caractéristiques d'angle d'émission et de réception similaires.

10. Dispositif selon l'une des revendications 2 à 9 qui précèdent, **caractérisé en ce que** les émetteurs au nombre d'au moins trois et les détecteurs au nombre d'au moins trois sont répartis sur la surface en deux dimensions, de telle sorte que le dispositif comprenne : des paires émetteur-détecteur ayant une distance émetteur-détecteur similaire et une caractéristique de longueur d'onde similaire, et dans lequel un angle aigu de 30° à 45° ou > 45° ou de presque 90° est formé lorsque leurs lignes de liaison directes se croisent virtuellement dans la surface en deux dimensions.

11. Dispositif selon l'une des revendications 1 à 10 qui précèdent, **caractérisé en ce que** le dispositif est configuré pour un contrôle métrologique d'un couplage de l'échantillon à l'appareil, par la comparaison de valeurs mesurées pour des paires émetteur-détecteur dans lesquelles l'émetteur est disposé dans la région de bord.

12. Dispositif selon la revendication 11, **caractérisé en ce que** le contrôle métrologique est effectué à l'aide de valeurs de seuil pour la lumière mesurée sur des trajets de lumière sélectionnés.

13. Dispositif selon l'une des revendications 4 à 12 qui précèdent lorsqu'elles dépendent de l'une des revendications 2 à 3, **caractérisé en ce que** le dispositif comporte des émetteurs disposés de façon périphérique au bord de la distribution émetteurs-détecteurs, et **en ce que** le dispositif est configuré de façon à vérifier un couplage correct du dispositif avec l'échantillon par la comparaison des valeurs mesurées de la lumière émise par les émetteurs disposés dans la région de bord.

14. Dispositif selon l'une des revendications 11 à 13 qui précèdent, **caractérisé en ce que** le dispositif est configuré de façon à vérifier, à l'aide d'un système opto-électronique comprenant des émetteurs et des détecteurs, s'il y existe ou non un contact avec l'échantillon, et à ne déclencher une mesure que si un contact avec l'échantillon est établi, et/ou est configuré de façon à vérifier le couplage de l'échantillon avec le dispositif au début d'une procédure de mesure, et à interrompre la procédure de mesure si le couplage de l'échantillon est incomplet.

15. Dispositif selon l'une des revendications 1 à 14 qui précèdent, **caractérisé en ce que** le dispositif est configuré de façon à effectuer une évaluation dans laquelle seuls sont pris en considération des trajets de lumière pour lesquels un couplage correct est présent.

16. Dispositif selon l'une des revendications 4 à 15 qui précèdent lorsqu'elles dépendent de l'une des revendications 2 à 3, **caractérisé en ce que**, pour un groupe de paires d'émetteurs et de détecteurs ayant approximativement la même caractéristique de longueur d'onde et approximativement la même distance dans chaque plage angulaire de 70 degrés d'une échelle angulaire appliquée dans le plan des émetteurs et des détecteurs, il y existe au moins une ligne de liaison d'une paire du groupe de l'émetteur au détecteur et/ou du détecteur à l'émetteur qui est orientée à l'intérieur de la plage angulaire spécifiée.

**17.** Dispositif selon l'une des revendications 1 à 16 qui précèdent, **caractérisé en ce que** le dispositif comprend des moyens pour générer une caractéristique de directivité, les moyens pour générer une caractéristique de directivité comprenant des éléments opaques qui sont constitués par un matériau opaque et qui arrêtent la lumière.

**18.** Dispositif selon l'une des revendications précédentes, **caractérisé en ce que** le dispositif comprend au moins un matériau opaque constituant une barrière vis-à-vis de la lumière, qui empêche au moins 50% ou au moins 95% de la lumière venant de l'un des émetteurs d'atteindre l'un des détecteurs sans traverser l'échantillon.

**19.** Dispositif selon l'une des revendications précédentes, **caractérisé en ce que** le dispositif comporte des moyens pour générer une caractéristique de directivité, la caractéristique de directivité étant influencée par des lentilles.

**20.** Dispositif selon l'une des revendications précédentes, **caractérisé en ce que** le dispositif comprend de plus: des capteurs pour d'autres conditions environnementales et/ou paramètres physiques ou chimiques et/ou d'autres circonstances de vie d'un organisme auquel appartient l'échantillon.

**21.** Dispositif selon l'une des revendications précédentes, **caractérisé en ce que** les émetteurs et les détecteurs sont réalisés sous la forme de dispositifs à semiconducteurs, les dispositifs à semiconducteurs n'étant pas éloignés de plus de 6 mm ou de moins de 1,5 mm de l'échantillon lorsque le dispositif se trouve dans sa position de mesure.

**22.** Dispositif selon l'une des revendications précédentes, **caractérisé en ce que** le dispositif est configuré de façon à effectuer une évaluation, l'évaluation prenant en considération la température et l'humidité atmosphérique de l'environnement, la température d'un capteur et/ou de l'échantillon.

**23.** Dispositif selon l'une des revendications précédentes, **caractérisé en ce que** le dispositif est configuré de façon à obtenir des valeurs à l'aide d'un procédé de mesure, et, lors d'un traitement ultérieur des valeurs, à traiter une information qui est associée à l'échantillon et à l'organisme auquel appartient l'échantillon.

**24.** Dispositif selon l'une des revendications précédentes, **caractérisé en ce que** chacun des émetteurs est un élément dans au moins deux groupes différents de paires émetteur-détecteur similaires, des paires émetteur-détecteur similaires ayant approximativement la même distance émetteur-détecteur et approximativement la même caractéristique de longueur d'onde .

**25.** Dispositif selon l'une des revendications précédentes, **caractérisé en ce que** chacun des détecteurs est un élément dans au moins six groupes différents de paires émetteur-détecteur similaires, des paires émetteur-détecteur similaires ayant approximativement la même distance émetteur-détecteur et approximativement la même caractéristique de longueur d'onde .

**26.** Dispositif selon l'une des revendications précédentes, **caractérisé en ce que** le dispositif est si petit et si compact que le dispositif peut être intégré dans un objet de la vie quotidienne ou peut également être utilisé comme appareil de poche portable.

**27.** Dispositif selon l'une des revendications précédentes, **caractérisé en ce que** le dispositif comprend des éléments constitués par des matériaux translucides et opaques qui influencent une caractéristique d'émission d'un premier des émetteurs au nombre d'au moins trois, et **en ce que** le matériau opaque forme un ou plusieurs trous, de telle sorte que la lumière venant du premier émetteur atteigne un premier des détecteurs au nombre d'au moins trois sur plusieurs trajets de lumière.

**28.** Dispositif selon l'une des revendications 4 à 27 qui précèdent lorsqu'elles dépendent de la revendication 3, **caractérisé en ce que** le dispositif comporte une barrière vis-à-vis de la lumière qui est disposée sur la carte de circuits imprimés électronique, qui est constituée par un matériau opaque et qui forme des cavités, et une pluralité d'émetteurs avec plus d'une caractéristique de longueur d'onde étant disposés dans au moins l'une des cavités.

**29.** Dispositif selon la revendication 28 qui précède, **caractérisé en ce qu'**un émetteur avec une caractéristique de longueur d'onde unique est disposé dans au moins une autre des cavités.

**30.** Dispositif selon l'une des revendications précédentes, **caractérisé en ce que** des émetteurs et des détecteurs sont disposés de telle sorte qu'une répartition de longueurs de trajets de lumière soit différente pour des longueurs d'onde différentes.

**31.** Dispositif selon l'une des revendications 4 à 30 qui précèdent lorsqu'elles dépendent de l'une des revendications 2 à 3, **caractérisé en ce que** l'échantillon est un tissu biologique avec des vaisseaux sanguins et des sillons cutanés et présente une inhomogénéité, et **en ce que** les émetteurs au nombre d'au moins trois et les détecteurs au nombre d'au moins trois sont répartis et orientés dans la surface en deux dimensions, de telle sorte que, lorsque le dispositif est disposé une première fois sur l'échantillon pour une première mesure, la lumière se propage le long de trajets de lumière qui traversent l'inhomogénéité de l'échantillon dans des directions différentes préférées, et, lorsque le dispositif est à nouveau disposé sur l'échantillon sous un angle différent de celui sous lequel il était disposé la première fois pour une répétition de la mesure, une orientation dans laquelle la lumière traverse le tissu biologique ne change pas de façon significative, de telle sorte que la répétition de la mesure produise un résultat de mesure qui est comparable à la première mesure.

**32.** Dispositif selon l'une des revendications précédentes, **caractérisé en ce que** le dispositif est configuré de façon à utiliser des trajets de lumière de longueurs différentes entre les émetteurs et les détecteurs, de façon à enregistrer des valeurs mesurées pour les trajets de lumière respectifs et à tirer des conclusions concernant les propriétés de l'échantillon en fonction de la profondeur en dessous d'un point sur la surface par une analyse mathématique des valeurs mesurées de ces trajets de lumière.

**33.** Procédé pour déterminer une saturation en oxygène dans l'échantillon à l'aide du dispositif comportant les éléments ou caractéristiques selon les revendications 1, 2 et 4 qui précèdent.

# Fig. 1

# Fig. 2

EP 4 005 469 B1

Fig. 3

24x 3.10

Fig. 4

16x 3.15

Fig. 6

12x 3.30

Fig. 5

32x 3.21

Fig. 7

Fig. 8

# Fig. 9

EP 4 005 469 B1

Fig. 10

Fig. 10a

Fig. 10b

Fig. 10c

Fig. 10d

Fig. 10e

Fig. 10f

Fig. 11

EP 4 005 469 B1

Fig. 12

Fig. 13

Fig.14

## Fig. 15

EP 4 005 469 B1

Fig. 16

Fig. 17

Fig.17a

EP 4 005 469 B1

# Fig.18

Fig. 18a  Fig. 18b  Fig. 18c

EP 4 005 469 B1

Fig. 18d

## Fig.18e

EP 4 005 469 B1

Fig. 19a

# Fig. 19b

EP 4 005 469 B1

Fig. 19c

# Fig. 19d

530 nm

565 nm

605 nm

684 nm

708 nm

796 nm

EP 4 005 469 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4427101
- US 7139076 B
- DE 10163972
- WO 9410901 A1
- WO 9410901 A
- US 2008232653 A1
- US 2002058865 A1
- WO 2010056973 A1
- US 2005020892 A1
- US 7139076 B1